# EUROPEAN PATENT APPLICATION

(11) **EP 4 624 492 A1**
(43) Date of publication of application: **01.10.2025**
(21) Application number: 23893747.8
(22) Date of filing: 17.11.2023
(51) Int. Cl.: C07K 16/28, A61K 39/395, A61P 35/00

(54) **ANTI-CCR8 ANTIBODY AND USE THEREOF**

(30) Priority: 22.11.2022 CN 202211466450
(71) Applicant: Shanghai Hongcheng Biopharmaceutical Co., Ltd., Shanghai 201203 (CN)
(72) Inventor: SU, Yunpeng, Shanghai 201203 (CN); LI, Yong, Shanghai 201203 (CN); ZHUANG, Weiliang, Shanghai 201203 (CN); JIANG, Diandong, Shanghai 201203 (CN); LIANG, Shaoqin, Shanghai 201203 (CN)
(74) Representative: Bianchetti & Minoja with Trevisan & Cuonzo IPS SRL
(86) International application number: PCT/CN2023/132346
(87) International publication number: WO 2024/109657

(57) **Abstract**

Provided is an antibody targeting CCR8 or antigen-binding fragment thereof and use of the same. The antibody targeting CCR8 or antigen-binding fragment thereof specifically binds to CCR8. The present application further provides the use of the antibody in blocking the binding of CCL1 to CCR8/signaling pathways induced thereon, depleting CCR8-positive Treg cells through ADCC effect, and enhancing anti-tumor immunity.

## Description

### Technical Field

The present invention relates to the fields of monoclonal antibodies and/or engineered antibodies. In particular, the present invention provides antibodies or antigen-binding fragments thereof that specifically bind to CCR8 and compositions containing the same. Also provided are nucleic acid molecules encoding the antibodies or the antigen-binding fragments thereof of the invention, vectors and host cells for expressing the antibodies or the antigen-binding fragments thereof of the invention, and methods of treatment and diagnosis /detection using the antibodies or the antigen-binding fragments thereof of the invention and uses thereof.

### Background Art

The immune system in the organism is the core defense system against tumor development and progression, and the inability of the immune system to recognize and eliminate malignant cells plays a key role in the pathogenesis of cancer. In the tumor microenvironment, high expression of multiple immune checkpoints reduces immune activation and suppresses anti-tumor immunity. Monoclonal antibodies against the suppressive immune checkpoints CTLA-4 and PD-1/PD-L1 produce significant anti-tumor responses by up-regulating immune activation in the tumor microenvironment. Currently, many monoclonal antibodies against CTLA-4 and PD-1/PD-L1 have been approved clinically for the treatment of various solid tumors, including the anti-CTLA-4 antibody ipilimumab, anti-PD-1 antibodies nivolumab and pembrolizumab, the anti-PD-L1 antibody atezolizumab, etc. Compared with the conventional therapies (including chemotherapy, radiotherapy and surgery), cancer immunotherapy has brought significant improvements in survival rate and quality of life for patients (K Esfahaniwait, et.al. A review of cancer immunotherapy: from the past, to the present, to the future, Curr Oncol. 2020 Apr; 27(Suppl 2): S87-S97).

Although immune checkpoint inhibitors have been clinically approved for the treatment of a variety of solid tumors, their response rates vary greatly among various tumor types, and the overall response rate is low. The overall response rate (ORR) of the immune checkpoint inhibitor in non-small cell lung cancer (NSCLC), urothelial carcinoma, and head and neck cancer is only about 20% and less than 10% in solid tumors such as gastric cancer (Schoenfeld A. J. and Hellmann M. D., Acquired Resistance to Immune Checkpoint Inhibitors. Cancer Cell. 2020 Apr 13; 37(4): 443-455. doi: 10.1016/j.ccell.2020.03.017). Some patients exhibit durable responses to immune checkpoint inhibitors, but many patients who are responsive initially later develop acquired resistance, for example, 50% of patients with NSCLC and gastric cancer developing acquired resistance (Schoenfeld A. J. and Hellmann M. D.). Some patients with solid tumors do not benefit from immune checkpoint inhibitors, but rather unfortunately undergo tumor hyperprogression (Hyperprogressive), which occurs in patients with various solid tumors in different proportions after immune checkpoint therapy, for example, 14% of patients with NSCLC experiencing hyperprogression (Champiat S. et al., Hyperprogressive disease: recognizing a novel pattern to improve patient management. Nat Rev Clin Oncol. 2018 Dec; 15(12): 748-762. doi: 10.1038/s41571-018-0111-2). At present, there is still a need to develop new immune checkpoint inhibitors or immunomodulators to supplement or overcome the existing shortcomings and problems of the current immune checkpoint inhibitors clinically used.

The low overall response rate to the immune checkpoint inhibitor and the development of acquired resistance and hyperprogression are related to the signaling pathways within tumor cells and multiple mechanisms in the tumor microenvironment (Baxter MA et al., Resistance to immune checkpoint inhibitors in advanced gastro-oesophageal cancers. Br J Cancer. 2021 Oct; 125(8): 1068-1079. doi: 10.1038/s41416-021-01425-7). Regulatory T-cells (Treg) play a key role in immune regulation and are closely related to the occurrence and development of tumors. Compared with the periphery, Treg cells increase in number and have enhanced functionality in the tumor microenvironment. Treg cells suppress anti-tumor immunity via mechanisms such as secreting suppressive cytokines such as IL-10, depleting IL-2, and direct contact suppression, which is closely related to the intrinsic and acquired resistance to immune checkpoint inhibitors (Baxter MA et al.). PD-1 blockade can induce recovery of the dysfunctional PD-1⁺CD8⁺T cells, but can also enhance PD-1⁺Treg cell-mediated immunosuppression. If the number of Treg cells expressing PD-1 in the tumor microenvironment is higher than that of PD-1⁺CD8⁺T cells, PD-1 blockade may lead to hyperprogression of tumors (Kumagai S et al., The PD-1 expression balance between effector and regulatory T cells predicts the clinical efficacy of PD-1 blockade therapies. Nat Immunol. 2020 Nov; 21(11): 1346-1358. Doi: 10.1038/s41590-020-0769-3). Studies in preclinical animal models and clinical settings have shown that the elimination of Treg cells in the tumor microenvironment can promote anti-tumor immunity and increase the therapeutic effect on PD-1/PD-L1 blockage. Clinically, it has been found that a combined administration of anti-CTLA-4 antibody ipilimumab and the anti-PD-1 antibody nivolumab is more beneficial to the survival of patients with melanoma and renal cell carcinoma (Hayashi H and Nakagawa K., Combination therapy with PD-1 or PD-L1 inhibitors for cancer. Int J Clin Oncol. 2020 May; 25(5):818-830. doi: 10.1007/s10147-019-01548-1).

The Treg-eliminating antibodies currently under clinical development, including antibodies against the targets such as CTLA-4, CD25, co-stimulatory factors OX40 and GITR, have made some progress in clinical practice (Togashi Y et al., Regulatory T cells in cancer immunosuppression - implications for anticancer therapy. Nat Rev Clin Oncol. 2019 Jun; 16(6): 356-371. doi: 10.1038/s41571-019-0175-7). However, since these targets are expressed at high levels in both Treg and effector T cells in the periphery, thus simultaneous elimination of Treg and effector T cells in the periphery results in their reduced clinical efficacy and increased toxicity, limiting their clinical application. In recent years, the chemokine receptor CCR8 has been found to have high expression on Treg cells in human tumors (including breast cancer, non-small cell lung cancer, colorectal cancer, melanoma, hepatocellular carcinoma and pancreatic ductal adenocarcinoma), but have low or no expression on Treg and effector T cells in the peripheral and normal tissues (Plitas G. et al., Regulatory T Cells Exhibit Distinct Features in Human Breast Cancer. Immunity. 2016 Nov 15; 45(5):1122-1134. doi: 10.1016/j.immuni.2016.10.032, as well as De Simone M. et. al., Transcriptional Landscape of Human Tissue Lymphocytes Unveils Uniqueness of Tumor-Infiltrating T Regulatory Cells. Immunity. 2016 Nov 15;45(5):1135-1147. doi: 10.1016/j.immuni.2016.10.021). High expression of CCR8 is associated with poor prognosis of breast cancer, non-small cell lung cancer, and colorectal cancer. Compared to the CCR8-negative Treg cells, the CCR8-positive Treg cells in the tumor microenvironment exert more strong suppression on the proliferation of effector T cells (Gang, Yi et al., Identification and functional analysis of heterogeneous FOXP3+ Treg cell subpopulations in human pancreatic ductal adenocarcinoma [J]. Science Bulletin, 2018. Doi:10.1016/j.scib.2018.05.028). In animal models, it has been found that CCR8 antibodies can specifically eliminate Treg cells in the tumor microenvironment, suppress tumor growth and show a synergistic effect with antibodies against PD-1 (U. S. Patent Application No. US20190071508 and Van Damme H. et al., Therapeutic depletion of CCR8+ tumor-infiltrating regulatory T cells elicits anti-tumor immunity and synergizes with anti-PD-1 therapy. J Immunother Cancer. 2021 Feb; 9(2):e001749. doi: 10.1136/jitc-2020-001749). Development of antibodies specific to human CCR8 may increase the therapeutic effect on immune checkpoints in clinical practice and have important application value.

### Summary of the Invention

The present invention provides anti-human CCR8 specific antibodies obtained by immunizing mice with cells over-expressing human CCR8 and CCR8-coding nucleic acids. Anti-CCR8 antibodies have high affinities for both human- and monkey-CCR8s, can block the binding of CCL1 to CCR8 /signaling pathways induced thereon, and can also eliminate CCR8-positive cells (e.g., Treg cells) through ADCC effect, effectively suppressing tumor growth in various *in vivo* models in mice (such as models of colorectal cancer, lung cancer, breast cancer, melanoma, etc.).

In one aspect, the present invention provides an anti-CCR8 antibody or an antigen-binding fragment, wherein the anti-CCR8 antibody or the antigen-binding fragment thereof comprises:
(i) three complementarity-determining regions HCDR1, HCDR2, and HCDR3 of a heavy-chain variable region as set forth in any of SEQ ID NOs: 21-40, and/or three complementarity-determining regions LCDR1, LCDR2, and LCDR3 of a light-chain variable region as set forth in any of SEQ ID NOs: 42-54;
(ii) a combination of the CDRs as set forth in (i), and wherein, as compared with the HCDR1, the HCDR2, the HCDR3, the LCDR1, the LCDR2, and/or the LCDR3, at least one amino acid addition, substitution or deletion or any combination thereof (e.g. 1, 2, or 3 amino acid additions, substitutions or deletions or any combinations thereof), preferably amino acid substitution(s), and preferably conservative amino acid substitution(s), is further comprised in the sequence(s) thereof, with the affinity for the CCR8 being maintained.

In some embodiments, the present invention provides an antibody that binds to CCR8 or an antigen-binding fragment thereof comprising a heavy-chain variable region and/or a light-chain variable region, wherein
the heavy-chain variable region comprises:
   (1) HCDR1, HCDR2, and HCDR3 having an amino acid sequence as set forth in SEQ ID NO: 1, SEQ ID NO: 2, 10, 11, or 41, and SEQ ID NO: 3, respectively; or HCDR1, HCDR2, and HCDR3 being the same with, or having at least one amino acid addition, substitution or deletion or any combination thereof (e.g. 1, 2, or 3 amino acid additions, substitutions or deletions or any combinations thereof) when compared with the amino acid sequence as set forth in SEQ ID NO: 1, SEQ ID NO: 2, 10, 11, or 41, and SEQ ID NO: 3, respectively; or
   (2) HCDR1, HCDR2, and HCDR3 having an amino acid sequence as set forth in SEQ ID NO: 4, SEQ ID NO: 5, and SEQ ID NO: 6, respectively; or HCDR1, HCDR2, and HCDR3 being the same with, or having at least one amino acid addition, substitution or deletion or any combination thereof (e.g. 1, 2, or 3 amino acid additions, substitutions or deletions or any combinations thereof) when compared with the amino acid sequence as set forth in SEQ ID NO: 4, SEQ ID NO: 5, and SEQ ID NO: 6, respectively; or
   (3) HCDR1, HCDR2, and HCDR3 having an amino acid sequence as set forth in SEQ ID NO: 7, SEQ ID NO: 8, and SEQ ID NO: 9, respectively; or HCDR1, HCDR2, and HCDR3 being the same with, or having at least one amino acid addition, substitution or deletion or any combination thereof (e.g. 1, 2, or 3 amino acid additions, substitutions or deletions or any combinations thereof) when compared with the amino acid sequence as set forth in SEQ ID NO: 7, SEQ ID NO: 8, and SEQ ID NO: 9, respectively;
   and/or
the light-chain variable region comprises:
   (1) LCDR1, LCDR2, and LCDR3 having an amino acid sequence as set forth in SEQ ID NO: 12, SEQ ID NO: 13, and SEQ ID NO: 14, respectively; or LCDR1, LCDR2, and LCDR3 being the same with, or having at least one amino acid addition, substitution or deletion or any combination thereof (e.g. 1, 2, or 3 amino acid additions, substitutions or deletions or any combinations thereof) when compared with the amino acid sequence as set forth in SEQ ID NO: 12, SEQ ID NO: 13, and SEQ ID NO: 14, respectively; or
   (2) LCDR1, LCDR2, and LCDR3 having an amino acid sequence as set forth in SEQ ID NO: 15, SEQ ID NO: 16, and SEQ ID NO: 17, respectively; or LCDR1, LCDR2, and LCDR3 being the same with, or having at least one amino acid addition, substitution or deletion or any combination thereof (e.g. 1, 2, or 3 amino acid additions, substitutions or deletions or any combinations thereof) when compared with the amino acid sequence as set forth in SEQ ID NO: 15, SEQ ID NO: 16, and SEQ ID NO: 17, respectively; or
   (3) LCDR1, LCDR2, and LCDR3 having an amino acid sequence as set forth in SEQ ID NO: 18, SEQ ID NO: 19, and SEQ ID NO: 20, respectively; or LCDR1, LCDR2, and LCDR3 being the same with, or having at least one amino acid addition, substitution or deletion or any combination thereof (e.g. 1, 2, or 3 amino acid additions, substitutions or deletions or any combinations thereof) when compared with the amino acid sequence as set forth in SEQ ID NO: 18, SEQ ID NO: 19, and SEQ ID NO: 20, respectively.

In some embodiments, the present invention provides an antibody that binds to CCR8 or an antigen-binding fragment thereof, comprising HCDR1, HCDR2, HCDR3, LCDR1, LCDR2, and LCDR3 as shown in any one of the combinations in the table below:

| Combination# | HCDR1 comprising or consisting of the following sequence (or a sequence having at least one amino acid difference therefrom) (SEQ ID NO: ) | HCDR2 comprising or consisting of the following sequence (or a sequence having at least one amino acid difference therefrom) (SEQ ID NO: ) | HCDR3 comprising or consisting of the following sequence (or a sequence having at least one amino acid difference therefrom) (SEQ ID NO: ) | LCDR1 comprising or consisting of the following sequence (or a sequence having at least one amino acid difference therefrom) (SEQ ID NO: ) | LCDR2 comprising or consisting of the following sequence (or a sequence having at least one amino acid difference therefrom) (SEQ ID NO: ) | LCDR3 comprising or consisting of the following sequence (or a sequence having at least one amino acid difference therefrom) (SEQ ID NO: ) |
|---|---|---|---|---|---|---|
| **1** | 1 | 2 | 3 | 12 | 13 | 14 |
| **2** | 1 | 10 | 3 | 12 | 13 | 14 |
| **3** | 1 | 11 | 3 | 12 | 13 | 14 |
| **4** | 1 | 41 | 3 | 12 | 13 | 14 |
| **5** | 4 | 5 | 6 | 15 | 16 | 17 |
| **6** | 7 | 8 | 9 | 18 | 19 | 20 |

In some embodiments, the present invention provides an antibody that binds to CCR8 or an antigen-binding fragment thereof comprising a heavy-chain variable region VH and/or a light-chain variable region VL, wherein,
(a) the heavy-chain variable region VH
   (i) comprises or consists of an amino acid sequence having at least 90%, 91%, 92%, 93%, 94%, 95%, 96%, 97%, 98%, or 99% identity to the amino acid sequence as set forth in any one selected from SEQ ID NOs: 21-40; or
   (ii) comprises or consists of the amino acid sequence as set forth in any one selected from SEQ ID NOs: 21-40; or
   (iii) comprises an amino acid sequence having at least one amino acid addition, substitution or deletion or any combination thereof (e.g. 1, 2, or 3 amino acid additions, substitutions or deletions or any combinations thereof) in comparison to the amino acid sequence as set forth in any one selected from SEQ ID NOs: 21-40, where preferably, said amino acid change occurs in the CDRs, and preferably, said amino acid change occurs in the framework regions (FRs).
   and/or
(b) light-chain variable region VL
   (i) comprises or consists of an amino acid sequence having at least 90%, 91%, 92%, 93%, 94%, 95%, 96%, 97%, 98%, or 99% identity to the amino acid sequence as set forth in any one selected from SEQ ID NOs: 42-54;
   (ii) comprises or consists of the amino acid sequence as set forth in any one selected from SEQ ID NOs: 42-54; or
   (iii) comprises an amino acid sequence having at least one amino acid addition, substitution or deletion or any combination thereof (e.g. 1, 2, or 3 amino acid additions, substitutions or deletions or any combinations thereof) in comparison to the amino acid sequence as set forth in any one selected from SEQ ID NOs: 42-54, where preferably, said amino acid change does not occur in the CDRs, and preferably, said amino acid change occurs in the FRs.

In some embodiments, the present invention provides an antibody that binds to CCR8 or an antigen-binding fragment thereof, comprising a heavy-chain variable region VH and a light-chain variable region VL as set forth in any one of the combinations in the table below:

| Combina tion# | a VH compri sing or consisti ng of the followi ng sequence (or a sequen ce having at least one amino acid differe nce therefr om) (SEQ ID NO:) | a VLcompr ising or consistin g of the following sequence (or a sequence having at least one amino acid difference therefrom ) (SEQ ID NO:) | Combina tion# | a VH compri sing or consisti ng of the followi ng sequen ce (or a sequen ce having at least one amino acid differe nce therefr om) (SEQ ID NO:) | a VL compri sing or consisti ng of the followi ng sequen ce (or a sequen ce having at least one amino acid differe nce therefr om) (SEQ ID NO:) | Combina tion# | a VH compri sing or consisti ng of the followi ng sequen ce (or a sequen ce having at least one amino acid differe nce therefr om) (SEQ ID NO:) | a VL compri sing or consisti ng of the followi ng sequen ce (or a sequen ce having at least one amino acid differe nce therefr om) (SEQ ID NO:) |
|---|---|---|---|---|---|---|---|---|
| **1** | 21 | 42 | **16** | 28 | 45 | **31** | 33 | 48 |
| **2** | 21 | 43 | **17** | 28 | 46 | **32** | 33 | 49 |
| **3** | 22 | 42 | **18** | 29 | 44 | **33** | 34 | 47 |
| **4** | 22 | 43 | **19** | 29 | 45 | **34** | 34 | 48 |
| **5** | 23 | 42 | **20** | 29 | 46 | **35** | 34 | 49 |
| **6** | 23 | 43 | **21** | 30 | 44 | **36** | 35 | 47 |
| **7** | 24 | 42 | **22** | 30 | 45 | **37** | 35 | 48 |
| **8** | 24 | 43 | **23** | 30 | 46 | **38** | 35 | 49 |
| **9** | 25 | 42 | **24** | 31 | 44 | **39** | 36 | 50 |
| **10** | 25 | 43 | **25** | 31 | 45 | **40** | 37 | 51 |
| **11** | 26 | 42 | **26** | 31 | 46 | **41** | 38 | 52 |
| **12** | 26 | 43 | **27** | 32 | 47 | **42** | 39 | 53 |
| **13** | 27 | 42 | **28** | 32 | 48 | **43** | 40 | 54 |
| **14** | 27 | 43 | **29** | 32 | 49 | | | |
| **15** | 28 | 44 | **30** | 33 | 47 | | | |

In some embodiments, the present invention provides an anti-CCR8 antibody or an antigen-binding fragment comprising a heavy chain and/or light chain, wherein
(a) the heavy chain
   (i) comprises or consists of an amino acid sequence having at least 85%, 90%, 91%, 92%, 93%, 94%, 95%, 96%, 97%, 98%, or 99% identity to the amino acid sequence selected from any one of SEQ ID NOs: 55-74;
   (ii) comprises or consists of the amino acid sequence selected from any one of SEQ ID NOs: 55-74; or
   (iii) comprises an amino acid sequence having at least one amino acid addition, substitution or deletion or any combination thereof (e.g. 1, 2, or 3 amino acid additions, substitutions or deletions or any combinations thereof) in comparison to the amino acid sequence as set forth in any one selected from SEQ ID NOs: 55-74, where preferably, said amino acid change does not occur in the CDRs of the heavy-chain variable region, more preferably, said amino acid change does not occur in the heavy-chain variable region, and most preferably, the amino acid change occurs in the heavy-chain constant region;
   and/or
(b) the light chain
   (i) comprises or consists of an amino acid sequence having at least 85%, 90%, 91%, 92%, 93%, 94%, 95%, 96%, 97%, 98%, or 99% identity to the amino acid sequence selected from any one of SEQ ID NOs: 75-82;
   (ii) comprises or consists of the amino acid sequence selected from any one of SEQ ID NOs: 75-82; or
   (iii) comprises an amino acid sequence having at least one amino acid addition, substitution or deletion or any combination thereof (e.g. 1, 2, or 3 amino acid additions, substitutions or deletions or any combinations thereof) in comparison to the amino acid sequence as set forth in any one selected from SEQ ID NOs: 75-82, where preferably, said amino acid change does not occur in the CDRs of the light-chain variable region, more preferably, said amino acid change does not occur in the light-chain variable region, and most preferably, the amino acid change occurs in the light-chain constant region.

In some embodiments, provided is an antibody or an antigen-binding fragment thereof of the invention, where the antibody or the antigen-binding fragment thereof comprises a heavy-chain constant region and/or a light-chain constant region; preferably, the light-chain constant region is the λ- or κ-chain constant region and the heavy-chain constant region is selected from mouse mIgG2a, human IgG1, human IgG2, human IgG3, or IgG4 subclass. In some preferred embodiments, the heavy-chain constant region is of the human IgG1 subclass or of the human IgG4 subclass with a S228P mutation.

In some embodiments, provided is an antibody or antigen-binding fragment thereof of the invention, wherein the antibody or the antigen-binding fragment thereof comprises a heavy-chain constant region, the sequence of which has one or more amino acid substitutions compared to that of a native human heavy-chain constant region, and preferably, the one or more amino acid substitutions enhance the ADCC effect of the antibody. In some preferred embodiments, the one or more amino acid substitutions occur at one or more of the following sites in the sequence of the heavy-chain constant region: 238, 239, 248, 249, 252, 254, 255, 256, 258, 265, 267, 268, 269, 270, 272, 276, 278, 280, 283, 285, 286, 289, 290, 292, 293, 294, 295, 296, 298, 301, 303, 305, 307, 309, 312, 315, 320, 322, 324, 326, 327, 328, 329, 330, 331, 333, 334, 335, 337, 338, 340, 360, 373, 376, 378, 382, 388, 389, 398, 414, 416, 419, 430, 434, 435, 437, 438, and 439, numbered according to EU numbering system. In some preferred embodiments, the one or more amino acid substitutions occur at one or more of the following sites in the sequence of the heavy-chain constant region: L234, L235, G236, S239, F243, T256, D265, H268, D270, K290, R292, S298, Y300, V305, K326, A330, I332, E333, K334, A339, and P396, numbered according to the EU numbering system. In some preferred embodiments, the one or more amino acid substitutions are selected from one or more of the substitutions G236A, S239D, F243L, T256A, K290A, R292P, S298A, Y300L, V305I, A330L, I332E, E333A, K334A, A339T, and P396L, numbered according to the EU numbering system. In some preferred embodiments, the one or more amino acid substitutions are selected from one or more of N297A substitution, N297Q substitution, L235A together with L237A substitutions, L234A together with L235A substitutions, E233P substitution, L234V substitution, L235A substitution, C236 deletion, P238A substitution, D265A substitution, A327Q substitution, and P329A substitution, numbered according to theEU numbering system. In some preferred embodiments, the one or more amino acid substitutions occur at one or more of the following sites in the sequence of the heavy-chain constant region: 235, 239, 243, 292, 300, 330, 332, and 396 , numbered according to the EU numbering system. In some preferred embodiments, the at least one amino acid substitution is selected from at least one of the substitutions S239D, L235V, F243L, R292P, Y300L, A330L, I332E, and P396L, numbered according to the EU numbering system.

In some preferred embodiments, the heavy-chain constant region has one or more sets of simultaneous mutations at the following combinations of sites selected from: (1) L235/F243/R292/Y300/P396, (2) F243/R292/Y300/V305/P396, (3) D270/K326/A330/K334, (4) S239/A330/I332, (5) S298/E333/K334, (6) L234/L235/G236/S239/H268/D270/S298, (7) M252/S254/T256, (8) L234/L235/D265, (9) G236/S239/I332, and (10) S239/I332, numbered according to the EU numbering system.

In some preferred embodiments, the heavy-chain constant region has one or more sets of the mutations selected from the following mutation combinations: (1) L235V/F243L/R292P/Y300L/P396L, (2) F243L/R292P/Y300L/V305I/P396L, (3) D270E/K326D/A330M/K334E, (4) S239D/A330L/I332E, (5) S298A/E333A/K334A, (6) L234Y/L235Q/G236W/S239M/H268D/D270E/S298A, (7) M252Y/S254T/T256E, (8) L234A/L235A/D265A, (9) L234F/L235E/D265A, (10) G236A/S239D/I332E, and (11) S239D/I332E, numbered according to the EU numbering system.

In some embodiments, provided is an antibody that binds to CCR8 or an antigen-binding fragment thereof, comprising a heavy chain (HC) and a light chain (LC) as set forth in any one of the combinations in the table below:

| Combinat ion# | a heavy chain compri sing or consisti ng of the follow ing sequen ce (or a sequen ce having at least one amino acid differen ce therefr om) (SEQ ID NO: ) | a light chain compri sing or consisti ng of the followi ng sequen ce (or a sequen ce having at least one amino acid differen ce therefr om) (SEQ ID NO: ) | Combinat ion# | a heavy chain compri sing or consisti ng of the followi ng sequen ce (or a sequen ce having at least one amino acid differen ce therefr om) (SEQ ID NO: ) | a light chain compri sing or consisti ng of the followi ng sequen ce (or a sequen ce having at least one amino acid differen ce therefr om) (SEQ ID NO: ) | Combinat ion# | a heavy chain compri sing or consisti ng of the followi ng sequen ce (or a sequen ce having at least one amino acid differen ce therefr om) (SEQ ID NO: ) | a light chain compri sing or consisti ng of the followi ng sequen ce (or a sequen ce having at least one amino acid differen ce therefr om) (SEQ ID NO: ) |
|---|---|---|---|---|---|---|---|---|
| **1** | 55 | 75 | **16** | 60 | 75 | **31** | 68 | 77 |
| **2** | 62 | 75 | **17** | 60 | 76 | **32** | 68 | 78 |
| **3** | 63 | 75 | **18** | 61 | 75 | **33** | 68 | 79 |
| **4** | 64 | 75 | **19** | 61 | 76 | **34** | 71 | 80 |
| **5** | 55 | 76 | **20** | 65 | 77 | **35** | 71 | 81 |
| **6** | 62 | 76 | **21** | 65 | 78 | **36** | 71 | 82 |
| **7** | 63 | 76 | **22** | 65 | 79 | **37** | 72 | 80 |
| **8** | 56 | 75 | **23** | 66 | 77 | **38** | 72 | 81 |
| **9** | 56 | 76 | **24** | 66 | 78 | **39** | 72 | 82 |
| **10** | 57 | 75 | **25** | 69 | 78 | **40** | 73 | 80 |
| **11** | 57 | 76 | **26** | 70 | 78 | **41** | 73 | 81 |
| **12** | 58 | 75 | **27** | 66 | 79 | **42** | 73 | 82 |
| **13** | 58 | 76 | **28** | 67 | 77 | **43** | 74 | 80 |
| **14** | 59 | 75 | **29** | 67 | 78 | **44** | 74 | 81 |
| **15** | 59 | 76 | **30** | 67 | 79 | **45** | 74 | 82 |

In some embodiments, the constant regions of the antibodies of the invention are afucosylated or less-fucosylated.

In some embodiments, the antibodies of the invention are monoclonal antibodies.

In some embodiments, the antibodies of the invention are murine-derived antibodies, chimeric antibodies, or humanized antibodies or human antibodies.

In some embodiments, the antigen-binding fragments of the invention include the following antibody fragment: a Fab, a Fab', a Fd, a Fab'-SH, a Fv, a single chain antibody (for instance, a scFv) or a (Fab')₂, a single-domain antibody, a diabody (dAb) or a linear antibody.

In another aspect, the present invention provides an isolated anti-CCR8 antibody or antigen-binding fragment thereof having one or more of the following characteristics:
(1) binding to the same or completely or partially overlapping epitopes on the human CCR8 protein as any of the anti-CCR8 antibodies or antigen-binding fragments thereof of the invention;
(2) competing with any of the anti-CCR8 antibodies or antigen-binding fragments thereof of the invention to bind to an epitope on the human CCR8 protein;
(3) displaying the same or similar binding affinity and/or specificity as the antibody of the invention to CCR8;
(4) having one or more biological characteristics of the antibody of the invention.

In some embodiments, provides is an antibody or antigen-binding fragment thereof of the invention having one or more of the following characteristics:
(1) binding to human/cynomolgus CCR8 with a high affinity, for example, exhibiting an EC₅₀ value not exceeding 1000 ng/mL, an EC₅₀ value not exceeding 950 ng/mL, an EC₅₀ value not exceeding 900 ng/mL, an EC₅₀ value not exceeding 850 ng/mL, an EC₅₀ value not exceeding 800 ng/mL, an EC₅₀ value not exceeding 750 ng/mL, an EC₅₀ value not exceeding 700 ng/mL, an EC₅₀ value not exceeding 650 ng/mL, an EC₅₀ value not exceeding 600 ng/mL, an EC₅₀ value not exceeding 550 ng/mL, an EC₅₀ value not exceeding 500 ng/mL, an EC₅₀ value not exceeding 450 ng/mL, an EC₅₀ value not exceeding 400 ng/mL, an EC₅₀ value not exceeding 350 ng/mL, an EC₅₀ value not exceeding 300 ng/mL, an EC₅₀ value not exceeding 250 ng/mL, an EC₅₀ value not exceeding 200 ng/mL, an EC₅₀ value not exceeding 180 ng/mL, an EC₅₀ value not exceeding 160 ng/mL, an EC₅₀ value not exceeding 150 ng/mL, an EC₅₀ value not exceeding 140 ng/mL, an EC₅₀ value not exceeding 130 ng/mL, an EC₅₀ value not exceeding 120 ng/mL, an EC₅₀ value not exceeding 110 ng/mL, an EC₅₀ value not exceeding 100 ng/mL, an EC₅₀ value not exceeding 95 ng/mL, an EC₅₀ value not exceeding 90 ng/mL, an EC₅₀ value not exceeding 85 ng/mL, an EC₅₀ value not exceeding 80 ng/mL, an EC₅₀ value not exceeding 75 ng/mL, an EC₅₀ value not exceeding 70 ng/mL, an EC₅₀ value not exceeding 65 ng/mL, an EC₅₀ value not exceeding 60 ng/mL, an EC₅₀ value not exceeding 55 ng/mL, an EC₅₀ value not exceeding 50 ng/mL, an EC₅₀ value not exceeding 45 ng/mL, an EC₅₀ value not exceeding 40 ng/mL, an EC₅₀ value not exceeding 35 ng/mL, an EC₅₀ value not exceeding 30 ng/mL, an EC₅₀ value not exceeding 25 ng/mL, an EC₅₀ value not exceeding 20 ng/mL, an EC₅₀ value not exceeding 18 ng/mL, an EC₅₀ value not exceeding 16 ng/mL, an EC₅₀ value not exceeding 15 ng/mL, an EC₅₀ value not exceeding 14 ng/mL, an EC₅₀ value not exceeding 13 ng/mL, an EC₅₀ value not exceeding 12 ng/mL, an EC₅₀ value not exceeding 11 ng/mL, an EC₅₀ value not exceeding 10 ng/mL, an EC₅₀ value not exceeding 8 ng/mL, an EC₅₀ value not exceeding 6 ng/mL, an EC₅₀ value not exceeding 4 ng/mL, an EC₅₀ value not exceeding 2 ng/mL, an EC₅₀ value not exceeding 1 ng/mL, or an even lower EC₅₀ value.
(2)blocking the binding of human/cynomolgus CCR8 to its ligand (e.g., CCL1), for example, exhibiting an IC₅₀ value not exceeding 1000 ng/mL, an IC₅₀ value not exceeding 950 ng/mL, an IC₅₀ value not exceeding 900 ng/mL, an IC₅₀ value not exceeding 850 ng/mL, an IC₅₀ value not exceeding 800 ng/mL, an IC₅₀ value not exceeding 750 ng/mL, an IC₅₀ value not exceeding 700 ng/mL, an IC₅₀ value not exceeding 650 ng/mL, an IC₅₀ value not exceeding 600 ng/mL, an IC₅₀ value not exceeding 550 ng/mL, an IC₅₀ value not exceeding 500 ng/mL, an IC₅₀ value not exceeding 450 ng/mL, an IC₅₀ value not exceeding 400 ng/mL, an IC₅₀ value not exceeding 350 ng/mL, an IC₅₀ value not exceeding 300 ng/mL, an IC₅₀ value not exceeding 250 ng/mL, an IC₅₀ value not exceeding 200 ng/mL, an IC₅₀ value not exceeding 180 ng/mL, an IC₅₀ value not exceeding 160 ng/mL, an IC₅₀ value not exceeding 150 ng/mL, an IC₅₀ value not exceeding 140 ng/mL, an IC₅₀ value not exceeding 130 ng/mL, an IC₅₀ value not exceeding 120 ng/mL, an IC₅₀ value not exceeding 110 ng/mL, an IC₅₀ value not exceeding 100 ng/mL, an IC₅₀ value not exceeding 95 ng/mL, an IC₅₀ value not exceeding 90 ng/mL, an IC₅₀ value not exceeding 85 ng/mL, an IC₅₀ value not exceeding 80 ng/mL, an IC₅₀ value not exceeding 75 ng/mL, an IC₅₀ value not exceeding 70 ng/mL, an IC₅₀ value not exceeding 65 ng/mL, an IC₅₀ value not exceeding 60 ng/mL, an IC₅₀ value not exceeding 55 ng/mL, an IC₅₀ value not exceeding 50 ng/mL, an IC₅₀ value not exceeding 45 ng/mL, an IC₅₀ value not exceeding 40 ng/mL, an IC₅₀ value not exceeding 35 ng/mL, an IC₅₀ value not exceeding 30 ng/mL, an IC₅₀ value not exceeding 25 ng/mL, an IC₅₀ value not exceeding 20 ng/mL, an IC₅₀ value not exceeding 18 ng/mL, an IC₅₀ value not exceeding 16 ng/mL, an IC₅₀ value not exceeding 15 ng/mL, an IC₅₀ value not exceeding 14 ng/mL, an IC₅₀ value not exceeding 13 ng/mL, an IC₅₀ value not exceeding 12 ng/mL, an IC₅₀ value not exceeding 11 ng/mL, an IC₅₀ value not exceeding 10 ng/mL, an IC₅₀ value not exceeding 8 ng/mL, an IC₅₀ value not exceeding 6 ng/mL, an IC₅₀ value not exceeding 4 ng/mL, an IC₅₀ value not exceeding 2 ng/mL, an IC₅₀ value not exceeding 1 ng/mL, or an even lower IC₅₀ value.
(3)blocking CCL1-induced β-arrestin recruitment, for example, exhibiting an IC₅₀ value not exceeding 10000 ng/mL, an IC₅₀ value not exceeding 9500 ng/mL, an IC₅₀ value not exceeding 9000 ng/mL, an IC₅₀ value not exceeding 8500 ng/mL, an IC₅₀ value not exceeding 8000 ng/mL, an IC₅₀ value not exceeding 7500 ng/mL, an IC₅₀ value not exceeding 7000 ng/mL, an IC₅₀ value not exceeding 6500 ng/mL, an IC₅₀ value not exceeding 6000 ng/mL, an IC₅₀ value not exceeding 5500 ng/mL, an IC₅₀ value not exceeding 5000 ng/mL, an IC₅₀ value not exceeding 4500 ng/mL, an IC₅₀ value not exceeding 4000 ng/mL, an IC₅₀ value not exceeding 3500 ng/mL, an IC₅₀ value not exceeding 3000 ng/mL, an IC₅₀ value not exceeding 2500 ng/mL, an IC₅₀ value not exceeding 2000 ng/mL, an IC₅₀ value not exceeding 1800 ng/mL, an IC₅₀ value not exceeding 1600 ng/mL, an IC₅₀ value not exceeding 1500 ng/mL, an IC₅₀ value not exceeding 1400 ng/mL, an IC₅₀ value not exceeding 1300 ng/mL, an IC₅₀ value not exceeding 1200 ng/mL, an IC₅₀ value not exceeding 1100 ng/mL, an IC₅₀ value not exceeding 1000 ng/mL, an IC₅₀ value not exceeding 950 ng/mL, an IC₅₀ value not exceeding 900 ng/mL, an IC₅₀ value not exceeding 850 ng/mL, an IC₅₀ value not exceeding 800 ng/mL, an IC₅₀ value not exceeding 750 ng/mL, an IC₅₀ value not exceeding 700 ng/mL, an IC₅₀ value not exceeding 650 ng/mL, an IC₅₀ value not exceeding 600 ng/mL, an IC₅₀ value not exceeding 550 ng/mL, an IC₅₀ value not exceeding 500 ng/mL, an IC₅₀ value not exceeding 450 ng/mL, an IC₅₀ value not exceeding 400 ng/mL, an IC₅₀ value not exceeding 350 ng/mL, an IC₅₀ value not exceeding 300 ng/mL, an IC₅₀ value not exceeding 250 ng/mL, an IC₅₀ value not exceeding 200 ng/mL, an IC₅₀ value not exceeding 180 ng/mL, an IC₅₀ value not exceeding 160 ng/mL, an IC₅₀ value not exceeding 150 ng/mL, an IC₅₀ value not exceeding 140 ng/mL, an IC₅₀ value not exceeding 130 ng/mL, an IC₅₀ value not exceeding 120 ng/mL, an IC₅₀ value not exceeding 110 ng/mL, an IC₅₀ value not exceeding 100 ng/mL, an IC₅₀ value not exceeding 80 ng/mL, an IC₅₀ value not exceeding 60 ng/mL, an IC₅₀ value not exceeding 40 ng/mL, an IC₅₀ value not exceeding 20 ng/mL, an IC₅₀ value not exceeding 10 ng/mL, or an even lower IC₅₀ value.

The present invention also provides a multi-specific antibody comprising a light-chain variable region and/or a heavy-chain variable region of the antibody or the antigen-binding fragment thereof described herein.

The present invention also provides a single-chain antibody comprising a light-chain variable region and a heavy-chain variable region of the antibody or the antigen-binding fragment thereof described herein.

The present invention also provides an immunoconjugate comprising the antibody or the antigen-binding fragment thereof described herein conjugated to a therapeutic agent or a diagnostic agent.

In yet another aspect, the present invention provides a polynucleotide molecule encoding any of the anti-CCR8 antibodies or any fragments thereof described herein.

In another aspect, the present invention provides an expression vector comprising the polynucleotide molecule described in the invention, and the vector is preferably a eukaryotic expression vector.

In another aspect, the present invention provides a host cell comprising the polynucleotide molecule described in the invention or the expression vector described in the invention. Preferably, the host cell is a eukaryotic cell, more preferably a mammalian cell.

In yet another aspect, the present invention provides a method for preparing an anti-CCR8 antibody or an antigen-binding fragment thereof described herein, the method comprising expressing the antibody or the antigen-binding fragment thereof in a host cell as described herein under the condition suitable for expressing the antibody or the antigen-binding fragment thereof, and recovering the expressed antibody or the antigen-binding fragment thereof from the host cell.

In yet another aspect, the present invention provides a pharmaceutical composition comprising the anti-CCR8 antibodies or antigen-binding fragments thereof, polynucleotides, vectors, host cells, immunoconjugates described herein, and optionally at least one pharmaceutically acceptable carrier or excipient.

In yet another aspect, the present invention provides a pharmaceutical combination comprising the antibodies or antigen-binding fragments thereof, polynucleotides, vectors, host cells, immunoconjugates, or pharmaceutical compositions as described herein, and one or more additional therapeutic agents. In some embodiments, the additional therapeutic agent is a chemotherapeutic agent. In some embodiments, the additional therapeutic agent is other antibodies. In some embodiments, the additional therapeutic agent is another monoclonal antibody. In some preferred embodiments, the additional therapeutic agent is a monoclonal antibody targeting an immune checkpoint. In some more preferred embodiments, the additional therapeutic agent is a monoclonal antibody targeting PD-1. In some more preferred embodiments, the additional therapeutic agent is a monoclonal antibody targeting PD-L1. In some more preferred embodiments, the additional therapeutic agent is a monoclonal antibody targeting CTLA-4.

In yet another aspect, the present invention provides a method for eliminating CCR8-positive Treg cells *in vitro* or *in vivo,* comprising exposure of the anti-CCR8 antibodies or antigen-binding fragments thereof, polynucleotides, vectors, host cells, immunoconjugates or pharmaceutical compositions described herein to a cell population comprising the CCR8-positive Treg cells, or administration of the same into the body of a subject.

In yet another aspect, the present invention provides use of the antibodies or antigen-binding fragments thereof, polynucleotides, vectors, host cells, immunoconjugates, pharmaceutical compositions or pharmacological combinations described herein in preparation of a medicament for treating and/or preventing tumors,autoimmune or infectious diseases, the tumor being preferably melanoma, breast cancer, colon carcinoma, rectal cancer, colorectal cancer, gastric cancer, esophageal cancer, head and neck cancer, lung cancer, ovarian cancer, kidney cancer, bladder cancer, liver cancer or thymus cancer.

In some embodiments, the present invention provides use of the antibodies or antigen-binding fragments thereof, polynucleotides, vectors, host cells, immunoconjugates or pharmaceutical compositions described herein in combination with other therapeutic agents in preparation of a medicament for treating and/or preventing tumors, autoimmune or infectious diseases, the tumor being preferably melanoma, breast cancer, colon carcinoma, rectal cancer, colorectal cancer, gastric cancer, esophageal cancer, head and neck cancer, lung cancer, ovarian cancer, kidney cancer, bladder cancer, liver cancer or thymus cancer. In some embodiments, the additional therapeutic agent is a chemotherapeutic agent. In some embodiments, the additional therapeutic agent is another antibody. In some embodiments, the additional therapeutic agent is another monoclonal antibody. In some preferred embodiments, the additional therapeutic agent is a monoclonal antibody targeting an immune checkpoint. In some more preferred embodiments, the additional therapeutic agent is a monoclonal antibody targeting PD-1. In some more preferred embodiments, the additional therapeutic agent is a monoclonal antibody targeting PD-L1. In some more preferred embodiments, the additional therapeutic agent is a monoclonal antibody targeting CTLA4.

In yet another aspect, the present invention provides the antibodies or antigen-binding fragments thereof, polynucleotides, vectors, host cells, immunoconjugates, pharmaceutical compositions or pharmacological combinations described herein for use in treating and/or preventing tumors,autoimmune or infectious diseases, the tumor being preferably melanoma, breast cancer, colon carcinoma, rectal cancer, colorectal cancer, gastric cancer, esophageal cancer, head and neck cancer, lung cancer, ovarian cancer, kidney cancer, bladder cancer, liver cancer or thymus cancer.

In yet another aspect, the present invention provides a method for treating and/or preventing tumors, autoimmune or infectious diseases, comprising administering to a subject in need hereof a therapeutically or prophylactically effective amount of the antibodies or antigen-binding fragments thereof, polynucleotides, vectors, host cells, immunoconjugates, pharmaceutical compositions or pharmacological combinations described herein. Preferably, the tumor is melanoma, breast cancer, colon carcinoma, rectal cancer, colorectal cancer, gastric cancer, esophageal cancer, head and neck cancer, lung cancer, ovarian cancer, kidney cancer, bladder cancer, liver cancer or thymus cancer.

In yet another aspect, the present invention provides a kit comprising the antibodies or antigen-binding fragments thereof, polynucleotides, vectors, host cells, immunoconjugates, pharmaceutical compositions or pharmacological combinations described herein, preferably further comprising a drug delivery device.

In another aspect, the present invention provides a method for detecting the presence of CCR8 in a sample using an antibody, an antigen-binding fragment thereof or an immunoconjugate described herein or a detection composition containing the antibody, the antigen-binding fragment thereof or the immunoconjugate.

### BRIEF DESCRIPTION OF THE DRAWINGS

Fig. 1. The binding activity of the anti-CCR8 antibody of the invention to human CCR8 is shown. A-D are the binding curves of purified and quantified antibodies from the monoclonal hybridoma cell culture supernatant to 293F-hCCR8.
Fig. 2. The binding activity of the anti-CCR8 antibody of the invention to cynomolgus CCR8 is shown. A-B are the binding curves of purified and quantified antibodies from the monoclonal hybridoma cell culture supernatant to 293T-cynoCCR8.
Fig. 3. It is shown that multiple antibody clones of the invention can block the binding of CCL1 to CCR8. A-C are the suppression curves showing blockade of 10 nM AlexaFluor-647-labeled human CCL1 binding to 293F-hCCR8 by purified and quantified antibodies from monoclonal hybridoma cell culture supernatant.
Fig. 4. The binding activity of the anti-CCR8 chimeric antibody of the invention to the human CCR8 is shown. A-B and C are the binding curves of the hIgG1- and mIgG2a-subclass chimeric antibodies to 293F-hCCR8, respectively.
Fig. 5. The binding activity of the anti-CCR8 chimeric antibody of the invention to the cynomolgus CCR8 is shown. A and B are the binding curves of the hIgG1- and mIgG2a-subclass chimeric antibodies to 293T-cynoCCR8, respectively.
Fig. 6. The activity of the anti-CCR8 chimeric antibody of the invention blocking the binding to CCL1 is shown. A-B and C are the curves showing blockade of the binding of 10 nM AlexaFluor-647-labeled human CCL1 to 293F-hCCR8 by hIgG1- and mIgG2a-subclass chimeric antibodies, respectively.
Fig. 7. Blockade of CCL1-induced β-arrestin recruitment by the CCR8 chimeric antibody of the invention is shown. A and B are the curves showing blockade of human CCL1-induced β-arrestin recruitment by hIgG1- and mIgG2a-subclass chimeric antibodies, respectively.
Fig. 8. The binding activity of the humanized anti-CCR8 antibody of the invention to the human CCR8 is shown. A-B, C-D, and E-G are the binding curves of the humanized antibodies from the clones 27B9-1G3, 559E1B10, and 563E10E12 to 293F-hCCR8, respectively.
Fig. 9. The binding activity of the anti-CCR8 humanized antibody of the invention to the cynomolgus CCR8 is shown, the binding curve of the 559E1B10- and 563E10E12-derived humanized antibodies to 293T-cynoCCR8.
Fig. 10. Blockade of CCL1 binding activity by the humanized anti-CCR8 antibody of the invention is shown. A-B and C are the curves showing blockade of the binding of 10 nM AlexaFluor-647-labeled human CCL1 to 293F-hCCR8 by 27B9-1G3, 559E1B10, and 563E10E12 derived humanized antibodies, respectively.
Fig. 11. Experiment on blockade of CCL1-induced β-arrestin recruitment by the anti-CCR8 humanized antibody of the invention is shown. A-B and C-E are the curves showing blockade of human CCL1-induced β-arrestin recruitment by the 559E1B10- and 563E10E12-derived humanized antibodies, respectively.
Fig. 12. It is shown that the chimeric antibody and the humanized antibody from 559E1B10 can activate Jurkat-human FcγRIIIa(158V)-NFAT in a dose-dependent manner.
Fig. 13. It is shown that the chimeric antibody and the humanized antibody from 563E10E12 can activate Jurkat-human FcγRIIIa(158V)-NFAT in a dose-dependent manner.
Fig. 14. The binding activity of the anti-CCR8 antibody of the invention to the HuT78 is shown.
Fig. 15. Specific binding of the anti-CCR8 antibody of the invention to the hCCR8 is shown. Binding activities of anti-CCR8 antibodies to CHOK1-hCCR8 (A) and CHO-K1 (B) cells.
Fig. 16. CCR8-dependent activation of Jurkat-human FcγRIIIa(158V)-NFAT by the anti-CCR8 antibody of the invention is shown. The target cells corresponding to Panels A and B are CHOK1-hCCR8 and CHOK1-Blank, respectively.
Fig. 17. Binding of the anti-CCR8 antibody of the invention to the hCCR4-transfectd 293F cells is shown. Binding of the anti-CCR8 antibody to the hCCR4-GFP-transfected 293F cells (A, GFP positive cells) and the blank 293F cells (B, all living cells).
Fig. 18. Activation of Jurkat-human FcγRIIIa(158V)-NFAT by incubation of Fc-mutated or afucosylated humanized antibodies from clones 559E1B10 and 563E10E12 with 293F-human CCR8 is shown. A-C are activation of Jurkat-human FcγRIIIa(158V)-NFAT by incubation of Fc-mutated or afucosylated antibodies 559E1B10_hzH1L1 (A), 563E10E12_hzH1L1_hIgG1 (B) and 563E10E12_hzH1L0_hIgG1(C) with 293F-human CCR8, respectively.
Fig. 19. Activation of Jurkat-human FcγRIIIa(158V)-NFAT by incubation of the Fc-mutated or afucosylated antibody 563E10E12_hzH1L0 with HuT78 is shown.
Fig. 20. ADCC effect induced by healthy human PBMCs and anti-CCR8 antibodies. Healthy human PBMCs were activated with IL-2 and co-incubated with the anti-CCR8 antibodies and CHOK1-hCCR8 cells for 5 hours. Then, the proportion of the PI-positive CHOK1-hCCR8 cells was determined, with the relationship of the proportion to the different antibodies and their concentrations shown in the curve.
Fig. 21. Elimination of Treg cells in the peripheral PBMCs by the anti-CCR8 antibody of the invention is shown. The proportions of Foxp3-positive cells within CD4⁺ T cells (A) and CD8 ⁺T cells (CD3⁺CD4⁻) within CD3⁺ T cells (B) after 96 hours of incubation of IL-2-activated PBMCs with the anti-CCR8 antibody.
Fig. 22. Suppression of MC38 tumor growth by the anti-CCR8 antibody of the invention is shown. CCR8-humanized B-hCCR8 mice were inoculated with the MC38 colon carcinoma cells. The mice were injected subcutaneously with 10 mg/kg of the antibody or the negative control (hIgG1) or with an equal volume of the vehicle control (PBS), administered twice weekly. The changes in tumor volume are shown in the curve.
Fig.23. The changes in the mouse body weight in the MC38 tumor model treated with the anti-CCR8 antibody of the invention are shown.
Fig. 24. Suppression of MC38 tumor growth by Fc-mutated and afucosylated anti-CCR8 antibodies is shown. CCR8-humanized B-hCCR8 mice were inoculated with the MC38 colon carcinoma cells for tumorgenesis. The mice were injected subcutaneously with 10 mg/kg of the anti-CCR8 antibody or an equal volume of the vehicle control (PBS) , administered twice weekly. The changes in tumor volume are shown in the curve.
Fig.25. The changes in the mouse body weight in the MC38 tumor model treated with Fc-mutated and afucosylated anti-CCR8 antibodies are shown.
Fig. 26. Suppression of the tumor growth of breast cancer EMT-6 in mice by the anti-CCR8 antibody of the invention administered as monotherapy and in combination with an anti-mPD-1 antibody is shown. The regimen for each group is 5-10 mg/kg of antibodies (iv), anti-mPD-1 antibody (ip) or an equal volume of vehicle (PBS) (iv), administered twice weekly. The changes in tumor volume are shown in the curve.
Fig.27. The changes in the mouse body weight during the treatment of the EMT-6 tumor model with the anti-CCR8 antibody of the invention are shown.

### Detailed Description

### Definition

The practice of the present invention will employ, unless otherwise indicated, conventional techniques of molecular biology (including recombinant technology), microbiology, cell biology, biochemistry, and immunology, which are all within the skill of the art.

To make the present invention more easily understood, certain technical and scientific terms are specifically defined as follows. Unless otherwise definitively defined in other sections of this specification, the technical and scientific terms or expressions used herein have the meanings commonly understood by ordinary technicians in the field to which the present invention belongs. Regarding definitions and terms in this field, professionals can refer at least in part to the Current Protocols in Molecular Biology (Ausubel). The abbreviations for amino acid residues follow a 3-letter code and/or 1-letter code denoting one of 20 common L-amino acids used in the art. As used herein (including the claims), singular forms include the corresponding plural forms, unless otherwise expressly provided herein.

The term "about" used in combination of a numerical value is intended to encompass the numerical values in a range from a lower limit less than the specified numerical value by 5% to an upper limit greater than the specified numerical value by 5%, including but not limited to ±5%, ±2%, ±1%, and ±0.1%, because these changes are suitable for carrying out the disclosed method.

The term "and/or" should be understood to mean any one of the optional items or a combination of any two or more of the optional items.

As used herein, the term "or" should be understood to have the same meaning as "and/or" defined above. For example, when separating items in a list, "or" or "and/or" should be interpreted as inclusive, i.e., including at least one in the list of quantities or elements, but also including more than one, and optionally, additional item unlisted. Only in the event that the term clearly indicates to the contrary, e.g. "only one" or "indeed one" or in the claims "composed of......", what it will refer to is only one number listed or one element of a list.

Unless clearly indicated the contrary in the context, the word "one" and "one" as used herein should be understood as "at least one".

The terms "CCR8","CC motif chemokine receptor type 8", "chemokine (CC motif) receptor 8", etc. in this specification refer to any of native CCR8s produced by expression of CCR8 in cells. Unless otherwise indicated, the term includes CCR8s of any vertebrate origins, such as mammals (e.g., primates (e.g., human and cynomolgus monkey) and rodents (e.g., mouse and rat)). The exemplary human CCR8 protein is referred to UniProt entry P51685, the exemplary cynomolgus CCR8 protein to UniProt entry G7NYJ2, and the exemplary mouse CCR8 protein to UniProt entry P56484.

Unless otherwise specified, the term "CCR8" includes variants, subtypes, species homologs of the human CCR8 or the CCR8 from other species and analogues having at least one common epitope of CCR8. The term includes unprocessed full-length CCR8 and any form of CCR8 that results from processing in the cell. The term encompasses "full-length" unprocessed CCR8" as well as any form of CCR8 or any fragment thereof that results from processing in the cell, for example, a splice variant or an allelic variant. In one embodiment, CCR8 refers to the a full-length CCR8 from human or cynomolgus monkey or fragments thereof (such as mature fragments thereof lacking the signal peptide).

As used herein, "CCL1" and "CC motif chemokine ligand 1" refer to any native CCL1 produced by expression of the CCL1 gene in cells. As its name suggests, CCL1 belongs to the CC chemokine family and is secreted by activated monocytes/macrophages, T lymphocytes and endothelial cells. Unless otherwise indicated, the term includes CCL1s of any vertebrate origins, such as mammals (e.g., primates (e.g., human and cynomolgus monkey) and rodents (e.g., mouse and rat)).

The term "immune response" refers to the action of, for example, lymphocytes, antigen presenting cells, phagocytic cells, granulocytes, and soluble macromolecules produced by the above cells or the liver (including antibodies, cytokines, and complement) that results in selective damage to, destruction of, or elimination from the human body of invading pathogens, cells, or tissues infected with pathogens, cancerous cells, or, in cases of autoimmunity or pathological inflammation, normal human cells or tissues.

The terms "Treg" and "regulatory T-cell" refers to a class of T cells which exhibit the immunosuppressive effect in immune response and typically suppress or down regulate induction and proliferation of effector T cells. Treg has an complicated influence on cancers, but due to the observation that Treg cells tend to be up-regulated in individuals with cancer and that they appear to be recruited to tumor sites, and many studies have suggested that significant presence of Treg cells in the tumor environment indicate a poor prognosis, so it is generally tend to believe that Treg cells suppress human anti-tumor immunity. Various immunotherapies are being studied for methods of treating cancer by targeting Treg cells. In some embodiments of the invention, the tumor contains tumor-infiltrating Treg cells. In some embodiments, the tumor contains CCR8-expressing cells. In some embodiments, the CCR8-expressing cells is Treg cell. In some embodiments of the invention, effector T cells do not express or barely express CCR8. In some embodiments, the antibodies of the invention completely/partially suppress/eliminate Treg cells. In some embodiments, the antibodies of the invention completely/partially suppress/eliminate Treg cells via ADCC mechanism, and/or block the binding of CCL1 to CCR8, suppress CCL1-induced signaling pathways. In some embodiments, the antibodies of the invention are used to treat cancers by completely/partially suppressing/eliminating Treg cells. In some embodiments, the antibodies of the invention are used to completely/partially suppress/eliminate Treg cells more effectively and/or efficiently than other methods of suppressing/eliminating Treg cells. In some embodiments, the antibodies of the invention are used to treat cancers by completely/partially blocking the binding of CCL1 to CCR8. In some embodiments, the antibodies of the invention are used to completely/partially block the binding of CCL1 and CCR8 more effectively and/or efficiently than other methods of blocking the binding of CCL1 and CCR8. In some more specific embodiments, the effect includes enhanced anti-tumor immune, enhanced immune response against tumor antigens, slowed tumor growth, reduced tumor size, increased secretion of anti-tumor cytokines, increased number or enhanced function of the tumor-infiltrating T effector cells, enhanced long-term memory effect of anti-tumor immunity, or any combination of the aforesaid. In some embodiments, the antibodies of the invention are used to completely/partially suppress/eliminate Treg cells with fewer adverse reactions than other methods of suppressing/eliminating Treg cells. In some more specific embodiments, the adverse reaction is an immune disorder, for example an autoimmune disorder, for example an autoimmune disease.

The term "signaling pathway" or "signaling activity" refers to a biochemical causality generally initiated by a protein-protein interaction such as binding of a growth factor to a receptor, for example, such as binding of CCL1 (ligand) to CCR8 (receptor), resulting in transmission of a signal from one portion of a cell to another portion of the cell. Typically, the transmission comprises specific phosphorylation on one or more tyrosine, serine or threonine residues on one or more proteins in a cascade reaction that leads to signal transduction. The penultimate process often involves intranuclear events leading to changes in gene expression.

The term "active" or "biological activity," or the term "biological properties" or "biological characteristics" is used interchangeably herein, including but not limited to affinity and specificity to epitopes/antigens, ability to neutralize or antagonize the activity of CCR8 *in vivo* or *in vitro,* IC₅₀, *in vivo* stability of antibodies and immunogenic properties of antibodies. Other identifiable biological properties or characteristics of antibodies known in the art include, for example, cross-reactivity (usually, cross-reactivity with non-human homologs of the targeted peptide, or with other proteins or tissues.), and the ability to maintain high expression levels of proteins in mammalian cells. The above-mentioned properties or characteristics are observed, determined or evaluated using technologies known in the art, the technology including but not limited to ELISA, FACS or BIACORE plasmon resonance analysis, unlimited *in vitro* or *in vivo* neutralization assay, receptor binding, cytokine or growth factor production and/or secretion, signal transduction and immunohistochemistry of tissue sections of different origin (including human beings, primate or any other origins).

The term "antibody" refers to any form of the antibody that has the desired biological activity. Therefore, as used in its broadest sense, the term specifically includes, but is not limited to, monoclonal antibodies (including full-length monoclonal antibodies), polyclonal antibodies, multi-specific antibodies (e.g., bispecific antibodies), humanized antibodies, fully human antibodies, chimeric antibodies and camelized single domain antibodies.

The term "isolated antibodies" refers to the purified state of a binding compound and, in this case, means that the molecule is substantially free of other biomolecules, such as nucleic acids, proteins, lipids, sugarsm, or other substances such as cell debris and growth medium. The term "isolated" does not mean complete absence of such substances or absence of water, buffers or salts, unless they exist in amounts that significantly interfere with the experimental or therapeutic use of the binding compounds described herein.

The term "monoclonal antibody" refers to an antibody obtained from a population of substantially homogeneous antibodies, i.e., the individual antibodies comprising the population are identical except for possible naturally occurring mutations that may be found in minor amounts. Monoclonal antibodies are highly specific, being directed against a single antigenic epitope. In contrast, conventional (polyclonal) antibody preparations typically include a large number of antibodies directed against (or specific for) different epitopes. The modifier "monoclonal" indicates the character of the antibody obtained from a substantially homogeneous population of antibodies, and is not to be construed as requiring production of the antibody by any particular method.

The term "full-length antibody" is intended to refer to immunoglobulin molecules comprising at least four polypeptide chains when naturally occurring: two heavy (H) chains and two light (L) chains inter-connected by disulfide bonds. Each heavy chain consists of a heavy-chain variable region (abbreviated herein as VH) and a heavy-chain constant region (abbreviated herein as CH). The heavy-chain constant region consists of three domains: CH1, CH2, and CH3. Each light chain consists of a light-chain variable region (abbreviated herein as VL) and a light-chain constant region. The light-chain constant region consists of one domain, CL. The VH and VL regions can be further subclassfied into complementarity determining regions (CDRs) of hyper-variability and more conservative regions interspersing thereamong, termed framework regions (FR). Each VH or VL region is composed of three CDRs and four FRs arranged from amino-terminus to carboxy-terminus in the following order: FR1, CDR1, FR2, CDR2, FR3, CDR3, FR4. The variable regions of the heavy- and light-chains contain a binding domain that interacts with an antigen. The constant regions of the antibodies can mediate the binding of the immunoglobulin to host tissues or factors, including various cells of the immune system (e.g., effector cells) and the first component (C1q) of the classical complement system.

The term "antigen-biniding fragment" of an antibody ("a parental antibody") includes fragments or derivatives of the antibody, typically including at least one fragment of the antigen binding or variable regions (e.g., one or more CDRs) of the parent antibody, the fragment which retains at least some of the binding specificities of the parent antibody. Examples of binding fragments of antibodies include, but are not limited to, Fab, Fab', F(ab')₂ and Fv fragments well known in the art; diabodies; linear antibodies; single-chain antibody molecules, e.g., sc-Fv; nanobodies (nanobody) and multi-specific antibodies formed from antibody fragments. In some preferred embodiments of the invention, the antigen-binding fragment of the invention is selected from Fab, Fab', F(ab')₂ and F fragments; diabodies; linear antibodies; single-chain antibody molecules, e.g., sc-Fv; nanobodies formed from antibody fragments (nanobody) and multi-specific antibodies. When antigen binding activity is expressed on the basis of molar concentration, the binding fragment or derivative generally retains at least 10% of its antigen binding activity. Preferably, the binding fragment or derivative retains at least 20%, 50%, 70%, 80%, 90%, 95% or 100% or higher of the antigen-binding affinity of the parental antibody. It is also expected that antigen-binding fragments of an antibody may include conservative or non-conservative amino acid substitutions that do not significantly alter their biological activity (referred to as "conservative variant" or "functionally conservative variants"). The term "binding compounds" refers to both antibodies and their binding fragments.

The term "single-chain Fv" or "scFv" antibodies refer to antibody fragments comprising the VH and VL domains of antibodies, wherein these domains are found in a single polypeptide chain. The Fv polypeptide further comprises a polypeptide linker between the VH and VL domains which enables the scFv to form the desired structure for antigen binding.

The term "domain antibody" is an immunologically functional immunoglobulin fragment containing only the heavy or light-chain variable region. In some cases, two or more VH regions are covalently linked via peptide linkers, forming a bivalency domain antibody. Two VH regions of bivalent domain antibodies can target the same or different antigens.

The term "bivalent antibody" includes 2 antigen-binding sites. In some cases, the two antigen-binding sites have the same antigen specificity. However, a bivalent antibody can be bispecific.

The term "diabodies" refers to small antibody fragments having two antigen-binding sites, which fragments comprise a heavy-chain variable domain (VH) connected to a light-chain variable domain (VL) in the same polypeptide chain (VH-VL or VL-VH). By using a linker with is too short to enable pairing between the two domains on the same chain, the domains are forced to pair with the complementary domains of another chain and create two antigen-binding sites.

In the present invention, the term "murine-derived antibody" or "hybridoma antibody" is the anti-CCR8 monoclonal antibody prepared according to the knowledge and skills in the field. In the preparation, CCR8 antigen is injected into the subject, and then the hybridoma expressing the antibody with the desired sequence or functional properties is isolated. Hybridoma technology is a technique in which two types of cells are fused while retaining their main characteristics. These two cells are mouse splenocytes immunized with the antigen and mouse myeloma cells. Mouse splenocytes immunized with specific antigens (B lymphocytes) is characterized mainly in that they have function of antibody secretion but can't be continuously cultured *in vitro,* while mouse myeloma cells can divide and proliferate indefinitely under culture conditions, that is, having so-called immortality. Under the action of a selective medium, only hybrid cells from fusion of B cell with myeloma cells can have the ability to be cultured continuously, forming cell clones that have two characteristics of having function of antibody secretion and maintaining cellular immortality at the same time. In some embodiments, the present invention is performed by immunizing mice with the CCR8 protein, then obtaining splenocytes from the immunized mice and fuse them with myeloma cells to obtain hybridoma cells that are able to express positive antibodies.

The term "chimeric antibody" is an antibody having the variable domain of a first antibody and the constant domain of a second antibody, where the first and second antibodies are from different species. Typically, the variable domain is obtained from antibodies of animals such as rodents ("Parental antibody"), while the constant domain sequences are obtained from human antibodies, which compared to the parent rodent antibodies, make the resultant chimeric antibodies less likely to induce adverse immune responses in human subjects. In some embodiments of the invention, the rodent is a mouse or a rat. In some preferred embodiments of the invention, the affinity of the chimeric antibody for the antigen is not or almost not lower than that of the parent murine antibody.

The term "humanized antibodies" refers to forms of antibodies which contain sequences from human and non-human (e.g., mouse and rat) antibodies. In general, a humanized antibody will comprise substantially all of at least one, typically two, variable domains, in which all or substantially all of the hypervariable loops correspond to those of a non-human immunoglobulin and all or substantially all of the framework (FR) regions are those of a human immunoglobulin sequence. The humanized antibody can optionally comprise at least a portion of a human immunoglobulin constant region (Fc).

The term "fully human antibodies" refers to antibodies that contain exclusively human immunoglobulin protein sequences. The fully human antibody may contain mouse carbohydrate chains if produced in mice, in mouse cells or in hybridomas derived from mouse cells. Likewise, "mouse antibodies" refers to antibodies that contain exclusively mouse immunoglobulin protein sequences. Alternatively, the fully human antibody may contain rat carbohydrate chains if produced in rats, in rat cells or in hybridomas derived from rat cells. Likewise, "rat antibodies" refers to antibodies that contain exclusively rat immunoglobulin protein sequences.

The light chains of antibodies can be classified into two types (called kappa (κ) and lambda (λ)). The heavy chains of antibodies can be classified into five different main classes on the basis of the amino acid sequences of their heavy-chain constant regions, such as IgA, IgD, IgE, IgG and IgM, and of these classes, several ones can be further classified into subclasses, such as, IgG1, IgG2, IgG3 and IgG4, IgA1, and IgA2. "Isotype" antibody refers to the antibody class (e.g., IgM, IgE, IgG such as IgG1, IgG2, or IgG4) which is provided by the heavy-chain constant region genes. Isotype also includes modified versions of one of these classes, where modifications have been made to alter the Fc function, for example, to enhance or reduce effector functions or binding to Fc receptors.

The term "Fc-region" herein is used to define a C-terminal region of an immunoglobulin heavy chain that contains at least a portion of the constant region. The term includes native sequence Fc-regions and variant Fc-regions. In some embodiments, a human IgG heavy chain Fc-region extends from Cys226, or from Pro230, to the carboxyl-terminus of the heavy chain. However, the C-terminal lysine (Lys447) of the Fc-region may or may not be present (numbering in this paragraph is in accordance with EU numbering system, also named EU Index, as Kabat et al., Sequences of Proteins of Immunological Interest, 5th Ed. Public Health Service, National Institutes of Health, Bethesda, MD, 1991).

The term "effector functions" refers to biological activities mediated by the Fc region of an antibody, which vary with the antibody isotype. Effector functions of antibodies include but are not limited to C1q binding and complement dependent cytotoxicity (CDC); Fc receptor binding; antibody-dependent cell-mediated cytotoxicity (ADCC); phagocytosis; opsonization; down regulation of cell surface receptors; B-cell activation, etc.

The term "epitope" refers to a protein determinant capable of specific binding to an antibody. Epitopes usually consist of various chemically active surface molecules such as amino acids or sugar side chains and usually have specific three dimensional structural characteristics, as well as specific charge characteristics. Conformational and nonconformational epitopes are distinguished in that the binding to the former but not the latter is lost in the presence of denaturing solvents.

Terms "cross-reaction" described herein refers to binding to antigen fragments of the same target molecule of human, monkey, and/or muridae (mouse or rat) origins. Therefore, "cross-reaction" should be understood as interspecies reactions between antigen-binding molecules (e.g., antibodies) and molecules in the same class expressed in different species (e.g., CCR8) . The cross-reaction specificity of monoclonal antibodies which recognize human CCR8, monkey CCR8, and/or murine (mouse or rat) CCR8 can be determined by FACS analysis.

"Affinity" or "binding affinity" refers to intrinsic binding affinity which reflects an interaction between members of a binding pair. The affinity of a molecule X for its partner Y can generally be represented by the equilibrium dissociation constant (K_{D}), which is the ratio of dissociation and association rate constants (k_{dis} and kₒₙ, respectively). Affinity can be measured by common methods known in the art. In some embodiments of the invention, surface plasmon resonance (SPR) technique is use to measure affinity, such as the affinity between the antibody of the invention and the antigen.

The term "no binding to" proteins or cells refer to no binding to proteins or cells, or no binding to them with high affinity, i.e., EC₅₀ of binding to proteins or cells being 1.0×10⁻⁷ M or higher, more preferably 1.0×10⁻⁶ M or higher, more preferably 1.0×10⁻⁵ M or higher, more preferably 1.0×10⁻⁴ M or higher, more preferably 1.0×10⁻³ M or higher.

The term "high affinity" for IgG antibodies refers to EC₅₀ of Antigens being 1.0×10⁻⁷ M or lower, preferably 5.0×10⁻⁸ M or lower, more preferably 1.0×10⁻⁸ M or lower, more preferably 5.0×10⁻⁹ M or lower, more preferably 1.0×10⁻⁹ M or lower, more preferably 5.0×10⁻¹⁰ M or lower, and more preferably 1.0×10⁻¹⁰ M or lower. For other antibody subtypes, binding with "high affinity" may vary. For example, binding to IgM subtype with "high affinity" means EC₅₀ is 10⁻⁷ M or lower, preferably 10⁻⁸ M or lower, more preferably 10⁻⁹ M or lower.

The term "blocking" with respect to a IgG antibody means that by administering the antibody, the antibody competes with the original ligand of a receptor, inhibiting the binding and interaction between the receptor and the ligand. This suppression can occur through a variety of mechanisms, including, for example, due to overlapping binding sites on the receptors, and/or changing the affinity for the ligand due to conformational changes of the receptor induced by antibodies, etc. The "functional" antibodies and antibody fragments so called are characterized by having the such properties. In some embodiments, capability of "blocking" refers to EC₅₀ value of 5.0×10⁻⁵ M or lower, 1.0×10⁻⁵ M or lower, 5.0×10⁻⁹ M or lower, 1.0×10⁻⁶ M or lower, preferably 5.0×10⁻⁹ M or lower, more preferably 1.0×10⁻⁷ M or lower, more preferably 5.0×10⁻⁸Mor lower, and more preferably 1.0×10⁻⁸ M or lower.

The term "antibody-dependent cellular cytotoxicity", "antibody-dependent cell-mediated cytotoxicity" or "ADCC" refers to cell-mediated immune defense, in which effector cells in the immune system actively lyses target cells of which surface antigens on cellular membrane bind to antibodies.

The term "complement-dependent cytotoxicity" or "CDC" means the effector functions of IgG and IgM antibodies, when trigger the classic complement pathway upon the antibody binding to the surface antigens, including formation of the membrane attack complex and lysis of target cells.

The terms "nucleic acids", "polynucleotide", "nucleic acid molecules" and "polynucleotide molecules" are used interchangeably herein (unless otherwise indicated in the context) and refer to deoxyribonucleic acid (DNA) or ribonucleic acid RNA (RNA) and polymers thereof in either single- or double-stranded form. Unless expressly limited, the term includes nucleic acids containing known analogs of natural nucleotides that have similar binding properties as the reference nucleic acid and are metabolized in a manner similar to naturally occurring nucleotides (See, U.S. Patents No. 8,278,036 to Kariko et al., which discloses mRNA molecules wherein uridine is replaced by pseudouridine, methods for synthesizing the mRNA molecules and methods for delivering therapeutic proteins in vivo). Unless otherwise indicated, a particular nucleic acid sequence also implicitly encompasses conservatively modified variants thereof (e.g., degenerate codon substitutions), alleles, orthologs, SNPs, and complementary sequences as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res. 19:5081 (1991); Ohtsuka et al., J. Biol. Chem. 260:2605-2608 (1985); and Rossolini et al., Mol. Cell. Probes 8:91-98 (1994)).

"Construct" refers to any recombinant polynucleotide molecule such as a plasmid, cosmid, virus, autonomously replicating polynucleotide molecule, phage, or linear or circular single stranded or double stranded DNA or RNA polynucleotide molecule, derived from any source, capable of integrating into the genome or autonomously replicating, comprising one or more polynucleotide molecules which have been linked to through functional operations (that is, operably linked to). In some preferred embodiments of the invention, the recombinant construct comprises a polynucleotide of the invention operably linked to a transcriptional initiation regulatory sequence that drives and/or direct the transcription of the polynucleotide of the invention in the host cell. The expression of the polynucleotide of the invention can be driven and/or directed with heterologous and non-heterogeneous (that is, endogenous) promoters.

"Vector" refers to any recombinant polynucleotide construct which can be used for transformation purposes (i.e., introducing heterologous DNAs into host cells). One type of vector is a "plasmid", referring to a circular double-stranded DNA loop into which additional DNA segments can be ligate. A further type of vector is a viral vector, it being possible for additional DNA segments to be ligated into the viral genome. Certain vectors are capable of autonomous replication in a host cell into which they are introduced (e.g., bacterial vectors having a bacterial origin of replication and episomal mammalian vectors). After introduction into the host cell, other vectors (e.g., non-episomal mammalian vectors) are integrated into the genome of a host cell, and thereby are replicated along with the host genome. Additionally, certain vectors are capable of directing the expression of genes to which they are operatively linked. Such vectors are referred to herein as "expression vectors".

The term "expression vector" used herein refers to a nucleic acid molecule which can replicate and express a gene of interest when transformed, transfected or transduced into a host cell. The expression vector comprises one or more phenotypic selectable markers and an origin of replication to ensure maintenance of the vector and to, if desirable, provide amplification within the host. In some preferred embodiments of the invention, the expression vector of the invention comprises a construct of the invention and/or a polynucleotide of the invention.

The term "host cell" refers to cells into which exogenous nucleic acid has been introduced, including the progeny of such cells. Host cells include "transformants" and "transformed cells," which include the primary transformed cell and progeny derived therefrom. Host cells are any type of cell system that can be used to produce the antibody molecules of the invention, including eukaryotic cells, e.g., mammalian cells., insect cells, yeast cells; and prokaryotic cells, e.g., *E. coli* cells. Host cells include cultured cells, but also transgenic animals, transgenic plants or cells in cultured plant or animal tissue.

For manipulation of cell or receptors, "activation", "stimulation" and "treatment with" can have the same meaning, for example, activation, stimulation or treatment of a cell or receptor with a ligand, unless otherwise or expressly provided the context. "Ligand" includes natural and synthetic ligands, such as cytokines, cytokine variants, analogs, mutant proteins and antibody-derived binding compounds. "Ligand" also includes small molecules, such as peptide mimetics of cytokines and peptide mimetics of antibodies. "Activation" can refer to cellular activation regulated by internal mechanisms as well as external or environmental factors. "Response/reaction", such as response of cells, tissues, organs or organisms, includes changes in biochemical or physiological behaviors (e.g., concentration in a biological compartment, density, adhesion or migration, gene expression rate or differentiation state), where the changes are associated with activation, stimulation or treatment, or with internal mechanisms such as genetic programming.

In some embodiments of the invention, the term "tumor" is intended to emphasize malignancy, and the terms "cancer" and "tumor" are used interchangeably to refer to any abnormality in an animal, uncontrolled growth or proliferation of cells or tissues. As used herein, the terms "cancer" and "tumor" include solid tumors and hematologic tumors, including pre-neoplastic lesions. Specific non-limiting examples of tumors includebreast cancer, squamous cell carcinoma, small cell lung cancer, pituitary cancer, esophageal cancer, astrocytoma, soft tissue sarcoma, lung cancer (such as non-small cell lung cancer, lung adenocarcinoma, squamous cell carcinoma of the lung), peritoneal cancer, hepatocellular carcinoma, gastric cancer, bowel cancer, pancreatic cancer, glioblastoma, cervical cancer, ovarian cancer, Liver cancer, colon carcinoma, colorectal cancer, endometrial cancer, kidney cancer, prostate cancer, bladder cancer, thyroid cancer, testicular cancer, cholangiocarcinoma, gallbladder cancer, melanoma, mesothelioma, thymoma and various head and neck cancers. In some embodiments, hematologic tumors include B/T-cell mixed leukemia, B-cell lymphoma, leukaemia granulocytic (acute and chronic), leukaemia lymphocytic (acute and chronic), child/Juvenile leukaemia lymphocytic, myelomonocytic leukemia, diffuse large B-cell lymphoma (DLBC), Hodgkin's lymphoma, non-Hodgkin's lymphoma, mantle cell lymphoma (MCL), multiple myeloma, myelodysplastic syndrome, etc. In some embodiments, the tumor is a benign neoplasm.

As used herein, the term "treating" or "healing" of any disease or condition refers in one embodiment to slow down, interrupt, block, ameliorate, stop, reduce, or reverse symptoms, complications or the onset of biochemical symptoms of the disease, relieve symptoms or prevent or suppress further development of a disease, condition or disorder (i.e., slowing or arresting or reducing the progression of a disease or at least one of its clinical symptoms). In another embodiment, "treating" or "healing" refers to relieving or improving at least one physical parameter, including those physical parameters that can't be discernible by the patient. In another embodiment, "treating" or "healing" refers to modulating the disease or disorder, either physically (e.g., stabilization of a discernible symptom), physiologically (e.g., stabilization of a physical parameter), or both. Unless otherwise specified herein, methods for assessing treatment and/or prevention of diseases are generally known in the art.

"Subject" includes any human or non-human animal. The term "non-human animal" includes all vertebrates, e.g. mammals and non-mammals, such as nonhuman primates, sheep, dogs, cats, horses, bovines, chickens, amphibians, reptiles, etc. As used herein, the term "cyno" or "cynomolgus" refers to the cynomolgus monkey or derived from cynomolgus monkey.

Administration "in combination with" one or more other therapeutic agents includes concurrent (concomitant) administration and continuous administration in any order.

"Therapeutically effective amount", "therapeutic effective dose" and "effective amount" refer to an amount of the CCR8 antibody or antigen-binding fragment thereof of the invention, when administered alone or in combination with other therapeutic agents to cells, tissues or subjects, effective to prevent or ameliorate the symptoms of one or more diseases or conditions or the progression of the disease or condition. A therapeutically effective dose also refers to an amount of an antibody or antigen-binding fragment thereof sufficient to result in an improvement in symptoms, e.g., treat, cure, prevent or ameliorate related medical conditions or increase the speed of treating, curing, preventing, or ameliorating such conditions. When an active ingredient to be given alone is administered to an individual, a therapeutically effective dose refers only to that ingredient. When administered in combination, a therapeutically effective dose refers to the combined amounts of the active ingredients that elicit the therapeutic effect, regardless of the combination, sequential or concurrent administration. An effective amount of a therapeutic agent will result in an increase in a diagnostic criterion or parameter by at least 10%, usually at least 20%, preferably at least about 30%, more preferably at least 40%, most preferably at least 50%.

"Pharmaceutically acceptable carrier" refers to ingredients in a pharmaceutical formulation or composition other than the active ingredient that are non-toxic to the subject. Pharmaceutically acceptable carriers include, but are not limited to, buffers, excipients, stabilizers or preservatives.

### Anti-CCR8 antibody

In one aspect, the present invention provides antibodies or antigen binding fragments thereof that specifically bind to CCR8. The terms "anti-CCR8 antibody", "anti-CCR8", "CCR8 antibody" and "an antibody that binds to CCR8" refer to an antibody that is capable of binding to CCR8 protein or a fragment thereof with sufficient avidity such that the antibody can be used as a diagnostic and/or therapeutic agent in targeting CCR8.

In some embodiments, the antibodies of the invention bind to human or cynomolgus CCR8 protein. In some embodiments, the antibodies of the invention bind to CHOK1-humanCCR8 cells or 293F-cynoCCR8 cells. In some embodiments, the antibodies of the invention inhibit/block the binding of CCR8 to its ligand CCL1. In some embodiments, the antibodies of the invention inhibit/block the induced β-arrestin recruitment.

Any suitable method for producing antibodies can be used to produce the antibodies of the invention. Any suitable forms of CCR8s can be used as immunogens (antigens) to produce antibodies. By way of example and not limitation, any CCR8 variants or fragments thereof can be used as immunogens. In some embodiments, hybridoma cells producing monoclonal anti-CCR8 antibodies of murine origin can be generated by methods known in the art.

Antibodies from rodents (e.g., mouse) may cause undesired antibody immunogenicity when used as therapeutic drugs *in vivo.* Repeated use of the antibodies may lead to generation of immune responses against the therapeutic antibodies by the human body. Such immune responses may at least lead to loss of therapeutic efficacy, and in severe cases, may lead to potentially fatal allergic reactions. One approach to reduce the immunogenicity of rodent antibodies includes the generation of chimeric antibodies, in which mouse variable regions are fused with human constant regions (Liu et al., (1987) Proc. Natl. Acad. Sci. USA 84:3439-43). However, retention of intact rodent variable regions in chimeric antibodies may still cause deleterious immunogenicity in patients. Grafting the complementarity determining region (CDR) loop of the rodent variable domain onto a human framework (i.e., humanization) has been used to further minimize rodent sequences (Jones et al., (1986) Nature 321 :522; Verhoeyen et al., (1988) Science 239:1534). In some embodiments, the antibodies of the invention are chimeric antibodies. In some preferred embodiments, the antibodies of the invention are humanized antibodies.

In some embodiments, the chimeric or humanized antibodies of the invention can be prepared on the basis of the sequence of a murine monoclonal hybridoma antibody prepared as described above. DNA encoding the heavy and light chain immunoglobulins can be obtained from the murine hybridoma of interest and engineered to contain non-murine (e.g., human) immunoglobulin sequences using standard molecular biology techniques.

In some embodiments, the chimeric CCR8 antibodies described in the invention can be prepared by operatively linking the heavy and light-chain variable regions of the hybridoma-derived immunoglobulins to the constant region of human IgG using methods known in the art (see, e.g., U.S. Patents No. 4,816,567 to Cabilly et al), and obtaining chimeric heavy chains and chimeric light chains. In some embodiments, constant regions comprised in the chimeric antibodies of the invention can be selected from any IgG subclasses, such as IgG1, IgG2, IgG3, IgG4, preferably IgG1.

In some embodiments, chimeric CCR8 antibodies can be obtained by transfecting expression cells with "mixed and matched" expression plasmids of chimeric light chains and chimeric heavy chains. The resultant "mixed and matched" antibodies' binding to CCR8 can be tested by the above binding assays and other conventional binding assays (e.g., ELISA).

As defined herein, "complementarity determining region" or "CDR region" or "CDR" is the regions of an antibody variable domain which are hypervariable in sequence and form structurally defined loops ("hypervariable loops") and/or contain the antigen-contacting residues ("antigen contacts"). The CDRs are primarily responsible for binding to an epitope of an antigen. Heavy- and light-chain CDRs are typically called CDR1, CDR2, and CDR3, sequentially numbered from the N-terminus. CDRs located in the heavy-chain variable domain of an antibody are referred to as HCDR1, HCDR2, and HCDR3, and CDRs located in the light-chain variable domain of an antibody are referred to as LCDR1, LCDR2, and LCDR3. In a given amino acid sequence of a light- or heavy-chain variable region, precise boundaries of the amino acid sequence in individual CDRs can be determined using any one of many well-known antibody CDR assignment systems or the combination thereof, the assignment system including, for example, Chothia based on the three-dimensional structure of antibodies and topology of CDR loops (Chothia et al. (1989) Nature 342: 877-883, Al-Lazikani et al., "Standard conformations for the canonical structures of immunoglobulins", Journal of Molecular Biology, 273, 927-948 (1997)), Kabat based on the variability of antibody sequences (Kabat et al., Sequences of Proteins of Immunological Interest, 4th Ed., U.S. Department of Health and Human Services, National Institutes of Health (1987)), AbM system (University of Bath), Contact system (University College London), internationality ImMunoGeneTics database (IMGT) (On the World Wide Webimgt.cines.fr/superior ), and North CDR definition based on affinity propagation clustering with a large number of crystal structures.

For example, according to the different regimes for identifying the CDRs, the residues of each CDR are described as follows.

The following are the ranges of the CDRs defined with Kabat, AbM, Chothia, Contact, and IMGT Schemes.

| CDR | Kabat Scheme | AbM Scheme | Chothia Scheme | Contact Scheme | IMGT Scheme |
|---|---|---|---|---|---|
| LCDR1 (Kabat and Chothia numbering systems) | L24-L34 | L24-L34 | L26-L32 | L30-L36 | L27-L32 |
| LCDR2 (Kabat and Chothia numbering systems) | L50-L56 | L50-L56 | L50-L52 | L46-L55 | L50-L52 |
| LCDR3 (Kabat and Chothia numbering systems) | L89-L97 | L89-L97 | L91-L96 | L89-L96 | L89-L96 |
| HCDR1 (Kabat numbering system) | H31-H35B | H26-H35B | H26-H32 | H30-H35B | H26-H35B |
| HCDR1 (Chothia numbering system) | H31-H35 | H26-H35 | H26-H32 | H30-H35 | H26-H35 |
| HCDR2 (Kabat and Chothia numbering systems) | H50-H65 | H50-H58 | H53-H55 | H47-H58 | H51-H57 |
| HCDR3 (Kabat and Chothia numbering systems) | H95-H102 | H95-H102 | H96-H101 | H93-H101 | H93-H102 |

Unless otherwise specified, in this invention, the term "CDR" or "CDR sequence" encompasses the CDR sequence determined in any of the manners described above. A CDR can also be identified on the basis of the position having the same Kabat number as the reference CDR sequence (e.g., one of the exemplary CDRs of the invention). Unless otherwise specified, in the present invention, when referring to the residue positions in the antibody variable region (including heavy-chain variable region residues and light-chain variable region residues), it refers to the numbering position of Kabat numbering system in Kabat et al., Sequences of Proteins of Immunological Interest, 5th Ed. Public Health Service, National Institutes of Health, Bethesda, Md. (1991). In some embodiments, HCDRs and LCDRs in the antibodies of the invention is identified according to Kabat Scheme.

Unless otherwise specified, the CDR boundaries of the antibodies of the invention can be determined by those skilled in the art according to any schemes in the art (for example, different assignment systems or combinations thereof).

It should be noted that the CDR boundaries in the variable regions of the same antibody obtained on the basis of different assignment systems may vary. Hence, the CDR sequences in the variable regions of the same antibody defined under different assignment systems may vary. Therefore, when involving definition of an antibody with the specific CDR sequences defined in the invention, the scope of the antibody also encompasses antibodies whose variable region sequences contain the specific CDR sequences mentioned above, but with the claimed CDR boundaries different from the specific CDR boundaries defined in the invention due to application of different schemes (for example, different assignment systems or combinations thereof).

Antibodies with different specificity (i.e., different binding sites for different antigens) have different CDRs. However, despite CDRs varying among antibodies, there are only a limited number of amino acid positions within the CDRs, which are directly involved in antigen binding. A minimum overlapping region can be determined using at least two of Kabat, Chothia, AbM, Contact and North schemes, thereby providing a "minimum binding unit" for antigen binding. The minimum binding unit can be a subsection of the CDR. As will be apparent to those skilled in the art, residues in the rest of the CDR sequence can be determined by the structure of the antibody and protein folding. Therefore, the present invention also considers variants of any given CDRs. For example, in one CDR variants, the amino acid residues of the minimum binding unit can remain unchanged, and the remaining CDR residues defined according to Kabat or Chothia scheme be replaced by conservative amino acid residues.

Unless otherwise specified, in this invention, the term "CDR" or "CDR sequence" encompasses the CDR sequence determined in any of the manners described above.

In the present invention, various expression plasmids of chimeric heavy- and light-chains are mixes and matched to transfect expression cells, producing anti-CCR8 chimeric antibodies.

For the humanized antibodies set forth in the invention, the murine-derived CDR regions can be inserted into a human germline framework region using methods known in the art. See also U.S. Patents No. 5,225,539 to Winter et al. and U.S. Patents Nos. 5,530,101; 5,585,089; 5,693,762; and 6,180,370 to Queen et al.

In some embodiments, the amino acid changes include amino acid deletion, addition or substitution, etc. In some embodiments, the anti-CCR8 antibodies or antigen-binding fragments thereof of the invention include those which have amino acid sequences mutated by amino acid deletion, addition or substitution but still having at least 90%, 91%, 92%, 93%, 94%, 95%, 96%, 97%, 98%, 99% or 100% identity to the above antibodies (particularly in the CDR regions depicted in the above sequences). In some embodiments, the antibodies of the invention have at least one, e.g. 1, 2, 3, 4 or 5, of amino acid mutations by deletion, addition or substitution in their CDR regions, compared to the CDR regions depicted in the specific sequences In some embodiments, the antibodies of the invention have at least one, e.g. 1, 2, 3, 4 or 5, of amino acid mutations by deletion, addition or substitution in their framework regions, compared to the framework regions in the specific sequences
The term "percent (%) amino acid sequence identity" or simply "identity" is defined as the percentage of amino acid residues in a candidate amino acid sequence that are identical with the amino acid residues in the reference amino acid sequence, after aligning the amino acid sequences (and introducing gaps, if necessary) to achieve the maximum percent sequence identity, and not considering any conservative substitutions as part of the sequence identity. Alignment for purposes of determining percent amino acid sequence identity can be achieved with various methods in the art, for example, using publicly available computer software such as BLAST, BLAST-2, ALIGN or MEGALIGN (DNASTAR) software/algorithm. Those skilled in the art can determine appropriate parameters for measuring alignment, including any algorithms required to achieve maximal alignment over the full-length of the sequences being compared.

In some embodiments, provided is an antibody or an antigen-binding fragment thereof of the invention, where the antibody or the antigen-binding fragment thereof comprises a heavy-chain constant region and/or a light-chain constant region; preferably, the light-chain constant region is the λ- or κ-chain constant region and the heavy-chain constant region is selected from mouse mIgG2a, human IgG1, human IgG2, human IgG3 or IgG4 subclass, or the modified forms thereof. In some preferred embodiments, the heavy-chain constant region is of the human IgG1 subclass or of the human IgG4 subclass with a S228P mutation. In some preferred embodiments, the modified version of the said constant region includes amino acid sequence modification and/or glycosylation.

In some embodiments, one or more amino acid modifications may be introduced into the Fc region of an antibody provided herein, thereby generating an Fc region variant. The Fc region variant may comprise a human Fc region (e.g., a human IgG1, IgG2, IgG3, or IgG4 Fc region) sequence comprising an amino acid modification (e.g. an addition, deletion, or substitution, preferably a substitution) at one or more amino acid positions.

In some embodiments, the antibody comprises at least one modification that enhances cell killing. In some embodiments, the enhanced cell-killing is enhanced antibody-dependent cellular cytotoxicity (ADCC) and/or complement dependent cytotoxicity (CDC). In some embodiments, the modification is defucosylation/reduction in fucosylation. In some embodiments, the modification is one or more mutations at one or more positions selected from the sites of 238, 239, 248, 249, 252, 254, 255, 256, 258, 265, 267, 268, 269, 270, 272, 276, 278, 280, 283, 285, 286, 289, 290, 292, 293, 294, 295, 296, 298, 301, 303, 305, 307, 309, 312, 315, 320, 322, 324, 326, 327, 328, 329, 330, 331, 333, 334, 335, 337, 338, 340, 360, 373, 376, 378, 382, 388, 389, 398, 414, 416, 419, 430, 434, 435, 437, 438 and 439 according to the EU numbering system in the heavy-chain constant region. As used herein, unless otherwise provided in the context, when relating to the position numbers of the constant region amino acids, reference is made to the EU numbering system. In the specification, mutation in the amino acid sequence can be designated as: single-letter code for a parental amino acid, followed by the number for the position, then followed by the single-letter code for a mutated amino acid. For example, mutation of leucine (L) at Position 234 into alanine (A) is represented as "L234A". Sometimes, in a sequence, a slash (/) is used to list multiple alternative options. For example, deletion of cysteine (C) at Position 236 can be represented as "C236 deletion". In some preferred embodiments, the modification is one or more mutations at one or more positions selected from L234, L235, G236, S239, F243, D265, H268, D270, R292, S298, Y300, V305, K326, A330, I332, E333, K334, and P396 according to the EU numbering system in the heavy-chain constant region. In some embodiments, the one or more mutations in the heavy-chain constant region are one or more mutations selected from N297A substitution, N297Q substitution, L235A and L237A substitutions, L234A and L235A substitutions, E233P substitution, L234V substitution, L235A substitution, C236 deletion, P238A substitution, D265A substitution, A327Q substitution, and P329A substitution according to the EU numbering system in the heavy-chain constant region. In some embodiments, the modification is one or more mutations in the heavy-chain constant region selected from G236A, S239D, F243L, T256A, K290A, R292P, S298A, Y300L, V305I, A330L, I332E, E333A, K334A, A339T, and P396L according to the EU numbering system. In some embodiments, the one or more mutations in the heavy-chain constant region are one or more mutations selected from L235V, S239D, S239M, F243L, H268D, D270E, R292P, S298A, Y300L, V305I, K326D, A330L, A330M, I332E, E333A, K334A, K334E and P396L according to the EU numbering system in the heavy-chain constant region. In some embodiments, the one or more mutations in the heavy-chain constant region are one or more mutations selected from M252Y, S254T, and T256E according to the EU numbering system in the heavy-chain constant region.

In some embodiments, the heavy-chain constant region has one or more sets of the mutations that occur simultaneously at the following combination of positions selected from: (1) L235/F243/R292/Y300/P396, (2) F243/R292/Y300/V305/P396, (3) D270/K326/A330/K334, (4) S239/A330/I332, (5) S298/E333/K334, (6) L234/L235/G236/S239/H268/D270/S298, (7) M252/S254/T256, (8) L234/L235/D265, (9) G236/S239/I332, and (10) S239/I332.

In some embodiments, the heavy-chain constant region has one or more sets of mutations selected from the following mutation combinations: (1) L235V/F243L/R292P/Y300L/P396L, (2) F243L/R292P/Y300L/V305I/P396L, (3) D270E/K326D/A330M/K334E, (4) S239D/A330L/I332E, (5) S298A/E333A/K334A, (6) L234Y/L235Q/G236W/S239M/H268D/D270E/S298A, (7) M252Y/S254T/T256E, (8) L234A/L235A/D265A, (9) L234F/L235E/D265A, (10) G236A/S239D/I332E, and (11) S239D/I332E.

In some embodiments, it may be desirable to create cysteine engineered antibodies, e.g., "thioMAbs," in which one or more residues of an antibody are substituted with cysteine residues. In some embodiments, one, two or more mutations (e.g., amino acid substitution) are introduced into the hinge region (CH1 Domain) such that the number of cysteine residues in the hinge region is altered, e.g., increased or decreased.

In some embodiments, an antibody provided herein may be further modified to contain other non-proteinaceous moieties that are known in the art and readily available.

### Expression of antibodies

In yet another aspect, the present invention provides a polynucleotide molecule encoding the anti-CCR8 antibody or any fragments thereof described herein. The nucleic acid molecule may comprise a polynucleotide molecule encoding an amino acid sequence of the light- and/or heavy-chain variable regions of the antibody or at least a portion thereof, or a polynucleotide molecule encoding an amino acid sequence of the light- and/or heavy-chains of the antibody or at least a portion thereof.

For example, the polynucleotide molecule of the invention comprises a nucleic acid encoding an amino acid sequence as set forth in any one selected from SEQ ID NOs: 1-95, or a nucleic acid encoding an amino acid sequence having at least 85%, 90%, 91%, 92%, 93%, 94%, 95%, 96%, 97%, 98% or 99% identity to an amino acid sequence as set forth in any one selected from SEQ ID NOs: 1-95.

In some embodiments, the polynucleotide molecules encoding the antibodies of the invention include those which have been mutated by nucleotide deletion, addition or substitution but still have at least 60%, 70%, 80%, 90%, 95% or 100% identity to the encoding regions corresponding to the CDR regions depicted in the above sequences.

In another aspect, the present invention provides an expression vector comprising the polynucleotide molecule as herein described, and the vector is preferably an eukaryotic expression vector. In some embodiments, the polynucleotide molecule as described herein is contained in one or more expression vectors.

In another aspect, the present invention provides a host cell comprising a polynucleotide molecule as described herein or an expression vector as described herein. Preferably, the host cell is a eukaryotic cell, more preferably a mammalian cell.

In yet another aspect, the present invention provides a method for preparing an anti-CCR8 antibody or an antigen-binding fragment thereof as described herein, the method comprising expressing the antibody or the antigen-binding fragment thereof in a host cell as described herein under the condition suitable for expressing the antibody or the antigen-binding fragment thereof, and recovering the expressed antibody or the antigen-binding fragment thereof from the host cell.

The present invention provides mammalian host cells for expressing the recombinant antibodies of the invention or any fragments thereof, including many immortalized cell lines available from the American Type Culture Collection (ATCC). These include in particular the Chinese hamster ovary (CHO) cells, NS0, SP2/0 cells, HeLa cells, baby hamster kidney (BHK) cells, monkey kidney cells (COS), human hepatocellular carcinoma cells, A549 cells, 293T cells and many other cell lines. Mammalian host cells include human, mouse, rat, dog, monkey, pig, goat, bovine, horse and hamster cells. The particularly preferred cell lines are selected by determining which cell lines have high expression levels.

In one embodiment, the present invention provides a method for preparing an anti-CCR8 antibody, wherein the method comprises, when introducing an expression vector into mammalian host cells, the antibodies are produced by culturing the host cells for a period of time sufficient to allow for expression of the antibody in the host cells or, more preferably, secretion of the antibody into the culture medium in which the host cells are grown.

Standard protein purification methods can be used to recover antibodies from the culture medium. Antibody molecules prepared as described herein may be purified by art-known techniques such as high performance liquid chromatography, ion exchange chromatography, gel electrophoresis, affinity chromatography, size exclusion chromatography, and the like. The actual conditions used to purify a particular protein also depend on factors such as net charge, hydrophobicity, hydrophilicity etc., and these will be apparent to those having skill in the art. The purity of the antibody molecules of the invention can be determined by any of a variety of well-known analytical methods including size exclusion chromatography, gel electrophoresis, high performance liquid chromatography, and the like.

It is likely that antibodies expressed by different cell lines or in transgenic animals have different glycosylation profiles from each other. However, all antibodies encoded by the nucleic acid molecules provided herein or comprising the amino acid sequences provided herein are the part of the invention, regardless of how the antibodies are glycosylated. Likewise, in certain embodiments, non-fucosylated antibodies are advantageous because they generally have more potent efficacy *in vitro* and *in vivo* than their fucosylated counterparts and are less likely to be immunogenic because their carbohydrate structures are normal components of native human IgG in serum.

### Pharmaceutical compositions and pharmaceutical formulations

In yet another aspect, the present invention provides a pharmaceutical composition comprising the anti-CCR8 antibodies or antigen-binding fragments thereof as described herein, polynucleotides molecules described herein, vectors described herein, host cells described herein, or immunoconjugates described herein, and pharmaceutically acceptable carriers or excipients. It should be understood that the anti-CCR8 antibody or the pharmaceutical composition thereof provided in the present invention may be integrated with carriers, excipients and other agents suitable in a formulation for combination administration, resulting in improved transfer, delivery, tolerance, and the like.

The term "pharmaceutical composition" refers to a formulation which is found in a form so as to permit the biological activity of the active ingredient contained in the formulation to exist in an effective form, and which contains no additional components that are unacceptably toxic to a subject to which the formulation would be administered.

Pharmaceutical formulations comprising an anti-CCR8 antibody of the invention described herein can be prepared by mixing the anti-CCR8 antibody of the invention having the desired degree of purity with one or more optional pharmaceutical excipients (Remington's Pharmaceutical Sciences, 16th Edition, Osol, A. Ed. (1980)), preferably in the form of aqueous solutions or lyophilized formulations.

The pharmaceutical compositions or formulations of the invention can further contain one or more active ingredients as necessary for particular indications being treated, preferably those with complementary activities that do not adversely affect each other. In some embodiments, the pharmaceutical compositions of the invention further comprise compositions of polynucleotide molecules encoding anti-CCR8 antibodies.

The pharmaceutical compositions of the invention may also contain one or more additional therapeutic agents which are necessary for particular indications being treated, encompassing any substance effective in prevention or treatment of tumors (e.g., cancer), preferably those with activities that do not adversely affect each other. For example, chemotherapy agents, hormones, etc. The therapeutic agents are present appropriately in combination at amounts effective for applications of interest. In some embodiments, the additional therapeutic agent is a chemotherapeutic agent. In some embodiments, the additional therapeutic agent is other antibodies. In some embodiments, the additional therapeutic agent is other monoclonal antibodies. In some preferred embodiments, the additional therapeutic agent is a monoclonal antibody targeting an immune checkpoint. In some more preferred embodiments, the additional therapeutic agent is a PD-1-targeted monoclonal antibody. In some more preferred embodiments, the additional therapeutic agent is a PD-L1-targeted monoclonal antibody. In some more preferred embodiments, the additional therapeutic agent is a CTLA4-targeted monoclonal antibody.

In yet another aspect, the present invention provides a pharmaceutical combination comprising the antibodies or the antigen-binding fragments thereof described herein, polynucleotides molecules described herein, vectors described herein, host cells described herein, immunoconjugates described herein, or pharmaceutical composition described herein, and one or more additional therapeutic agents.

In yet another aspect, the present invention provides a kit comprising the antibodies or the antigen-binding fragments thereof described herein, polynucleotides molecules described herein, vectors described herein, host cells described herein, immunoconjugates described herein, pharmaceutical composition described herein, or the pharmaceutical combination described herein.

### Combination products or kits

In some embodiments, the present invention also provides a combination product comprising the anti-CCR8 antibodies or the fragments thereof of the invention, and one or more additional therapeutic agents (e.g., chemotherapy agents, other antibodies, cytotoxic agents, anti-infective active agents, small molecule drugs or immunomodulators, etc).

In some embodiments, the combination product is used to prevent or treat CCR8-/immune checkpoint-related diseases and/or CCR8- and/or immune checkpoint-mediated diseases. In some embodiments, the additional therapeutic agent is an established standard therapeutic agent. In some embodiments, the additional therapeutic agent is a chemotherapeutic agent. In some embodiments, the additional therapeutic agent is other antibodies. In some embodiments, the additional therapeutic agent is other monoclonal antibodies. In some preferred embodiments, the additional therapeutic agent is a monoclonal antibody targeting an immune checkpoint. In some more preferred embodiments, the additional therapeutic agent is a PD-1-targeted monoclonal antibody. In some more preferred embodiments, the additional therapeutic agent is a PD-L 1-targeted monoclonal antibody. In some more preferred embodiments, the additional therapeutic agent is a CTLA4-targeted monoclonal antibody.

In some embodiments, two or more components of the combination product can be administered sequentially, separately or simultaneously to a subject.

In some embodiments, the present invention also provides a kit comprising the anti-CCR8 antibodies or the fragments thereof, the pharmaceutical compositions or the combination products of the invention, and optionally a package insert directing administration.

In some embodiments, the present invention also provides a pharmaceutical preparation comprising the anti-CCR8 antibodies or the fragments thereof, the pharmaceutical compositions or the combination products of the invention, optionally comprising further a package insert directing administration.

### Medical uses and methods of treatment

Any of the anti-CCR8 antibodies or the corresponding immunoconjugates provided herein can be used in therapeutic methods. It should also be understood that, in discussing "antibody", compositions containing antibodies are also included. The anti-CCR8 antibody of the invention may be used in therapeutic or prophylactic methods described in any of the embodiments of the invention in a therapeutically effective amount or a prophylactically effective amount.

In yet another aspect, the present invention provides the use of the antibodies or antigen-binding fragments thereof, polynucleotides, expression vectors, host cells, immunoconjugates or pharmaceutical compositions described herein in preparation of a medicament. The present invention further provides the use of pharmaceutical combinations described herein in preparation of a medicament. Thus, the present invention essentially provides the use of an effective amount of the antibodies or antigen-binding fragments thereof, polynucleotides, expression vectors, host cells, immunoconjugates or pharmaceutical compositions described herein in combination with other therapeutic agents in preparation of a medicament Among them, the medicament is used forpreventing and/or treating tumors, autoimmune or infectious diseases in subjects, the tumor preferably being, for example, melanoma, breast cancer, colon carcinoma, rectal cancer, colorectal cancer, gastric cancer, esophageal cancer, head and neck cancer, lung cancer, ovarian cancer, kidney cancer, bladder cancer, liver cancer or thymic carcinoma and the metastatic cancers thereof

In yet another aspect, the present invention provides the antibodies or antigen-binding fragments thereof, polynucleotides, expression vectors, host cells, immunoconjugates, pharmaceutical compositions or pharmacological combinations described herein for use in treating and/or preventing tumors,autoimmune or infectious diseases, the tumor being preferably, for example, melanoma, breast cancer, colon carcinoma, rectal cancer, colorectal cancer, gastric cancer, esophageal cancer, head and neck cancer, lung cancer, ovarian cancer, kidney cancer, bladder cancer, liver cancer or thymic carcinoma and the metastatic cancers thereof.

In yet another aspect, the present invention provides a method for treating and/or preventing tumors, autoimmune or infectious diseases, comprising administering to a subject in need hereof a therapeutically or prophylactically effective amount of the antibodies or antigen-binding fragments thereof, polynucleotides, expression vectors, host cells, immunoconjugates, pharmaceutical compositions or pharmacological combinations described herein. The tumor is preferably, for example, melanoma, breast cancer, colon carcinoma, rectal cancer, colorectal cancer, gastric cancer, esophageal cancer, head and neck cancer, lung cancer, ovarian cancer, kidney cancer, bladder cancer, liver cancer or thymus cancer and the metastatic cancers thereof.

In some embodiments, the administration ways of the invention include but are not limited to oral, intravenous, subcutaneous, intramuscular, intra-arterial, intra-articular (e.g., in arthritic joints) administration, by inhalation, aerosol delivery or localized administration to lesions, etc.

The term "treatment" refers to clinical intervention in an attempt to alter the natural course of a disorder in an individual being treated. Desirable effects of treatment include, but are not limited to, preventing occurrence or recurrence of disease, alleviation of symptoms, diminishing in any adverse experiences or direct or indirect pathological consequences of the disease, decreasing the rate of disease progression, amelioration or palliation of the disease state, and remission or improved prognosis. The antibodies of the invention can reduce the severity of the disease from one or more aspects, that is, exerting a therapeutic effect. In certain embodiments, the therapeutic effect is manifested in one or more situations: the prolonged mean life span of a patient (survival); delayed disease progression; and reduced need for medical care.

The present invention also provides co-administering therapeutically effective amount(s) of one or more therapies (e.g., treatment modalities and/or other therapeutic agents) to a subject. The antibodies of the invention may be used alone or in combination with other therapeutic agents in therapies. In some embodiments, the antibody of the invention is co-administered with at least one additional therapeutic agent.

### Methods for Diagnosis and Detection

In another aspect, the present invention provides a method for detecting the presence of CCR8 in the sample using the antibody or the antigen-binding fragment thereof described herein. The term "detecting" as used herein includes quantitative or qualitative detection. In some embodiments, the sample is a biological sample. In certain embodiments, the biological sample is blood, serum or other liquid samples of biological sources. In certain embodiments, the biological sample comprises a cell or tissue. In certain embodiments, CCR8 is the human CCR8 or the cynomolgus CCR8. The method comprises the steps of contacting the antibody or the antigen-binding fragment thereof described herein or a detection composition containing the antibody or the antigen-binding fragment thereof with a sample, and detecting the presence of binders or the binding signals arising from the binding of the antibody or the antigen-binding fragment thereof to CCR8. When used for detection purposes, the antibody or the antigen-binding fragment thereof described herein may be labeled to indicate whether the binder is formed. In certain embodiments, the method can be an *in vitro* or *in vivo* method.

In some embodiments, CCR8 is detected prior to treatment, e.g., prior to initiation of treatment or prior to a certain treatment after a treatment interval. In one embodiment, provided is an anti-CCR8 antibody or an antigen-binding fragment for use in a method of diagnosis or detection.

The present invention includes all combinations of the specific embodiments described. Further embodiments and the full scope of applicability of the invention will become apparent from the detailed description provided hereinafter. It should be understood, however, that the detailed description and specific Examples, while indicating the preferred embodiments of the invention, are provided by way of illustration only, since various changes and modifications within the spirit and scope of the invention will become apparent to those skilled in the art from this detailed description. All publications, patents and patent applications cited herein, including citations, are incorporated herein by reference in their entirety for all purposes.

The compounds of the invention can be prepared by a variety of synthetic methods known to those skilled in the art, including the specific embodiments listed below, embodiments formed by combining the specific embodiments with other methods, and the equivalent substitutions known to those skilled in the art, and the preferred embodiments include but are not limited to the Examples of the invention.

### Examples

The present invention is illustrated by the following Examples, but is not intended to be limited in any way thereby. The present invention has been described in detail herein, and specific embodiments thereof are also disclosed. It will be apparent to those skilled in the art that various changes and modifications can be made to the specific embodiments of the invention without departing from the spirit and scope of the invention.

### Example 1. Immunization of Animals and Preparation of Anti-Human CCR8 Mouse Hybridoma Antibodies

Human full-length CCR8 gene (DNA or mRNA) and engineered cell strain overexpressing human CCR8 were used to immunize mice, and monkey full-length CCR8 gene (DNA or mRNA) and engineered cell strain overexpressing monkey CCR8 were also used in immunization or immune boosting. SP2/0 cell fusion was performed and positive clones were screened by flow cytometry. The detailed procedures are as follows:
**Immunization of Animals:** The gene coding the full-length human CCR8 (UniProtKB/Swiss-Prot: P51685) was constructed into the plasmid PCDNA3.1 or PCDNA3.4 (established by Shanghai Ruizhi Chemical Research Co., Ltd.) and used as an immunogen to perform DNA immmunization of female Balb/c mice at 4 µg/mouse. The DNA immunization was repeated 6 - 7 times at an interval of 2 weeks. 293F cells expressing human CCR8 (established by Shanghai Ruizhi Chemical Research Co., Ltd., 293F-hCCR8) was injected intraperitoneally 10 days post the last immunization for immune boosting, and three days later, the spleen of the mouse was excised for cell fusion.

Female Balb/c mice were immunized intraperitoneally with 293F cells expressing human CCR8 (293F-hCCR8) as an immunogen (2-5x10⁶ cells/mouse). The immunization with cells was repeated 3 times at an interval of 2 weeks. 293F-hCCR8 cells (2-5x10⁶ cells/mouse) was re-injected intraperitoneally 10 days post the last immunization for immune boosting, and three days later, the spleen of the mouse was excised for cell fusion.

Female Balb/c mice were mRNA-immunized with a mRNA coding the full-length human CCR8 (UniProtKB/Swiss-Prot: P51685) as an immunogen (prepared by Shanghai Hongcheng BioPharmaceutical Co., Ltd., 50 µg/mouse). The mRNA immunization was repeated 3 or 4 times at an interval of 3 weeks. 293F-hCCR8 cells were injected intraperitoneally 10 days post the last immunization for immune boosting, and three days later, the spleen of the mouse was excised for cell fusion.

**Cell fusion:** Mouse splenocytes were fused electrically with SP2/0 cells (ATCC No. CRL-1581) at a proportion of 2:1 (BTX Electrofusion instrument: ECM2001⁺), cultured in HAT medium (GIBCO, Cat. No. H0262) in 96-well culture plates, and 10 days later, screened for antibodies in hybridoma cell supernatant.

**Screening human CCR8- and cynomolgus CCR8-specific positive clones:** Cells expressing human CCR8 (CHOK1-hCCR8, established by Shanghai ChemPartner Co., Ltd.) and cells expressing cynomolgus CCR8 (CHOK1-cynoCCR8, established by Shanghai ChemPartner Co., Ltd.) were plated onto 96-well U-bottom assay plates at a density of 5x10⁷ cells/mL in 100 µL/well. 100 µL of hybridoma cell culture supernatant was added and incubated at 4°C for 1 hour. Cells were washed twice with FACS buffer (PBS containing 1% FBS) and centrifuged at 300 g. Alexa488-labelled anti-mouse IgG antibody was added and incubated at 4°C for 1 hour. Cells were washed three times with the FACS buffer (PBS solution containing 1% FBS) and centrifuged at 300 g. The mean fluorescence intensity (Median Fluorescence Intensity, MFI) was read with flow cytometry (BECKMAN COULTER CytoFLEX). By comparing with the negative control (CHOK1 cells not expressing CCR8), positive clones were screened out. The mean fluorescence intensities of clones in Table 1 binding to the human CCR8 (CHOK1-hCCR8) and the cynomolgus CCR8 (CHOK1-cynoCCR8) were higher than that of the blank control cell (CHOK1), indicating that these clones specifically bind to the human and cynomolgus CCR8s.

**Table 1. Binding of Supernatant from Positive Hybridoma Clones to Human CCR8 (CHOK1-hCCR8), Cynomolgus CCR8 (CHOK1-cynoCCR8) and Blank CHOK1 Cells (Mean Fluorescence Intensity, MFI)**

| Clone number | **CHOK1-hCCR8** | **CHOK1-cynoCCR8** | **CHOK1** |
|---|---|---|---|
| 27B9-1G3 | 2659 | 14023 | 89 |
| 559E1B10 | 7346 | 21912 | 75 |
| 563E10E12 | 6366 | 16905 | 85 |
| 569D11B5 | 5804 | 13164 | 91 |
| 589D7C7 | 6121 | 15230 | 87 |

### Example 2. Preparation and Identification of Anti-human CCR8 Murine Monoclonal Antibodies

The hybridoma cells showing the binding activity to human CCR8 and cynomolgus CCR8 in Table 1 were cultured in a serum-free medium. Ten days later, the culture supernatant was collected and monoclonal antibodies were purified with a Protein A column (Borgron (Shanghai) Biotechnology Co., Ltd., Part Number: AA0272), obtaining purified monoclonal antibodies. Flow cytometry was used to detect the binding activity of anti-human CCR8 antibody and its blocking activity against CCL1 binding.

### 2.1 Binding activity of anti-Human CCR8 antibodies to human CCR8 and cynomolgus CCR8

Binding assay with anti-CCR8 antibodies and cells expressing human CCR8 or cynomolgus CCR8:
293F cell lines expressing human CCR8 (293F-hCCR8, established by Shanghai ChemPartner Co., Ltd.) or 293T cell lines expressing cynomolgus CCR8 (293T-cyno CCR8, established by Kyinno Biotechnology Co., Ltd) were adjusted to 1×10⁶ cells/mL, placed in 96-well U-bottom plates at 100 µL/well, centrifuged and the supernatant was discarded. The anti-human CCR8 antibody, the control antibody (the anti-human CCR8 433H, clone number: 433H, BD Pharmingen, Cat. No. 624092; 10A11, with the sequences in Patent WO2020138489 (the sequence number of the light-chain variable region: 59 and the sequence number of the heavy-chain variable region: 41), which are fused respectively with the human- and mouse-constant region sequences in Table 5 herein to yield the antibodies 10A11_hIgG1 and 10A11_mIgG2a), and the IgG1 isotype (Biointron Biotechnology Co., Ltd., Cat. No. B117901) were diluted with the FACS buffer (PBS solution containing 1% FBS) to the initial working concentrations and serially diluted with the FACS buffer solution. Cells were resuspended in the serially diluted antibodies at 100 µL/well, pipetted to mix well, and incubated at 4°C for 1 hour. After incubation, the cells were centrifuged and washed with the FACS buffer three times. Cell pellets were resuspended by adding 100 µL of the diluted secondary antibody (Goat anti-Human IgG (H+L) Cross-Adsorbed Secondary Antibody, Alexa Fluor^{™} 647, Invitrogen, Cat. No. A21445) to each well, pipetted to mix well, and incubated at 4°C for 45 minutes. After incubation, the cells were centrifuged and washed three times before being resuspended with the FACS buffer at 100 µL/well. The median fluorescence intensity (MFI) was read with a flow cytometer (BECKMAN COULTER CytoFLEX) and the experimental data were analyzed with the software Graphpad Prism 8.0. EC₅₀ was calculated by fitting the dose-effect curve of the antibody wtih the chosen four-parameter regression model, with the logarithms of the antibody concentrations on the x-axis and the corresponding MFI values on the y-axis.

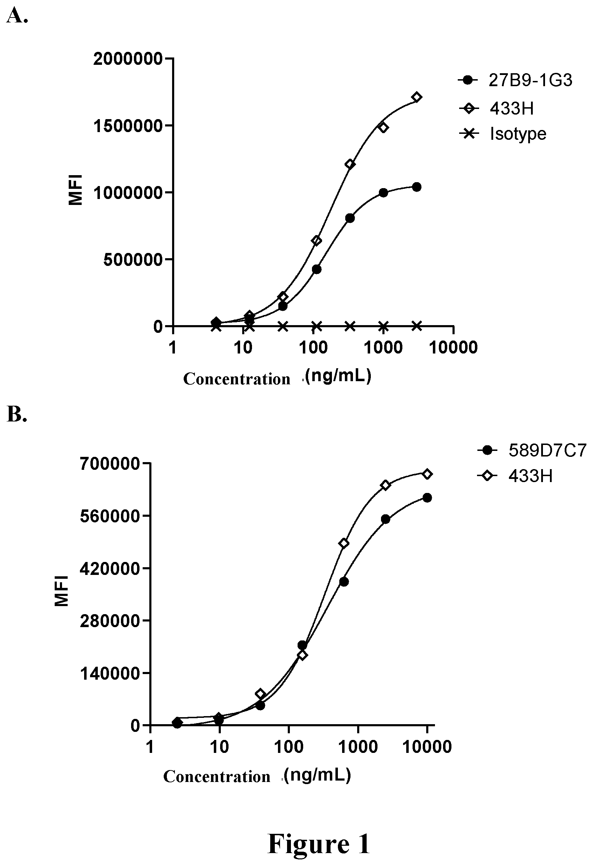

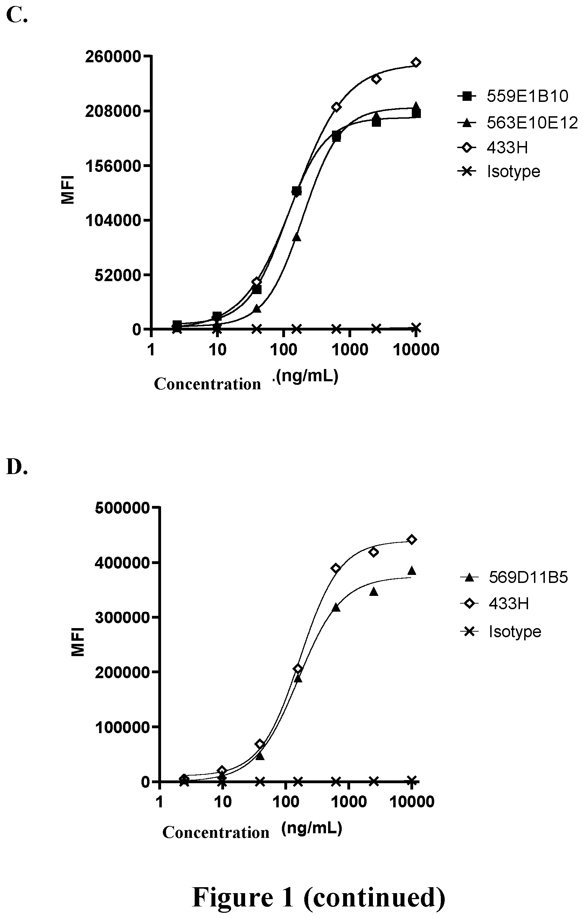

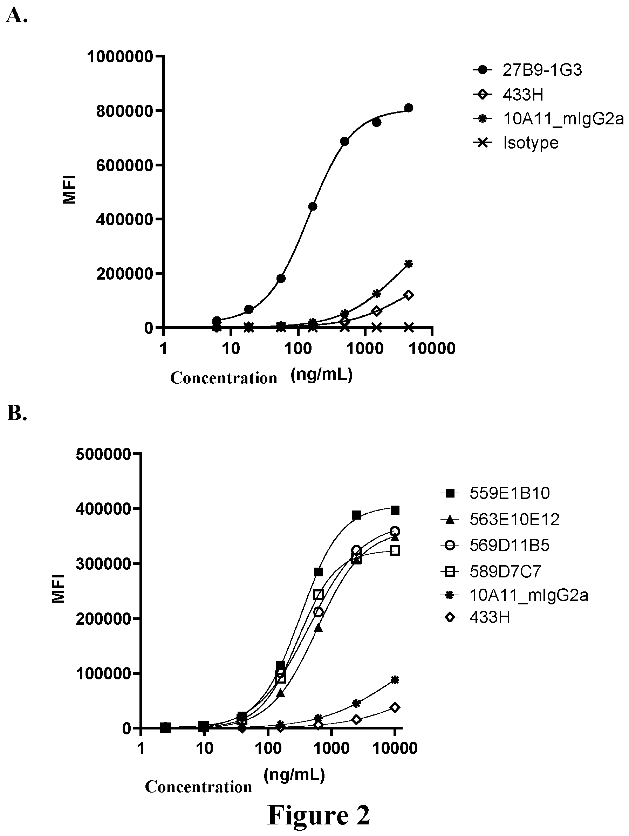

As shown in Figs 1 and 2 and in Table 2, the antibody clones such as 27B9-1G3, 559E1B10, 563E10E12, 569D11B5 and589D7C7 showed strong binding activity to 293F-hCCR8, with EC₅₀ values of 107.0 - 360.6 ng/mL; EC₅₀ values of binding of 433H and 10A11_mIgG2a as the controls to 293F-hCCR8 are 206.0 and 185.1 ng/mL, respectively. The antibody clones 27B9-1G3, 559E1B10, 563E10E12, 569D11B5 and 589D7C7 all demonstrated stronger binding activity to cynomolgus CCR8, with EC₅₀ values of 146.1-590.8 ng/mL, while the binding activities of the control antibodies 433H and 10A11_mIgG2ato cynomolgus CCR8 are very weak, with EC₅₀ values of 15312.5 and 5770.5 ng/mL, respectively.

**Table 2. Binding Activity of Anti-CCR8 Antibodies to Human CCR8 and Cynomolgus CCR8**

| Clone number | **293F-hCCR8 EC₅₀, ng/mL** | **293T-cynoCCR8 EC₅₀, ng/mL** |
|---|---|---|
| 27B9-1G3 | 149.9 | 146.1 |
| 559E1B10 | 107.0 | 321.5 |
| 563E10E12 | 194.7 | 590.8 |
| 569D11B5 | 158.9 | 443.9 |
| 589D7C7 | 360.6 | 298.1 |
| 433H | 206.0 | 15312.5 |
| 10A11_mIgG2a | 185.1 | 5770.5 |
| Isotype | N/A | N/A |

| | | |
|---|---|---|
| N/A: Not applicable, no valid EC₅₀ obtained | | |

### 2.2 Receptor ligand blocking assay of Anti-CCR8 Antibody against CCL1

CCL1 is the ligand for CCR8, and CCR8 is the only known receptor for CCL1. High expression of CCL1 in a tumor tissue is negatively correlated with prognosis of cancer. CCL1 can induce Treg migration by binding to CCR8, block the binding of CCL1 to CCR8 can suppress Treg migration and tumor growth (see Klarquist J. et al., Ccl22 Diverts T Regulatory Cells and Controls the Growth of Melanoma. Cancer Res. 2016 Nov 1;76(21): 6230-6240 and Xu Y et al., Sox2 Communicates with Tregs Through CCL1 to Promote the Stemness Property of Breast Cancer Cells. Stem Cells. 2017 Dec; 35(12): 2351-2365). Anti-CCR8 antibodies which have function of blocking the binding of CCL1 to CCR8 may possess more stronger anti-tumor activity. A competitive binding assay was used to detected the activity of the anti-CCR8 antibody blocking the binding of AlexaFluor-647-labelled human CCL1 to 293F-hCCR8. The detailed procedures are as follows:
293F cell lines expressing human CCR8 (293F-hCCR8, established by Shanghai ChemPartner Co., Ltd.) were adjusted to 1×10⁶ cells/mL, placed in 96-well U-bottom plates at 100 µL/well, centrifuged and the supernatant was discarded. The anti-CCR8 antibody, the control antibody (the anti-human CCR8, clone number: 433H, BD Pharmingen, Cat. No. 624092), the hIgG1 isotype (Biointron Biotechnology Co., Ltd., Cat. No.B117901) were diluted with the FACS buffer (PBS solution containing 1% FBS) to the 2-fold initial working concentrations and serially diluted with the FACS buffer. Cells were resuspended with the serially diluted antibodies at 50 µL/well, pipetted to mix well; followed by addition of 20 nM human CCL1-AlexaFluor-647 (Almac, CAF-7) solution at 50 µL/well, pipetted to mix well, and incubated at 4°C for 1 hour. After incubation, the cells were centrifuged and washed three times before being resuspended with the FACS buffer at 100 µL/well. The median fluorescence intensity (MFI) was read with a flow cytometer (BECKMAN COULTER CytoFLEX) and the experimental data were analyzed with the software Graphpad Prism 8.0. IC₅₀ was calculated by fitting the dose-effect curve of the antibody wtih the chosen four-parameter regression model, with the logarithms of the antibody concentrations on the x-axis and the corresponding MFI values on the y-axis.

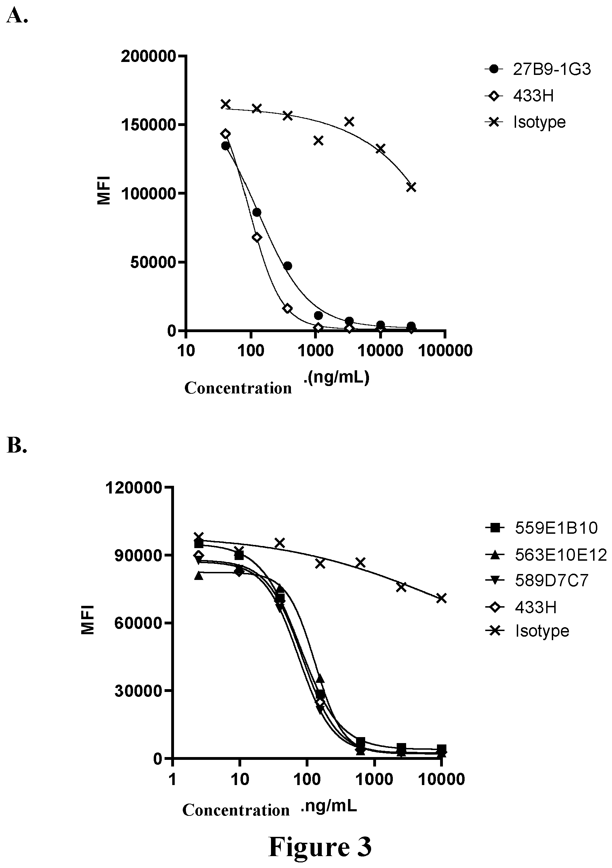

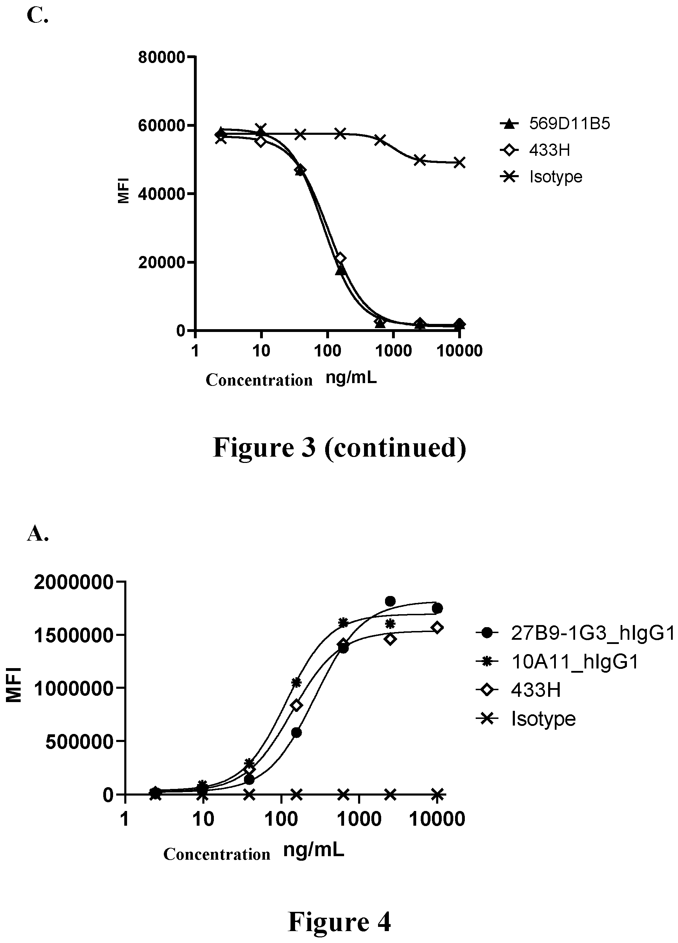

As shown in Fig. 3 and in Table 3, all of the antibody clones 27B9-1G3, 559E1B10, 563E10E12, 569D11B5 and 589D7C7 can block the binding of CCL1 to CCR8, with IC₅₀ values of blocking activity being 75.0-131.7 ng/mL and IC₅₀ value of blocking activity against the control 433H being 94.3ng/mL.

**Table 3. Receptor ligand blocking Activity of CCL1 by Anti-CCR8 Antibody**

| Clone number | **IC₅₀, ng/mL** |
|---|---|
| 27B9-1G3 | 126.8 |
| 559E1B10 | 78.5 |
| 563E10E12 | 131.7 |
| 569D11B5 | 88.2 |
| 589D7C7 | 75.0 |
| 433H | 94.3 |
| IgG1 isotype | N/A |

| | |
|---|---|
| N/A: No blocking activity or poorer blocking activity, no valid IC₅₀ value obtained. | |

### Example 3. Sequencing of Anti-CCR8 Antibodies and Functional Characterization of Chimeric Antibodies

### 3.1 Sequencing of anti-CCR8 antibody and construction, expression and purification of its chimeric antibodies

The hybridoma clones with better performance in the above activity characterization were sequenced, and the sequences are shown in Table 4.

The sequenced light- and heavy-chain variable regions (see Table 4) were constructed respectively into a human Fc constant region (IgG1/K, see in Table 5) or a murine Fc constant region (mIgG2a) for *in vitro* functional characterization or *in vivo* pharmacodynamic studies. Upon sequencing, the constructed hIgG1- and mIgG2a-subclass chimeric antibodies were confirmed to have sequences consistent with those shown in Tables 4 and 5.

Expression was performed using Expi293F cells (Thermofisher Cat. No. A1452) and purification was performed with Protein A column. The detailed procedures are as follows:
Expression of the antibodies in Expi293 cells: One day prior to transfection, Expi293 cells were diluted to 1.5×10⁶ cells/mL and incubated in a shaker in 120 rpm at 37°C, 8% CO₂. On the second day, the density and viability of Expi293 cells were measured. The cell density for transfection should be 3×10⁶ cells/mL, the cell viability be greater than 95%. Preparation of PEI/plasmid complex: PEI (1 mg/mL, polysciences, Cat. No. 24765-1) was inverted to mix well. Plasmid was diluted with OPM-293 CD05 medium (OPM Biosciences, Cat. No. 81075-001), with the plasmid amount used at 1 µg/mL of transfection volume and the culture medium volume for plasmid dilution being 1/20 of the transfection volume. The heavy/light chain plasmids were mixed gently at a ratio of 1:1.5. PEI reagent was diluted with OPM-293 CD05 medium, with the amount of PEI used two times that of the plasmid used and the culture medium volume for PEI dilution being 1/20 of the transfection volume. The resultant mixture was gently inverted to mix well and incubated at room temperature for 5 minutes. The diluted PEI reagent was added into to the diluted plasmid and gently inverted to mix well. The PEI/plasmid complex was incubated at room temperature for 15 minutes, then the resultant solution was added dropwise to shake flask while gently rotating the flask during the addition. After transfection, the flask was incubated in a shaker at 120 rpm at 37°C, 8% CO₂. On the second day after transfection (after 24 hours post transfection), 10% OPM-293 ProFeed (OPM Biosciences, Cat. No. F081918) was added into the flask while gently rotating the flask during the addition. Then, the flask was placed back to the shaker to continue the incubation for 5-7 days. The supernatant was harvested.
Purification of antibodies with Protein A column: A gravity chromatographic column was prepared. The top cover of the gravity chromatographic column was removed. A gasket was placed at the bottom of the gravity column and pressed tightly. The filler Protein A (Cytiva, Cat. No. 17549801) was prepared. The required filler-suspension volume was accurately calculated from the target volume of the filler and suspension ratio of the filler, with the required filler-suspension volume = the target volume of filler / the suspension ratio of filler. The filler was vortexed thoroughly to ensure that the filler was fully suspended. The filler suspension was added to the bottom of the gravity chromatographic column. At least 10 CV of the equilibration buffer PBS was added to the gravity chromatographic column. After the equilibration was completed, the pH was detected at the outlet. If the target pH was not reached, proceeded with the addition of the equilibration buffer until the desired pH equilibrium was achieved. a certain volume of the sample was added slowly to the gravity chromatographic column. At least 10 CV of the washing buffer was added to the gravity chromatographic column. 5 CV of the elution buffer (10-50 mM NaAc, pH 3.0 - 3.5) was slowly added onto the gravity chromatographic column and incubated for 3-5 minutes to collect the eluate. The elution step was repeated as needed. Neutralization: The pH was adjusted to the target value using a neutralizing buffer (1 M Tris). The protein concentration was determined using Nanodrop. The buffer for storing antibody was exchanged to PBS through ultrafiltration.

**Table 4. Sequences of Anti-CCR8 Antibodies**

| **Sequences of the heavy-chain variable regions of hybridoma antibodies** | | |
|---|---|---|
| Clone number | Sequences (CDR regions are underlined) | Sequence Number |
| 27B9-1G3 | EIQLQQSGPELEKPGASVKISCKASGYSFTGYNMNWVKKSNG KSLEWIGNIDPYYGGTNYNQKFKGKATLTVDKSSSTAYMQL KSLTSEDSAVYYCARETWFITTGVIKKGFYFDYWGQGTTLT VSS | SEQ ID NO: 40 |
| 559E1B10 | KVQLQQSGPVLVKPGASVKMSCKASGYTFTDYYVNWMKQN YGKSPEWIGVINPHNGDTRYNQKFKGKATLTVDKSSSTANM ELTSLTSGDSAVYYCASWLRRGYYFDSWGQGTTLTVSS | SEQ ID NO: 39 |
| 563E10E12 | EVQLIESGGGLVQPKGSLKLSCAASGFNFN**AYAMN**WVRQAP GKGLEWVA**RIRSKSNNFATYYADSVTD**RFTISRDDSESMLYL QMNNLKTEDTAMYYCVR**HGRRDLLYSMDF**WGQGTSVTVS S | SEQ ID NO: 36 |
| 569D11B5 | EVQLIESGGGLVQPKGSLKLSCAASGFNFNAYAMNWVRQAP GKGLEWVARIRSKSNDFATYYADSVKGRFTISRDDSENMLF LQMNNLKTEDTAMYYCVRHGRRDLLYSMDFWGQGTSVTV SS | SEQ ID NO: 37 |
| 589D7C7 | EVQLVESGGGLVQPKGSLKLSCAASGFNFNAYAMNWVRQAP GKGLEWVARIRSKSNNFATYYADSVKDRFTISRDDSESMVY LEMNNLKTEDTAMYYCVRHGRRDLLYSMDFWGQGTSVTV SS | SEQ ID NO: 38 |

| **Sequences of the light-chain variable regions of hybridoma antibodies** | | |
|---|---|---|
| Clone number | Sequences (CDR regions are underlined) | Sequence Number |
| 27B9-1G3 | EIVLTQSPTTMAASPGEKITLACSASSIISSNYLHWYQQKPGFS PKLLIYRTSSLASGVPARFSGSGSGTSYSLTIGTMEAEDVATY YCQQGSSIPRTFGGGTKLEIK | SEQ ID NO:54 |
| 559E1B10 | DIVMTQSPATLSVTPGDRVSLSCRASQSISDYLHWYQQKSHE SPRLLLKYVSQSISGIPSRFSGSGSGSDFTLTINSVEPEDVGVYY CQNGHSFPLTFGAGTKLELK | SEQ ID NO:53 |
| 563E10E12 | DVVMTQAAFSNPVTLGTSTSISCRSSKSLLHSNGITYLFWYL QKPGQSPQLLIYQMSNLASGVPDRFSGSGSGTDFTLRISRVEA EDVGVYYCAQSLELPLTFGGGTKLEIK | SEQ ID NO:50 |
| 569D11B5 | DIVMTQAAFSNPVTLGTSASISCRSSKSLLHSNGITYLFWYLQ KPGQSPQLLIYQMSNLASGVPDRFSGSGSGTDFTLRISRVEAE DVGVYYCAQSLELPLTFGGGTKLEIK | SEQ ID NO:51 |
| 589D7C7 | DIVMTQAAFSNPVTLGTSASISCRSSKSLLHSNGITYLFWYLQ KPGQSPQLLIYQMSNLASGVPDRFSGSGSGTDFTLRISRVEAE DVAVYYCAQSLELPLTFGGGTKLEIK | SEQ ID NO:52 |

**Table 5. Sequences of Constant Regions of Chimeric and Humanized Antibodies**

| Sequence name | Heavy chain constant region | Light chain constant region |
|---|---|---|
| hIgG1/K |  (SEQ ID NO: 85) |  (SEQ ID NO: 86) |
| mIgG2a | AKTTAPSVYPLAPVCGDTTGSSVTLGCLVKGYFPEP VTLTWNSGSLSSGVHTFPAVLQSDLYTLSSSVTVTSS TWPSQSITCNVAHPASSTKVDKKIEPRGPTIKPCPPC KCPAPNLLGGPSVFIFPPKIKDVLMISLSPIVTCVVV DVSEDDPDVQISWFVNNVEVHTAQTQTHREDYNS TLRVVSALPIQHQDWMSGKEFKCKVNNKDLPAPIE RTISKPKGSVRAPQVYVLPPPEEEMTKKQVTLTCM VTDFMPEDIYVEWTNNGKTELNYKNTEPVLDSDG SYFMYSKLRVEKKNWVERNSYSCSVVHEGLHNHH TTKSFSRTPGK (SEQ ID NO: 83) | RADAAPTVSIFPPSSE QLTSGGASVVCFLN NFYPKDINVKWKID GSERQNGVLNSWTD QDSKDSTYSMSSTLT LTKDEYERHNSYTC EATHKTSTSPIVKSF NRNEC (SEQ ID NO: 84) |
| VLPLL | ASTKGPSVFPLAPSSKSTSGGTAALGCLVKDYFPEP VTVSWNSGALTSGVHTFPAVLQSSGLYSLSSVVTV PSSSLGTQTYICNVNHKPSNTKVDKKVEPKSCDKT HTCPPCPAPELVGGPSVFLLPPKPKDTLMISRTPEVT CVVVDVSHEDPEVKFNWYVDGVEVHNAKTKPPEE QYNSTLRVVSVLTVLHQDWLNGKEYKCKVSNKAL PAPIEKTISKAKGQPREPQVYTLPPSRDELTKNQVS LTCLVKGFYPSDIAVEWESNGQPENNYKTTPLVLD SDGSFFLYSKLTVDKSRWQQGNVFSCSVMHEALH NHYTQKSLSLSPGK (SEQ ID NO: 87) |  (SEQ ID NO: 86) |
| DE | ASTKGPSVFPLAPSSKSTSGGTAALGCLVKDYFPEP VTVSWNSGALTSGVHTFPAVLQSSGLYSLSSVVTV PSSSLGTQTYICNVNHKPSNTKVDKKVEPKSCDKT HTCPPCPAPELLGGPDVFLFPPKPKDTLMISRTPEVT CVVVDVSHEDPEVKFNWYVDGVEVHNAKTKPRE EQYNSTYRVVSVLTVLHQDWLNGKEYKCKVSNK ALPAPEEKTISKAKGQPREPQVYTLPPSRDELTKNQ VSLTCLVKGFYPSDIAVEWESNGQPENNYKTTPPV LDSDGSFFLYSKLTVDKSRWQQGNVFSCSVMHEA LHNHYTQKSLSLSPGK (SEQ ID NO: 88) |  (SEQ ID NO: 86) |
| DLE | ASTKGPSVFPLAPSSKSTSGGTAALGCLVKDYFPEP VTVSWNSGALTSGVHTFPAVLQSSGLYSLSSVVTV PSSSLGTQTYICNVNHKPSNTKVDKKVEPKSCDKT HTCPPCPAPELLGGPDVFLFPPKPKDTLMISRTPEVT CVVVDVSHEDPEVKFNWYVDGVEVHNAKTKPRE EQYNSTYRVVSVLTVLHQDWLNGKEYKCKVSNK ALPLPEEKTISKAKGQPREPQVYTLPPSRDELTKNQ VSLTCLVKGFYPSDIAVEWESNGQPENNYKTTPPV LDSDGSFFLYSKLTVDKSRWQQGNVFSCSVMHEA LHNHYTQKSLSLSPGK (SEQ ID NO: 89) |  (SEQ ID NO: 86) |

### 3.2 Binding activity of anti-CCR8 chimeric antibodies to human CCR8 and cynomolgus CCR8

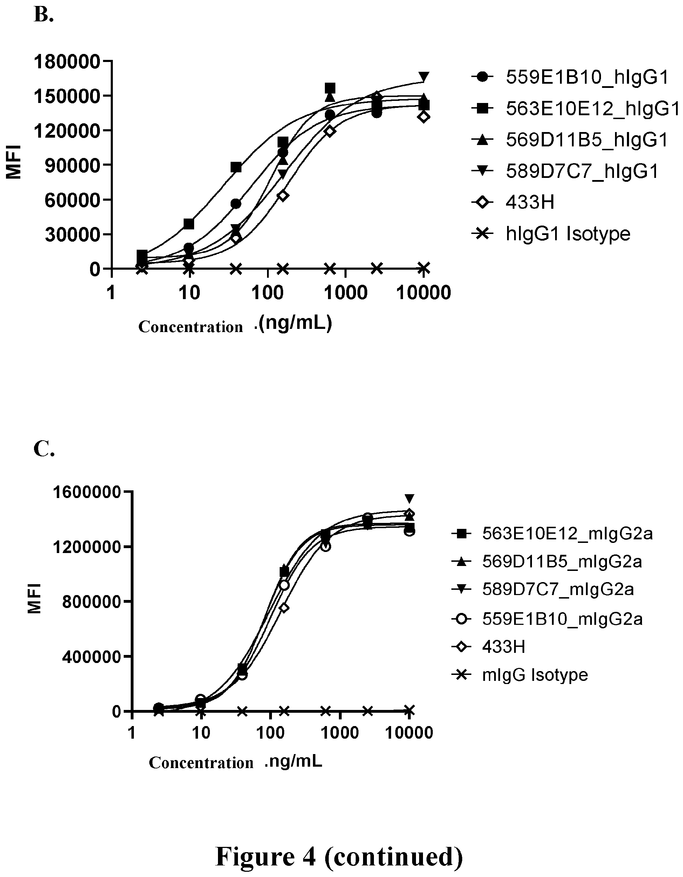

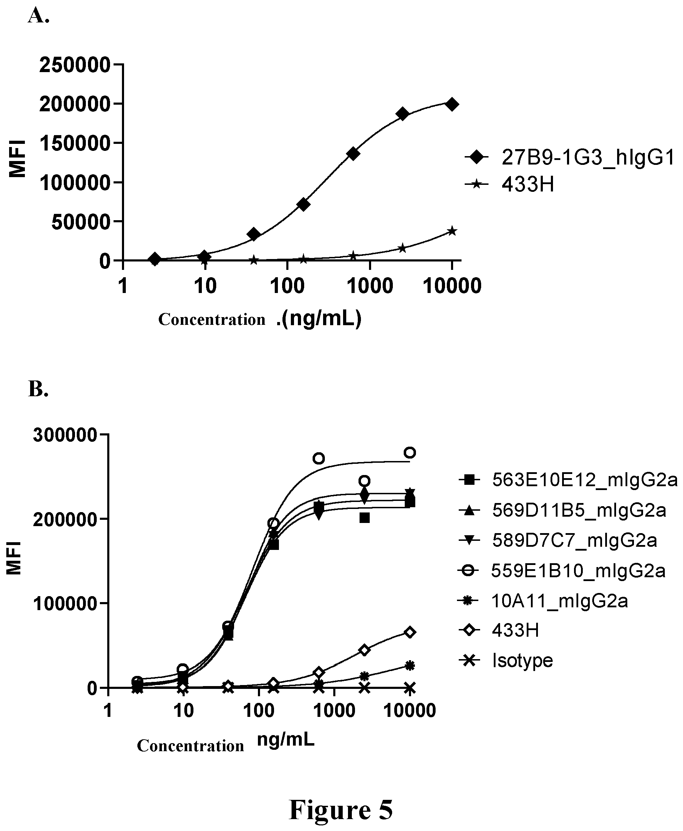

The binding activity of anti-CCR8 chimeric antibodies to human and cynomolgus CCR8s was detected using the approach from the binding assay in **Example 2-2.1.** The results are shown in the Figs. 4 and 5 and in Table 6. EC₅₀ values of the binding activity of the hIgG1- and mIgG2a-subclass chimeric antibodies 27B9-1G3, 559E1B10, 563E10E12, 569D11B5, and 589D7C7 to 293F-hCCR8 cells were 27.3-273.4 ng/mL, and EC₅₀ value of the binding activity of 433H to 293F-hCCR8 cells was 153.4 ng/mL. The hIgG1-subclass chimeric antibody 27B9-1G3, the mIgG2a-subclass chimeric antibodies 563E10E12, 569D11B, 589D7C7 and 559E1B10 showed a strong binding activity to cynomolgus CCR8, with EC₅₀ values of 66.2-303.5 ng/ml, while the binding activities of 433H and 10A11_hIgG1 to cynomolgus CCR8 were weaker, with EC₅₀ values of 14580.5 and 6309.0 ng/ml, respectively.

**Table 6. Binding Activity of Anti-CCR8 Chimeric Antibodies to Human CCR8 and Cynomolgus CCR8**

| Antibody number | **293F-hCCR8** EC₅₀, ng/mL | **293T-cynoCCR8** EC₅₀, ng/mL |
|---|---|---|
| 27B9-1G3_hIgG1 | 273.4 | 305.5 |
| 559E1B10_hIgG1 | 61.7 | NT |
| 563E10E12_hIgG1 | 27.3 | NT |
| 569D11B5_hIgG1 | 112.8 | NT |
| 589D7C7_hIgG1 | 164.3 | NT |
| 559E1B10_mIgG2a | 100.4 | 81.9 |
| 563E10E12_mIgG2a | 83.3 | 66.4 |
| 569D11B5_mIgG2a | 83.5 | 72.0 |
| 589D7C7_mIgG2a | 94.7 | 66.2 |
| 433H | 153.4 | 14580.5 |
| 10A11_hIgG1 | NT | 6309.0 |
| hIgG1 isotype | N/A | N/A |

| | | |
|---|---|---|
| NT: No detection; N/A: No binding activity or poor binding activity, no valid EC₅₀ value obtained. | | |

### 3.3 Receptor ligand blocking assay of anti-CCR8 chimeric antibodies against CCL1

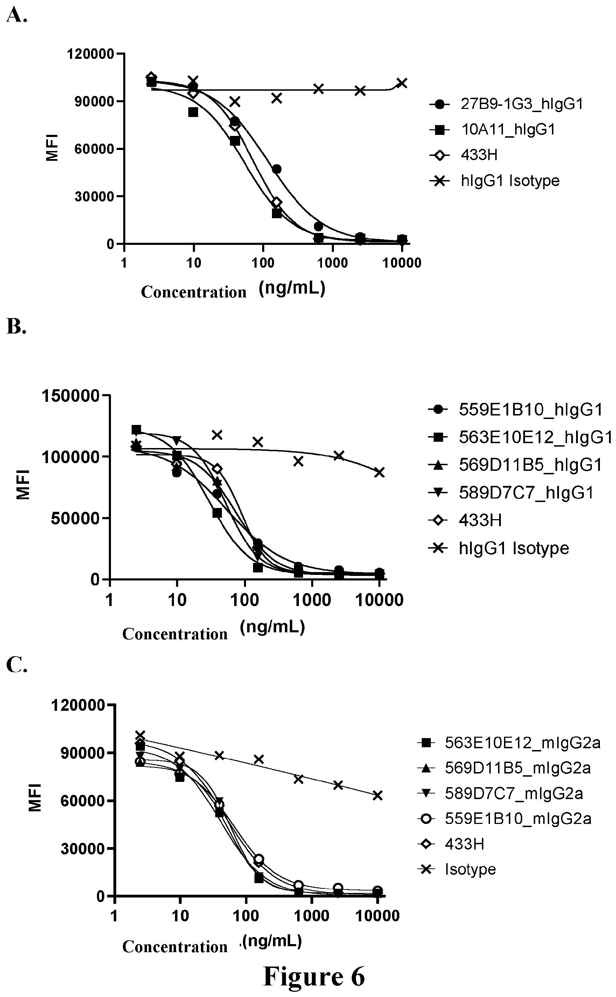

The receptor ligand blocking activity of anti-CCR8 chimeric antibodies against CCL1 was detected using the approach from **Example 2-2.2.** The results are shown in Fig. 6 and Table 7. Either hIgG1- or mIgG2a-subclass chimeric antibodies block the binding of CCL1 to 293F-hCCR8 in a dose dependent manner, with the IC₅₀ values of blocking activity being 29.5-273.4 ng/mL and the IC₅₀ values of blocking activity against 433H and 10A11_hIgG1 being 70.5 and 54.6ng/mL.

**Table 7. The receptor ligand blocking Activity of Anti-CCR8 Chimeric Antibody against CCL1**

| Clone number | 293F-hCCR8 IC₅₀, ng/mL |
|---|---|
| 27B9-1G3_hIgG1 | 273.4 |
| 559E1B10_hIgG1 | 54.9 |
| 563E10E12_hIgG1 | 29.5 |
| 569D11B5_hIgG1 | 74.8 |
| 589D7C7_hIgG1 | 52.6 |
| 559E1B10 _mIgG2a | 64.9 |
| 563E10E12_mIgG2a | 41.1 |
| 569D11B5_mIgG2a | 54.4 |
| 589D7C7_mIgG2a | 57.9 |
| 10A11_hIgG1 | 54.6 |
| 433H | 70.5 |
| IgG1 isotype | N/A |

| | |
|---|---|
| N/A: No binding activity, no valid EC₅₀ value obtained | |

### 3.4 Blockade of 1-induced β-arrestin recruitment by anti-CCR8 chimeric antibodies

CCL1 induces chemotaxis and receptor endocytosis through binding to CCR8, the latter being dependent on expression and recruitment of β-arrestin, and an β-arrestin assay is used to detect the role of CCL1 in CCR8 activation (James M Fox et al., Structure/function relationships of CCR8 agonists and antagonists. Amino-terminal extension of CCL1 by a single amino acid generates a partial agonist. J Biol Chem. 2006 Dec 1;281(48): 36652-61. doi: 10.1074/jbc.M605584200.and Libao Liu et al., Biological characterization of ligands targeting the human CC chemokine receptor 8 (CCR8) reveals the biased signaling properties of small molecule agonists. Biochem Pharmacol. 2021 Jun;188:114565. doi: 10.1016/j.bcp.2021.114565). Blockade of CCL1-induced β-arrestin recruitment by anti-CCR8 antibodies was detected using Tango-H_CCR8-CHO-K1 cells (Genomeditech (Shanghai) Co., Ltd., GM-C09028). Blockade of CCL1-induced β-arrestin recruitment by anti-CCR8 antibodies was detected using the experimental approach provided by Genomeditech (Shanghai) Co., Ltd. The detailed procedures are as follows:
Tango-H_CCR8-CHO-K1 cells were seeded into cell culture flasks, followed by adding doxycycline (Selleck, S4163-100mg) to the culture medium at a final concentration of 10 µg/mL, and continuously cultured for about 48 hours, to induce expression of human CCR8 by doxycycline. The post-induction Tango-H_CCR8-CHO-K1 cells were digested with Trypsin containing 0.25% EDTA (Gibco, 25200072) and centrifuged at 200×g for 5 min with the supernatant discarded. The cells were resuspended with fresh F12K complete medium (Gibco, 21127022), detected for cell viability, counted, adjusted further with the fresh medium to 5×10⁵ cells/mL, seeded at 100 µL/well into a white opaque plate, placed in a incubator at 37°C, 5% CO₂, and cultured overnight to allow the cells to adhere. The anti-CCR8 antibodies, the positive control antibody, and the hIgG1 isotype (Biointron Biotechnology Co., Ltd., B117901) were diluted with F12K complete medium to the 2-fold initial working concentrations and serially diluted with the F12K complete medium. The culture media in Tango-H_CCR8-CHO-K1 cell plates was gently discarded and the serially diluted antibodies were added at 50 µL per well. Then 50 µL of human CCL1 (R&D, 272-I) solution diluted with F12K complete medium to the final concentration of 20 nM was added to each well. After incubation at 37°C, 5% CO₂ for 6 hours, the plate was equilibrated for at least 15 minutes. 100 µL of the luciferase substrate solution (Vazyme, DD1203) was added to each well, mixed and then incubated at room temperature in the dark for 5 min. Values of the relative light units (RLUs) were read with a microplate reader. The experimental data were analyzed with the software Graphpad Prism 8.0. The dose-effect curve of the antibody was fitted using the chosen four-parameter regression model, with the logarithms of the antibody concentrations on the x-axis and the corresponding RLU values on the y-axis.

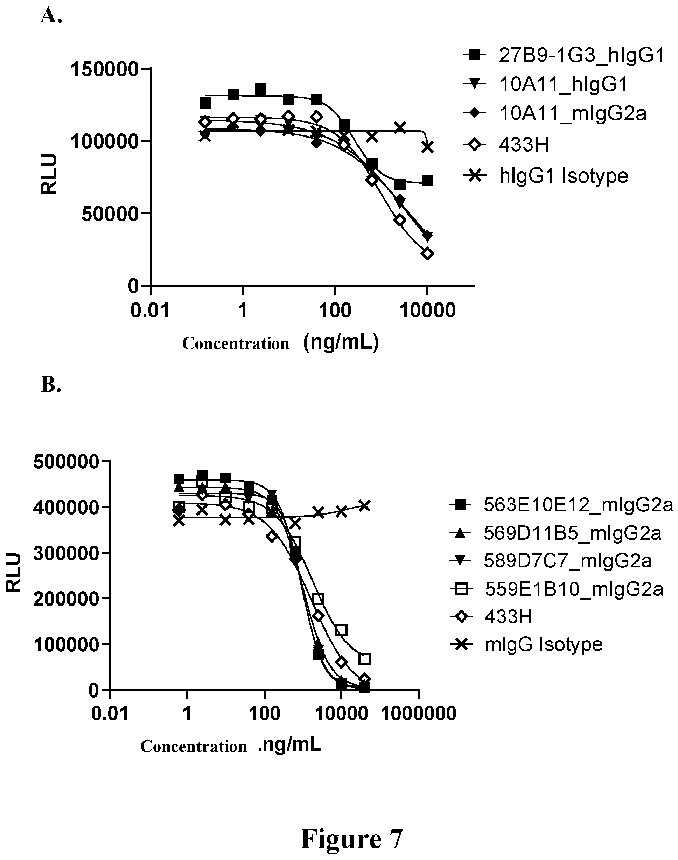

As shown in Fig. 7 and Table 8, the hIgG1- or mIgG2a-subclass chimeric antibodies 27B9-1G3, 563E10E12, 569D11B5, 589D7C7, 559C12G12 and 559E1B10 block CCL1-induced β-arrestin recruitment, with IC₅₀ values of 260.2-1757.0 ng/mL, and IC₅₀ values of 433H, 10A11_hIgG1 and 10A11_mIgG2a were 1291.5, 3268.0 and 3250.0 ng/mL.

**Table 8 Blockade of CCL1-induced β-arrestin recruitment by anti-CCR8 chimeric antibodies**

| Antibody name | **IC₅₀, ng/mL** |
|---|---|
| 27B9-1G3_hIgG1 | 260.2 |
| 563E10E12_mIgG2a | 902.3 |
| 569D11B5_mIgG2a | 996.5 |
| 589D7C7_mIgG2a | 992.8 |
| 559E1B10_mIgG2a | 1757.0 |
| 433H | 1291.5 |
| 10A11_hIgG1 | 3268.0 |
| 10A11_mIgG2a | 3250.0 |
| hIgG1 isotype | N/A |

| | |
|---|---|
| N/A: No blocking activity or weaker blocking activity, no valid IC₅₀ value obtained | |

### Example 4. Humanization of Anti-CCR8 Antibodies

CDRs were determined with Kabat numbering, human germline genes with the highest homology to the murine sequences were selected as the antibody frameworks, and CDRs in the murine sequences is grafted onto the human antibody frameworks. According to the importance of amino acid residues, back mutation was introduced, that is, some key amino acids in the grafted framework region were back mutated into the corresponding original mouse amino acids. Several variants were designed for the heavy- and light- chains, respectively.

### 4.1 Humanization of the anti-CCR8 antibody 27B9-1G3

The sequences of the murine variable regions and the amino acid sequences of the selected human germline gene are shown in the following Table 9 and the humanized heavy- and light-chain sequences designed thereby are shown in Table 10.

The humanized heavy- and light-chains were combined to obtain the following humanized antibodies:

| Names of the humanized antibodies | Number of the heavy-chain variable region | Number of the light-chain variable region |
|---|---|---|
| 27B9-1G3_ hzH1L1_hIg G1 | 27B9-1G3_hzVH01 | 27B9-1G3_hzVL01 |
| 27B9-1G3_ hzH1L2_hIg G1 | 27B9-1G3_hzVH01 | 27B9-1G3_hzVL02 |
| 27B9-1G3_ hzH1L3_hIg G1 | 27B9-1G3_hzVH01 | 27B9-1G3_hzVL03 |
| 27B9-1G3_ hzH2L1_hIg G1 | 27B9-1G3_hzVH02 | 27B9-1G3_hzVL01 |
| 27B9-1G3_ hzH2L2_hIg G1 | 27B9-1G3_hzVH02 | 27B9-1G3_hzVL02 |
| 27B9-1G3_ hzH2L3_hIg G1 | 27B9-1G3_hzVH02 | 27B9-1G3_hzVL03 |
| 27B9-1G3_ hzH3L1_hIg G1 | 27B9-1G3_hzVH03 | 27B9-1G3_hzVL01 |
| 27B9-1G3_ hzH3L2_hIg G1 | 27B9-1G3_hzVH03 | 27B9-1G3_hzVL02 |
| 27B9-1G3_ hzH3L3_hIg G1 | 27B9-1G3_hzVH03 | 27B9-1G3_hzVL03 |
| 27B9-1G3_ hzH4L1_hIg G1 | 27B9-1G3_hzVH04 | 27B9-1G3_hzVL01 |
| 27B9-1G3_ hzH4L2_hIg G1 | 27B9-1G3_hzVH04 | 27B9-1G3_hzVL02 |
| 27B9-1G3_ hzH4L3_hIg G1 | 27B9-1G3_hzVH04 | 27B9-1G3_hzVL03 |

### 4.2 Humanization of the anti-CCR8 antibody 559E1B10

The sequences of the murine variable regions and the amino acid sequences of the selected human germline gene are shown in the following Table 11 and the humanized heavy- and light-chain sequences designed thereby are shown in Table 12.

The humanized heavy- and light-chains were combined to obtain the following humanized antibodies:

| Names of the humanized antibodies | Heavy chain | Light chain |
|---|---|---|
| 559E1B10_hzH0L0_hIgG1 | 559E1B10_hzH0 | 559E1B10_hzL0 |
| 559E1B10_hzH1L0_hIgG1 | 559E1B10_hzH1 | 559E1B10_hzL0 |
| 559E1B10_hzH2L0_hIgG1 | 559E1B10_hzH2 | 559E1B10_hzL0 |
| 559E1B10_hzH3L0_hIgG1 | 559E1B10_hzH3 | 559E1B10_hzL0 |
| 559E1B10_hzH0L1_hIgG1 | 559E1B10_hzH0 | 559E1B10_hzL1 |
| 559E1B10_hzH1L1_hIgG1 | 559E1B10_hzH1 | 559E1B10_hzL1 |
| 559E1B10_hzH2L1_hIgG1 | 559E1B10_hzH2 | 559E1B10_hzL1 |
| 559E1B10_hzH3L1_hIgG1 | 559E1B10_hzH3 | 559E1B10_hzL1 |
| 559E1B10_hzH0L2_hIgG1 | 559E1B10_hzH0 | 559E1B10_hzL2 |
| 559E1B10_hzH1L2_hIgG1 | 559E1B10_hzH1 | 559E1B10_hzL2 |
| 559E1B10_hzH2L2_hIgG1 | 559E1B10_hzH2 | 559E1B10_hzL2 |
| 559E1B10_hzH3L2_hIgG1 | 559E1B10_hzH3 | 559E1B10_hzL2 |

### 4.3 Humanization of the anti-CCR8 antibodies 563E10E12, 569D11B5, and 589D7C7

The sequences of the three antibody clones 563E10E12, 569D11B5, and 589D7C7 are similar to each other and considered to be a family of sequences. Thus, it is considered to merge the humanization of the three antibodies collectively. The sequences of the murine variable regions and the amino acid sequences of the selected human germline gene are shown in the following Table 13 and the humanized heavy- and light-chain sequences designed thereby are shown in Table 14.

The humanized heavy- and light-chains were combined to obtain the following humanized antibodies:

| Names of the humanized antibodies | Heavy chain | Light chain |
|---|---|---|
| 563E10E12_hzH0L0_hIgG1 | 563E10E12_hzH0 | 563E10E12_hzL0 |
| 563E10E12_hzH1L0_hIgG1 | 563E10E12_hzH1 | 563E10E12_hzL0 |
| 563E10E12_hzH2L0_hIgG1 | 563E10E12_hzH2 | 563E10E12_hzL0 |
| 563E10E12_hzH3L0_hIgG1 | 563E10E12_hzH3 | 563E10E12_hzL0 |
| 563E10E12_hzH4L0_hIgG1 | 563E10E12_hzH4 | 563E10E12_hzL0 |
| 563E10E12_hzH5L0_hIgG1 | 563E10E12_hzH5 | 563E10E12_hzL0 |
| 563E10E12_hzH6L0_hIgG1 | 563E10E12_hzH6 | 563E10E12_hzL0 |
| 563E10E12_hzH1L1_hIgG1 | 563E10E12_hzH1 | 563E10E12_hzL1 |
| 563E10E12_hzH2L1_hIgG1 | 563E10E12_hzH2 | 563E10E12_hzL1 |
| 563E10E12_hzH3L1_hIgG1 | 563E10E12_hzH3 | 563E10E12_hzL1 |
| 563E10E12_hzH4L1_hIgG1 | 563E10E12_hzH4 | 563E10E12_hzL1 |
| 563E10E12_hzH5L1_hIgG1 | 563E10E12_hzH5 | 563E10E12_hzL1 |
| 563E10E12_hzH6L1_hIgG1 | 563E10E12_hzH6 | 563E10E12_hzL1 |

**Table 9 Scheme for Humanization of the Antibody 27B9-1G3**

| Name | FR1 | CDR1 | FR2 | CDR2 | FR3 | CDR3 | FR4 |
|---|---|---|---|---|---|---|---|
| 27B9-1G3_mVL | EIVLTQSPTTMAAS PGEKITLAC | SASSIISSN YLH | WYQQKPG FSPKLLIY | RTSSLAS | GVPARFSGSGSGTS YSLTIGTMEAEDV ATYYC | QQGSSIPR T | FGGGTKL EIK |
| IGKV3-11*01 | EIVLTQSPATLSLSP GERATLSC | RASQSVS SYLA | WYQQKPG QAPRLLIY | DASNRAT | GIPARFSGSGSGTD FTLTISSLEPEDFAV YYC | | |
| 27B9-1G3_mVH | EIQLQQSGPELEKP GASVKISCKASGYS FT | GYNMN | WVKKSNG KSLEWIG | NIDPYYGG TNYNQKFK G | KATLTVDKSSSTA YMQLKSLTSEDSA VYYCAR | ETWFITTG VIKKGFY FDY | WGQGTTL TVSS |
| IGHV169*06 | QVQLVQSGAEVKK PGSSVKVSCKASG GTFS | SYAIS | WVRQAPG QGLEWMG | GIIPIFGTA NYAQKFQ G | RVTITADKSTSTAY MELSSLRSEDTAV YYCAR | | |

| | | | | | | | |
|---|---|---|---|---|---|---|---|
| Note: refer to SEQ ID NO: 90 for the sequence of IGKV3-11*01, and to SEQ ID NO: 91 for the sequence of IGHV169*06 | | | | | | | |

**Table 10 Sequences of the Humanized Heavy- and Light-Chains of the Antibody 27B9-1G3**

| Sequence number of VH and VL | Sequence | **Sequence number** | Constant region |
|---|---|---|---|
| 27B9-1G3_hzVL01 | EIVLTQSPATLSLSPGERATLSCSASSIISSNYLHWYQQKPGQAPRLLIYRTSSLASGIP ARFSGSGSGTDFTLTISSLEPEDFAVYYCQQGSSIPRTFGGGTKVEIK | SEQ ID NO: 47 | hK |
| 27B9-1G3_hzVL02 | EIVLTQSPATLSLSPGERATLACSASSIISSNYLHWYQQKPGQAPRLLIYRTSSLASGIP ARFSGSGSGTDYTLTISSLEPEDFAVYYCQQGSSIPRTFGGGTKVEIK | SEQ ID NO: 48 | hK |
| 27B9-1G3_hzVL03 | EIVLTQSPATLSLSPGERATLACSASSIISSNYLHWYQQKPGQSPRLLIYRTSSLASGVP ARFSGSGSGTSYTLTISSLEPEDFAVYYCQQGSSIPRTFGGGTKVEIK | SEQ ID NO: 49 | hK |
| 27B9-1G3_hzVH01 | QVQLVQSGAEVKKPGSSVKVSCKASGGTFSGYNMNWVRQAPGQGLEWMGNIDPY YGGTNYNQKFKGRVTITADKSTSTAYMELSSLRSEDTAVYYCARETWFITTGVIKKG FYFDYWGQGTTVTVSS | SEQ ID NO: 32 | hIgG1 |
| 27B9-1G3_hzVH02 | QVQLVQSGAEVKKPGSSVKVSCKASGYSFTGYNMNWVRQAPGQGLEWMGNIDPY YGGTNYNQKFKGRVTITADKSTSTAYMELSSLRSEDTAVYYCARETWFITTGVIKKG FYFDYWGQGTTVTVSS | SEQ ID NO: 33 | hIgG1 |
| 27B9-1G3_hzVH03 | QVQLVQSGAEVKKPGSSVKVSCKASGGTFSGYNMNWVRQAPGQGLEWMGNIDPY YGGTNYNQKFKGKATITADKSTSTAYMELSSLRSEDTAVYYCARETWFITTGVIKK GFYFDYWGQGTTVTVSS | SEQ ID NO: 34 | hIgG1 |
| 27B9-1G3_hzVH04 | QVQLVQSGAEVKKPGSSVKVSCKASGYSFTGYNMNWVRQAPGQGLEWMGNIDPY YGGTNYNQKFKGKATITADKSTSTAYMELSSLRSEDTAVYYCARETWFITTGVIKK GFYFDYWGQGTTVTVSS | SEQ ID NO: 35 | hIgG1 |

**Table 11 Scheme for Humanization of the Antibody 559E1B10**

| Sequence name | FR1 | CDR1 | FR2 | CDR2 | FR3 | CDR3 | FR4 |
|---|---|---|---|---|---|---|---|
| 559E1B10_mVH | KVQLQQSGPVLVK PGASVKMSCKASG YTFT | DYYVN | WMKQNY GKSPEWI G | VINPHNGD TRYNQKFK G | KATLTVDKSSSTAN MELTSLTSGDSAVY YCAS | WLRRGY YFDS | WGQG TTLTV SS |
| hVH 1-46 | QVQLVQSGAEVKK PGASVKVSCKASG YTFT | SYYMH | WVRQAP GQGLEW MG | IINPSGGST SYAQKFQG | RVTMTRDTSTSTVY MELSSLRSEDTAVY YCAR | | |
| 559E1B10_mVL | DIVMTQSPATLSVT PGDRVSLSC | RASQSIS DYLH | WYQQKS HESPRLL LKY | VSQSIS | GIPSRFSGSGSGSDFT LTINSVEPEDVGVYY C | QNGHSFP LT | FGAGT KLELK |
| hVK 3-11 | EIVLTQSPATLSLSP GERATLSC | RASQSVS SYLA | WYQQKP GQAPRLL IYD | ASNRAT | GIPARFSGSGSGTDF TLTISSLEPEDFAVY YC | | |

| | | | | | | | |
|---|---|---|---|---|---|---|---|
| Note: refer to SEQ ID NO: 92 for the sequence of hVH 1-46, and to SEQ ID NO: 93 for the sequence of hVK 3-11 | | | | | | | |

**Table 12 Humanized Heavy- and Light-chain Sequences of the Antibody 559E1B10**

| Sequence number of VH and VL | Sequence | **Sequence number** | Constant region |
|---|---|---|---|
| 559E1B10_hzH0 | QVQLVQSGAEVKKPGASVKVSCKASGYTFTDYYVNWVRQAPGQGLEWMGVINPH NGDTRYNQKFKGRVTMTRDTSTSTVYMELSSLRSEDTAVYYCASWLRRGYYFDSW GQGTTVTVSS | SEQ ID NO: 28 | hIgG1 |
| 559E1B10_hzH1 | QVQLVQSGAEVKKPGASVKVSCKASGYTFTDYYVNWVRQAPGQGLEWMGVINPH NGDTRYNQKFKGRVTMTVDKSTSTVYMELSSLRSEDTAVYYCASWLRRGYYFDSW GQGTTVTVSS | SEQ ID NO: 29 | hIgG1 |
| 559E1B10_hzH2 | QVQLVQSGAEVKKPGASVKVSCKASGYTFTDYYVNWVRQAPGQGLEWIGVINPHN GDTRYNQKFKGRATLTVDKSTSTVYMELSSLRSEDTAVYYCASWLRRGYYFDSWG QGTTVTVSS | SEQ ID NO: 30 | hIgG1 |
| 559E1B10_hzH3 | KVQLVQSGAEVKKPGASVKVSCKASGYTFTDYYVNWVRQAPGQGLEWIGVINPHN GDTRYNQKFKGKATLTVDKSTSTAYMELSSLRSEDTAVYYCASWLRRGYYFDSWG QGTTVTVSS | SEQ ID NO: 31 | hIgG1 |
| 559E1B10_hzL0 | EIVLTQSPATLSLSPGERATLSCRASQSISDYLHWYQQKPGQAPRLLIYYVSQSISGIP ARFSGSGSGTDFTLTISSLEPEDFAVYYCQNGHSFPLTFGGGTKVEIK | SEQ ID NO: 44 | hK |
| 559E1B10_hzL1 | EIVLTQSPATLSLSPGERATLSCRASQSISDYLHWYQQKPGQAPRLLLKYVSQSISGIP ARFSGSGSGTDFTLTISSLEPEDFAVYYCQNGHSFPLTFGGGTKVEIK | SEQ ID NO: 45 | hK |
| 559E1B10_hzL2 | DIVMTQSPATLSLSPGERATLSCRASQSISDYLHWYQQKPGQAPRLLLKYVSQSISGI PARFSGSGSGTDFTLTISSLEPEDFAVYYCQNGHSFPLTFGGGTKVEIK | SEQ ID NO: 46 | hK |

**Table 13 Scheme for Humanization of the Antibodies 563E10E12, 569D11B5, and 589D7C7**

| Name | FR1 | CDR1 | FR2 | CDR2 | FR3 | CDR3 | FR4 |
|---|---|---|---|---|---|---|---|
| 563E10E12_mVH | EVQLIESGGGLV QPKGSLKLSCAA SGFNFN | AYAMN | WVRQAP GKGLEW VA | RIRSKSNNF ATYYADSV TD | RFTISRDDSESMLY LQMNNLKTEDTA MYYCVR | HGRRDLL YSMDF | WGQGTS VTVSS |
| 569D11B5 mVH | EVQLIESGGGLV QPKGSLKLSCAA SGFNFN | AYAMN | WVRQAP GKGLEW VA | RIRSKSNDF ATYYADSV KG | RFTISRDDSENMLF LQMNNLKTEDTA MYYCVR | HGRRDLL YSMDF | WGQGTS VTVSS |
| 589D7C7_mVH | EVQLVESGGGLV QPKGSLKLSCAA SGFNFN | AYAMN | WVRQAP GKGLEW VA | RIRSKSNNF ATYYADSV KD | RFTISRDDSESMVY LEMNNLKTEDTAM YYCVR | HGRRDLL YSMDF | WGQGTS VTVSS |
| hVH 3-73 | EVQLVESGGGLV QPGGSLKLSCAA SGFTFS | GSAMH | WVRQAS GKGLEW VG | RIRSKANS YATAYAAS VKG | RFTISRDDSKNTAY LQMNSLKTEDTAV YYCTR | | |
| 563E10E12_mVL | DVVMTQAAFSNP VTLGTSTSISC | RSSKSLL HSNGITY LF | WYLQKPG QSPQLLIY | QMSNLAS | GVPDRFSGSGSGTD FTLRISRVEAEDVG VYYC | AQSLELPL T | FGGGTK LEIK |
| 569D11B5_mVL | DIVMTQAAFSNP VTLGTSASISC | RSSKSLL HSNGITY LF | WYLQKPG QSPQLLIY | QMSNLAS | GVPDRFSGSGSGTD FTLRISRVEAEDVG VYYC | AQSLELPL T | FGGGTK LEIK |
| 589D7C7_mVL | DIVMTQAAFSNP VTLGTSASISC | RSSKSLL HSNGITY LF | WYLQKPG QSPQLLIY | QMSNLAS | GVPDRFSGSGSGTD FTLRISRVEAEDVA VYYC | AQSLELPL T | FGGGTK LEIK |
| hVK 2-28 | DIVMTQSPLSLPV TPGEPASISC | RSSQSLL HSNGYN YLD | WYLQKPG QSPQLLIY | LGSNRAS | GVPDRFSGSGSGTD FTLKISRVEAEDVG VYYC | | |

| | | | | | | | |
|---|---|---|---|---|---|---|---|
| Note: refer to SEQ ID NO: 94 for the sequence of hVH 3-73, and to SEQ ID NO: 95 for the sequence of hVK 2-28 | | | | | | | |

**Table 14 Sequences of Humanized Heavy and Light Chains of the Antibodies 563E10E12, 569D11B5, and 589D7C7**

| Sequence number of VH and VL | Sequence | **Sequence number** | Constant region |
|---|---|---|---|
| 563E10E12_hzH0 | EVQLVESGGGLVQPGGSLKLSCAASGFTFSAYAMNWVRQASGKGLEWVGRIRSKSNNFA TYYADSVTDRFTISRDDSKNTAYLQMNSLKTEDTAVYYCVRHGRRDLLYSMDFWGQGTT VTVSS | SEQ ID NO: 22 | hIgG1 |
| 563E10E12_hzH1 | EVQLVESGGGLVQPGGSLKLSCAASGFNFNAYAMNWVRQASGKGLEWVGRIRSKSNNF ATYYADSVTDRFTISRDDSKNTAYLQMNSLKTEDTAVYYCVRHGRRDLLYSMDFWGQG TTVTVSS | SEQ ID NO: 21 | hIgG1 |
| 563E10E12_hzH2 | EVQLVESGGGLVQPGGSLKLSCAASGFTFSAYAMNWVRQASGKGLEWVARIRSKSNNFA TYYADSVTDRFTISRDDSKSTLYLQMNSLKTEDTAVYYCVRHGRRDLLYSMDFWGQGTT VTVSS | SEQ ID NO: 23 | hIgG1 |
| 563E10E12_hzH3 | EVQLVESGGGLVQPGGSLKLSCAASGFNFNAYAMNWVRQASGKGLEWVARIRSKSNNF ATYYADSVTDRFTISRDDSKSTLYLQMNSLKTEDTAVYYCVRHGRRDLLYSMDFWGQGT TVTVSS | SEQ ID NO: 24 | hIgG1 |
| 563E10E12_hzH4 | EVQLVESGGGLVQPGGSLKLSCAASGFNFNAYAMNWVRQASGKGLEWVARIRSKSNDF ATYYADSVKDRFTISRDDSKSTLYLQMNSLKTEDTAVYYCVRHGRRDLLYSMDFWGQGT TVTVSS | SEQ ID NO: 25 | hIgG1 |
| 563E10E12_hzH5 | EVQLVESGGGLVQPGGSLKLSCAASGFNFNAYAMNWVRQASGKGLEWVARIRSKSNDF ATYYADSVKGRFTISRDDSKNTLYLQMNSLKTEDTAVYYCVRHGRRDLLYSMDFWGQG TTVTVSS | SEQ ID NO: 26 | hIgG1 |
| 563E10E12_hzH6 | EVQLVESGGGLVQPGGSLKLSCAASGFNFNAYAMNWVRQASGKGLEWVARIRSKSNNF ATYYADSVKDRFTISRDDSKSTVYLQMNSLKTEDTAVYYCVRHGRRDLLYSMDFWGQG TTVTVSS | SEQ ID NO: 27 | hIgG1 |
| 563E10E12_hzL0 | DIVMTQSPLSLPVTPGEPASISCRSSKSLLHSNGITYLFWYLQKPGQSPQLLIYQMSNLASG VPDRFSGSGSGTDFTLKISRVEAEDVGVYYCAQSLELPLTFGGGTKVEIK | SEQ ID NO: 42 | hK |
| 563E10E12_hzL1 | DVVMTQSPLSLPVTPGEPASISCRSSKSLLHSNGITYLFWYLQKPGQSPQLLIYQMSNLASG VPDRFSGSGSGTDFTLKISRVEAEDVGVYYCAQSLELPLTFGGGTKVEIK | SEQ ID NO: 43 | hK |

### Example 5. Functional Characterization of Humanized Anti-CCR8 Antibodies

The light- and heavy-chain variable regions of the humanized antibodies 27B9-1G3, 559E1B10 and 563E10E12 were constructed respectively into the human-Fc constant region (hIgG1/K, its sequence seen in Table 5). The corresponding genes were synthesized and sequenced to confirm consistency of their sequences with the designed sequences. Expression was performed in Expi293 cells with the method in Example 3 and purification was performed with Protein A column. Experiments on binding of human- and cynomolgus-CCR8, blocking of CCL1 binding and blocking of the CCL1-induced β-arrestin recruitment were conducted respectively to characterize the functions of the resultant antibodies.

### 5.1 Binding assay of humanized anti-CCR8 antibodies

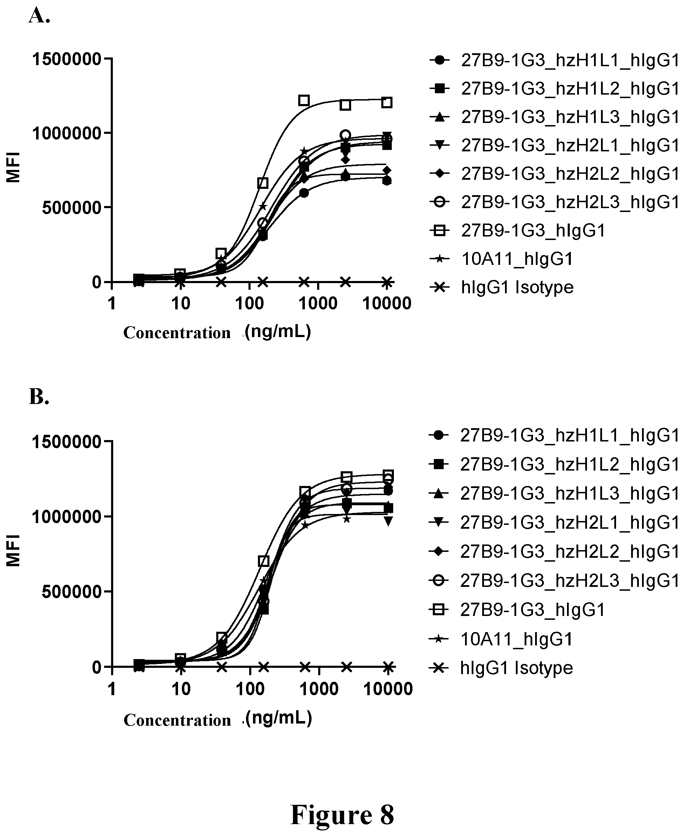

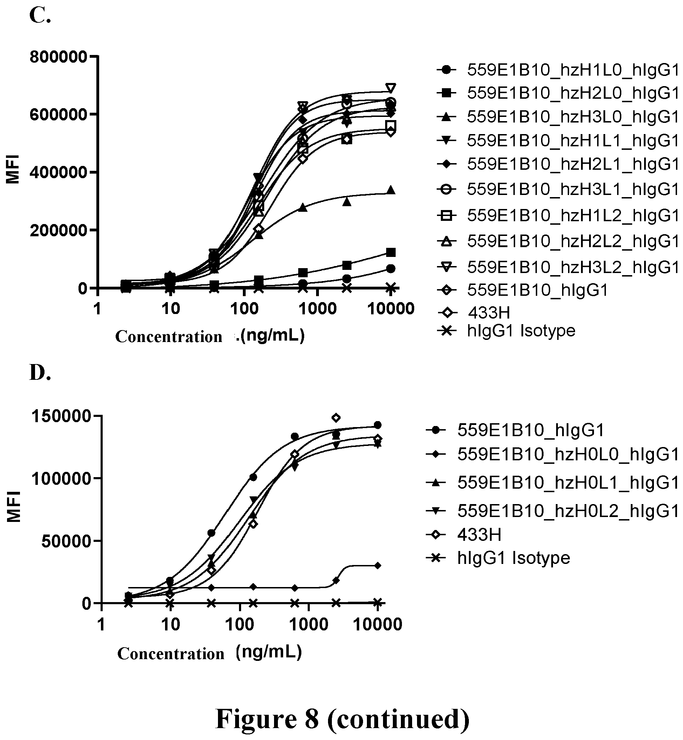

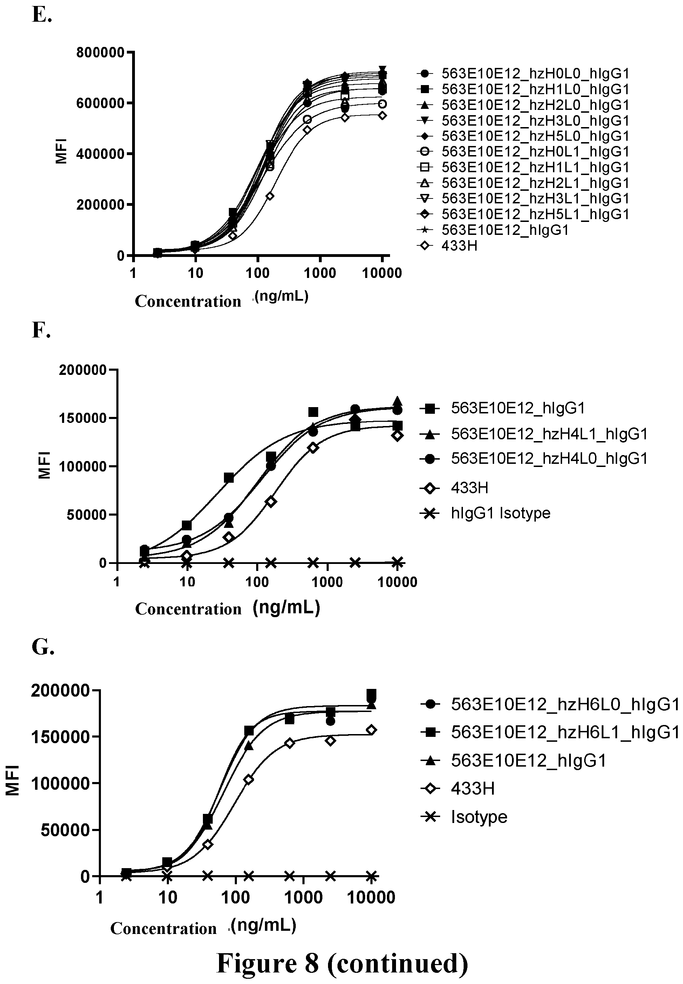

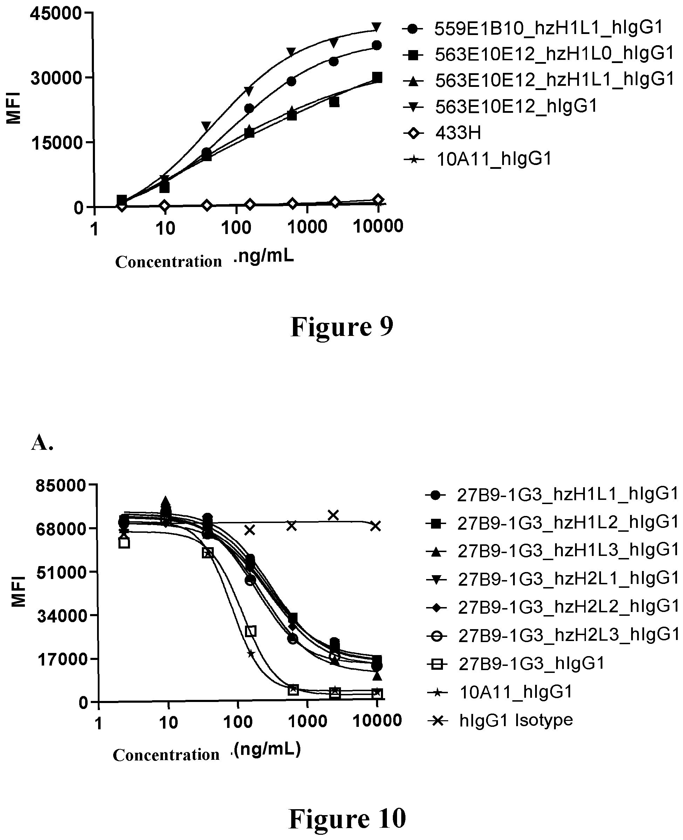

The binding activity of humanized anti-CCR8 antibodies to human CCR8 and cynomolgus CCR8 was detected using the approach in **Example 2-2.1.** The results are shown in the Figs. 8 and 9 and Table 15. The binding activities of the 27B9-1G3-humanized antibodies binding to 293F-hCCR8 is comparable to that of the chimeric parent antibody thereof. The binding activities of most of the 559E1B10- humanized antibodies binding to 293F-hCCR8 are comparable to that of the chimeric parent antibody thereof. The binding activities of most of the 563E10E12- humanized antibodies to 293F-hCCR8 are comparable to that of the chimeric parent antibody thereof. The humanized antibodies 559E1B10_hzH1L1, 563E10E12_hzH1L0, and 563E10E12_hzH1L1 show strong binding activity to 293T-cynoCCR8, while 433H and 10A11_hIgG1 show no binding activity to the cynomolgus CCR8.

**Table 15. EC₅₀ Values of Binding of Humanized Anti-CCR8 antibodies to the Human CCR8 (293F-hCCR8) and the Cynomolgus CCR8 (293T-cynoCCR8)**

| Antibody number | **Binding activity to 293F-hCCR8** | **Binding activity to 293T-cynoCCR8** |
|---|---|---|
| | **EC₅₀, ng/mL** | **EC₅₀, ng/mL** |
| 27B9-1G3_hIgG1 | 139.3 | NT |
| 27B9-1G3_hzH1L1_hIgG1 | 188.3 | NT |
| 27B9-1G3_hzH1L2_hIgG1 | 236.9 | NT |
| 27B9-1G3_hzH1L3_hIgG1 | 174.7 | NT |
| 27B9-1G3_hzH2L1_hIgG1 | 246.3 | NT |
| 27B9-1G3_hzH2L2_hIgG1 | 189.8 | NT |
| 27B9-1G3_hzH2L3_hIgG1 | 211.6 | NT |
| 27B9-1G3_hzH3L1_hIgG1 | 175.5 | NT |
| 27B9-1G3_hzH3L2_hIgG1 | 200.2 | NT |
| 27B9-1G3_hzH3L3_hIgG1 | 197.5 | NT |
| 27B9-1G3_hzH4L1_hIgG1 | 177.6 | NT |
| 27B9-1G3_hzH4L2_hIgG1 | 195.6 | NT |
| 27B9-1G3_hzH4L3_hIgG1 | 220.4 | NT |
| 559E1B10_hIgG1 | 100.9 | NT |
| 559E1B10_hzH1L0_hIgG1 | N/A | NT |
| 559E1B10_hzH2L0_hIgG1 | N/A | NT |
| 559E1B10_hzH3L0_hIgG1 | 130.0 | NT |
| 559E1B10_hzH1L1_hIgG1 | 113.5 | 87.6 |
| 559E1B10_hzH2L1_hIgG1 | 141.1 | NT |
| 559E1B10_hzH3L1_hIgG1 | 176.2 | NT |
| 559E1B10_hzH1L2_hIgG1 | 149.7 | NT |
| 559E1B10_hzH2L2_hIgG1 | 204.0 | NT |
| 559E1B10_hzH3L2_hIgG1 | 133.0 | NT |
| 559E1B10_hzH0L0_hIgG1 | N/A | NT |
| 559E1B10_hzH0L1_hIgG1 | 134.5 | NT |
| 559E1B10_hzH0L2_hIgG1 | 97.9 | NT |
| 563E10E12_hIgG1 | 73.0 | 47.3 |
| 563E10E12_hzH0L0_hIgG1 | 107.2 | NT |
| 563E10E12_hzH1L0_hIgG1 | 102.7 | 22.7 |
| 563E10E12_hzH2L0_hIgG1 | 102.7 | NT |
| 563E10E12_hzH3L0_hIgG1 | 132.7 | NT |
| 563E10E12_hzH5L0_hIgG1 | 130.0 | NT |
| 563E10E12_hzH0L1_hIgG1 | 118.2 | NT |
| 563E10E12_hzH1L1_hIgG1 | 124.5 | 26.4 |
| 563E10E12_hzH2L1_hIgG1 | 136.4 | NT |
| 563E10E12_hzH3L1_hIgG1 | 114.1 | NT |
| 563E10E12_hzH5L1_hIgG1 | 129.1 | NT |
| 563E10E12_hzH4L1_hIgG1 | 115.6 | NT |
| 563E10E12_hzH4L0_hIgG1 | 101.5 | NT |
| 563E10E12_hzH6L0_hIgG1 | 56.9 | NT |
| 563E10E12_hzH6L1_hIgG1 | 59.3 | NT |
| 10A11_hIgG1 | 139.9 | N/A |
| 433H | 156.2 | N/A |
| hIgG1 isotype | N/A | N/A |

| | | |
|---|---|---|
| NT: No Tested; N/A: No binding activity or poor binding activity, no valid EC₅₀ value. | | |

### 5.2 Receptor ligand blocking activty of humanized anti-CCR8 antibodies to CCL1

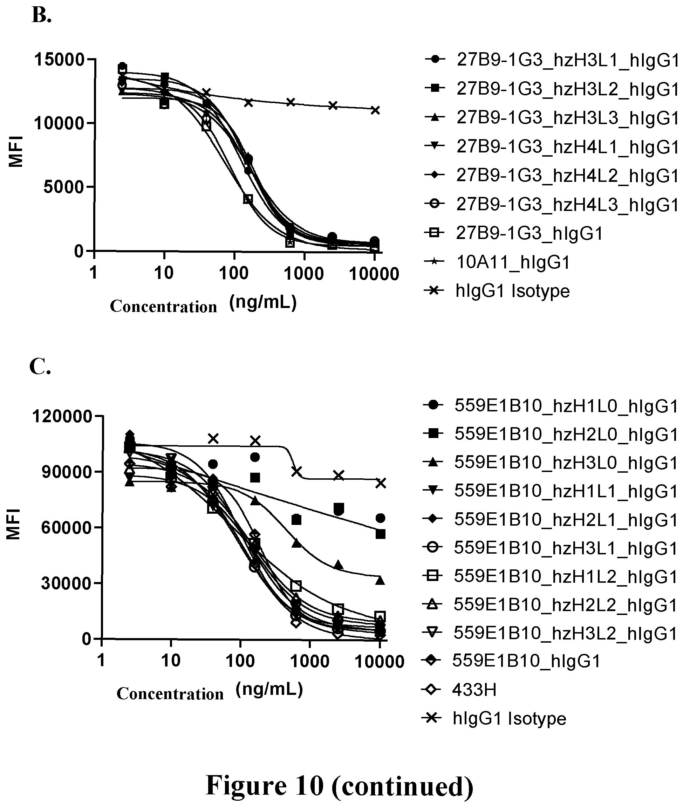

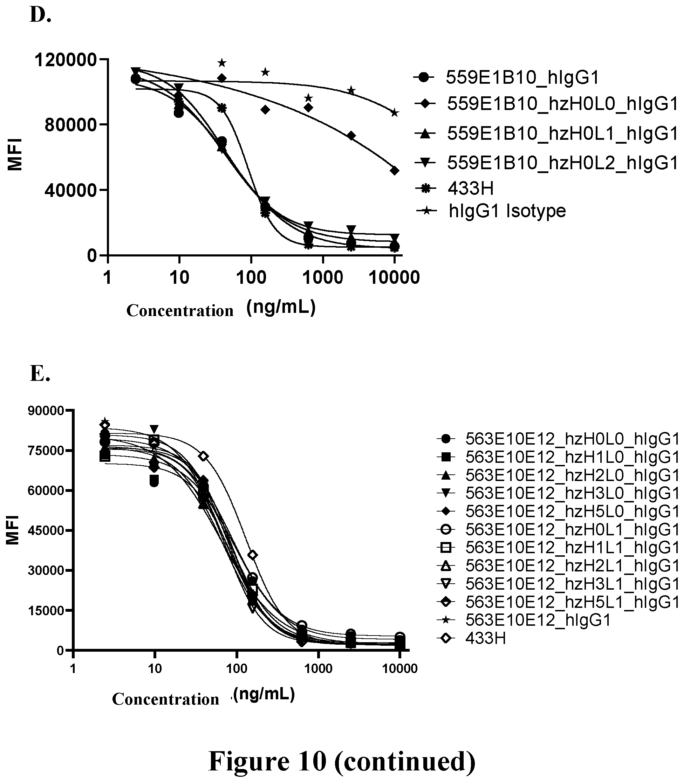

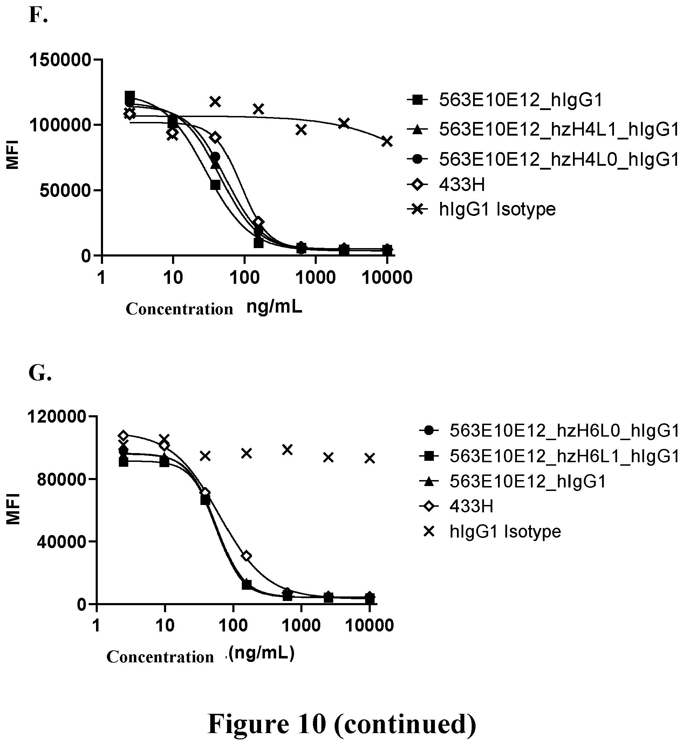

The receptor ligand blocking activity of anti-CCR8 humanized antibodies to CCL1 was detected using the approach from the receptor ligand blocking assay in **Example 2-2.2.** The results are shown in the Fig. 10 and in Table 16. Some of the humanized antibodies of 27B9-1G3, such as27B9-1G3_hzH3L1 and 27B9-1G3_hzH3L2 show blocking activities comparable to that of the chimeric parent antibody thereof. Most of the 559E1B10- humanized antibodies, such as559E1B10_hzH2L1, 559E1B10_hzH3L1, 559E1B10_hzH1L2, 559E1B10_hzH3L2, 559E1B10_hzH0L1, and 559E1B10_hzH0L2 show blocking activities comparable to that of the chimeric parent antibody thereof. All of 563E10E12- humanized antibodies show blocking activities comparable to that of the chimeric parent antibody thereof. The 559E1B10- humanized antibodies 559E1B10_hzH3L1, 559E1B10_hzH1L2, 559E1B10_hzH0L1, and 559E1B10_hzH0L2 as well as all of 563E10E12- humanized antibodies show blocking activities superior to that of 433H or similar to that of 433H

**Table 16. IC₅₀ Values of Blockade of CCL1 on Human CCR8 (293F-hCCR8) by Humanized Anti-CCR8 Antibodies**

| Clone number | **IC₅₀, ng/mL** |
|---|---|
| 27B9-1G3_hIgG1 | 95.95 |
| 27B9-1G3_hzH1L1_hIgG1 | 310.2 |
| 27B9-1G3_hzH1L2_hIgG1 | 291.2 |
| 27B9-1G3_hzH1L3_hIgG1 | 227.9 |
| 27B9-1G3_hzH2L1_hIgG1 | 268.6 |
| 27B9-1G3_hzH2L2_hIgG1 | 298.7 |
| 27B9-1G3_hzH2L3_hIgG1 | 191.9 |
| 27B9-1G3_hzH3L1_hIgG1 | 121.1 |
| 27B9-1G3_hzH3L2_hIgG1 | 149.9 |
| 27B9-1G3_hzH3L3_hIgG1 | 188.9 |
| 27B9-1G3_hzH4L1_hIgG1 | 162.8 |
| 27B9-1G3_hzH4L2_hIgG1 | 161.9 |
| 27B9-1G3_hzH4L3_hIgG1 | 167.3 |
| 559E1B10_hIgG1 | 124.6 |
| 559E1B10_hzH1L0_hIgG1 | N/A |
| 559E1B10_hzH2L0_hIgG1 | N/A |
| 559E1B10_hzH3L0_hIgG1 | 442.8 |
| 559E1B10_hzH1L1_hIgG1 | 149.7 |
| 559E1B10_hzH2L1_hIgG1 | 101.7 |
| 559E1B10_hzH3L1_hIgG1 | 88.0 |
| 559E1B10_hzH1L2_hIgG1 | 76.9 |
| 559E1B10_hzH2L2_hIgG1 | 139.5 |
| 559E1B10_hzH3L2_hIgG1 | 121.0 |
| 559E1B10_hzH0L0_hIgG1 | N/A |
| 559E1B10_hzH0L1_hIgG1 | 43.8 |
| 559E1B10_hzH0L2_hIgG1 | 41.3 |
| 563E10E12_hIgG1 | 51.3 |
| 563E10E12_hzH0L0_hIgG1 | 95.8 |
| 563E10E12_hzH1L0_hIgG1 | 85.2 |
| 563E10E12_hzH2L0_hIgG1 | 68.9 |
| 563E10E12_hzH3L0_hIgG1 | 76.1 |
| 563E10E12_hzH5L0_hIgG1 | 85.1 |
| 563E10E12_hzH0L1_hIgG1 | 86.2 |
| 563E10E12_hzH1L1_hIgG1 | 83.5 |
| 563E10E12_hzH2L1_hIgG1 | 64.0 |
| 563E10E12_hzH3L1_hIgG1 | 73.2 |
| 563E10E12_hzH5L1_hIgG1 | 87.8 |
| 563E10E12_hzH4L0_hIgG1 | 54.4 |
| 563E10E12_hzH4L1_hIgG1 | 45.8 |
| 563E10E12_hzH6L0_hIgG1 | 54.3 |
| 563E10E12_hzH6L1_hIgG1 | 57.9 |
| 10A11_hIgG1 | 85.2 |
| 433H | 96.5 |
| hIgG1 isotype | N/A |

| | |
|---|---|
| N/A: The binding activity is poorer and no valid EC₅₀ value obtained. | |

### 5.3. The experiment on blockage of CCL1-induced β-arrestin recruitment by humanized anti-CCR8 antibodies

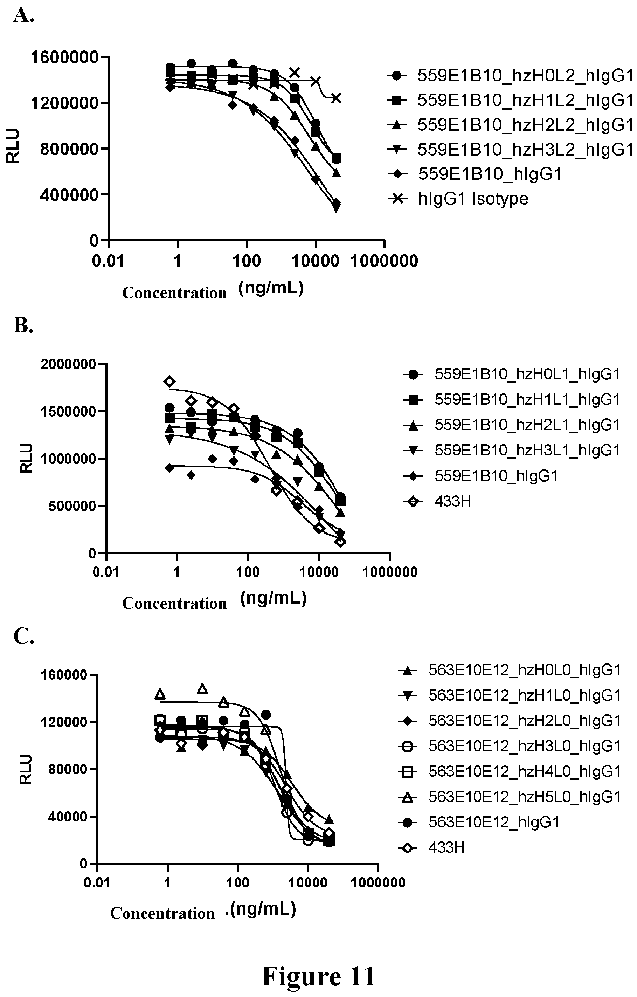

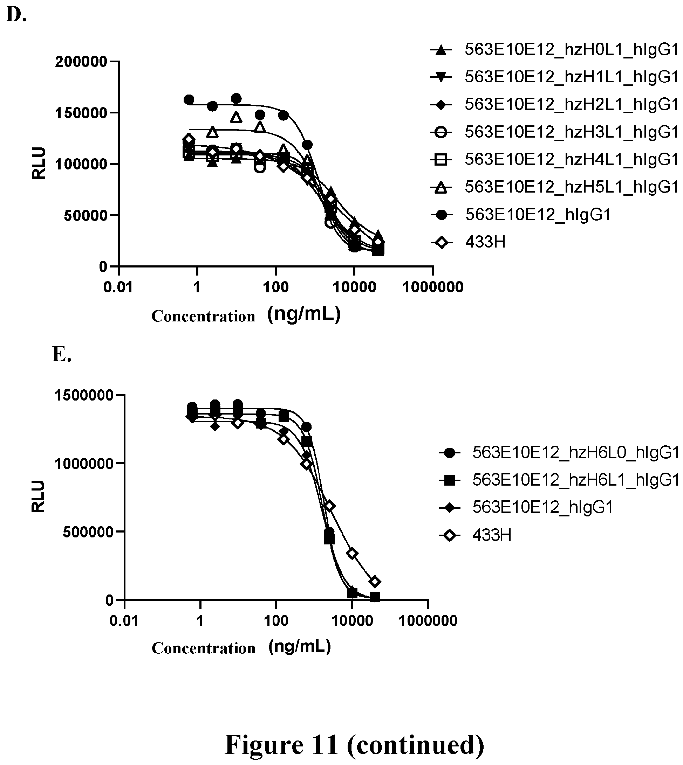

The blockage of CCL1-induced β-arrestin recruitment by the humanized anti-CCR8 antibody was determined using the approach in **Example 3-3.4.** The results are shown in the Fig. 11 and in Table 17. Some of 27B9-1G3 humanized antibodies, such as 27B9-1G3_hzH3L1 and 27B9-1G3_hzH3L2 show blocking activities comparable to that of the chimeric parent antibody thereof. Most of 559E1B10- humanized antibodies, such as 559E1B10_hzH2L1, 559E1B10_hzH3L1, 559E1B10_hzH1L2, 559E1B10_hzH3L2, 559E1B10_hzH0L1, and 559E1B10_hzH0L2 show blocking activities comparable to that of the chimeric parent antibody thereof. All of 563E10E12- humanized antibodies show blocking activities comparable to that of the chimeric parent antibody thereof. The 559E1B10- humanized antibodies 559E1B10_hzH3L1, 559E1B10_hzH1L2, 559E1B10_hzH0L1, and 559E1B10_hzH0L2 as well as all of the 563E10E12- humanized antibodies show blocking activities superior to that of 433H or similar to that of 433H

**Table 17. IC₅₀ Values of Blockade of CCL1-Induced β-Arrestin Recruitment by Humanized Anti-CCR8 Antibodies**

| Antibody number | **IC₅₀, ng/mL** |
|---|---|
| 559E1B10_hIgG1 | 10206 |
| 559E1B10_hzH0L2_hIgG1 | 11178 |
| 559E1B10_hzH1L2_hIgG1 | 7335 |
| 559E1B10_hzH2L2_hIgG1 | 6685 |
| 559E1B10_hzH3L2_hIgG1 | 71150 |
| 559E1B10_hzH0L1_hIgG1 | 118024 |
| 559E1B10_hzH1L1_hIgG1 | 28064 |
| 559E1B10_hzH2L1_hIgG1 | 46450 |
| 559E1B10_hzH3L1_hIgG1 | 14794 |
| 563E10E12_hIgG1 | 1717 |
| 563E10E12_hzH0L0_hIgG1 | 3514 |
| 563E10E12_hzH1L0_hIgG1 | 1464 |
| 563E10E12_hzH2L0_hIgG1 | 1458 |
| 563E10E12_hzH3L0_hIgG1 | 1163 |
| 563E10E12_hzH4L0_hIgG1 | 1494 |
| 563E10E12_hzH5L0_hIgG1 | 1697 |
| 563E10E12_hzH0L1_hIgG1 | 3652 |
| 563E10E12_hzH1L1_hIgG1 | 1615 |
| 563E10E12_hzH2L1_hIgG1 | 1524 |
| 563E10E12_hzH3L1_hIgG1 | 1527 |
| 563E10E12_hzH4L1_hIgG1 | 2101 |
| 563E10E12_hzH5L1_hIgG1 | 1375 |
| 563E10E12_hzH6L0_hIgG1 | 1839 |
| 563E10E12_hzH6L1_hIgG1 | 1652 |
| 433H | 2364 |
| hIgG1 isotype | N/A |

| | |
|---|---|
| N/A: Not applicable, no blocking activity. | |

### 5.4 Determination of the humanized anti-CCR8 antibody-dependent FcyRIIIa activation using Jurkat-human FcyRIIIa(158V)-NFAT

Based on mouse models, it has been found that anti-CCR8 antibodies suppress tumor growth mainly by eliminating Treg cells in tumor tissues through ADCC effect (Helena Van Damme et al., Therapeutic depletion of CCR8+ tumor-infiltrating regulatory T cells elicits antitumor immunity and synergizes with anti-PD-1 therapy. J Immunother Cancer. 2021 Feb;9(2):e001749. doi: 10.1136/jitc-2020-001749). The anti-CCR8 antibody-dependent FcyRIIIa activation was determined by incubating Jurkat-human FcyRIIIa(158V)-NFAT with CCR8-expressing cells. The Fab portions of the CCR8 antibodies bind to target sites on target cells binds and the Fc portions thereof bind to the Fcy receptors on effector cells, which activates the NFAT signaling pathway of the effector cells. ADCC activities of the antibodies are reflected by quantification of the luciferase activity induced through the activation of the NFAT signaling pathway.
The detailed procedures are as follows:
293F-human CCR8 cells (Shanghai ChemPartner Co., Ltd., 293F-hCCR8) were digested with Trypsin containing 0.25% EDTA (Gibco, 25200072) and centrifuged at 300 g for 5 min, and the resultant cell suspension was adjusted with DMEM medium to 3×10⁵ cells/mL, seeded at 100 µL/well into a white opaque plate, placed in a incubator at 37°C, 5% CO₂, and cultured overnight to allow the cells to adhere. The anti-CCR8 antibodies, the positive control antibody, and the hIgG1 isotype (Biointron Biotechnology Co., Ltd., B117901) were diluted with 1640 medium to the 2-fold initial working concentrations and serially diluted by 5-fold with the culture medium. The collected Jurkat-human FcγRIIIa(158V)-NFAT cells (Jiman Biotechnology (Shanghai) Co., Ltd., GM-C05619) were centrifuged at 300 g for 5 minutes, the supernatant was discarded, and 1640 medium was added to adjust the cell concentration to 3×10⁶ cells/mL. 293F-hCCR8 cell culture plates were taken out, the supernatant was aspirated, and 50 µL of Jurkat-human FcγRIIIa(158V)-NFAT cells and 50 µL of the serially diluted anti-CCR8 antibodies were added. After induction at 37°C, 5% CO₂ for 6 hours, the plate was equilibrated for at least 15 minutes at room temperature. 100 µL of the luciferase substrate solution (Vazyme, DD1203) was added to each well, mixed and then imcubated at room temperature in the dark for 5 min. Values of the relative light units (RLUs) were read with a microplate reader. The experimental data were analyzed with the software Graphpad Prism 8.0. The dose-effect curve of the anti-CCR8 antibody was fitted using the chosen four-parameter regression model, with the logarithms of the anti-CCR8 antibody concentrations on the x-axis and the corresponding RLU values on the y-axis.

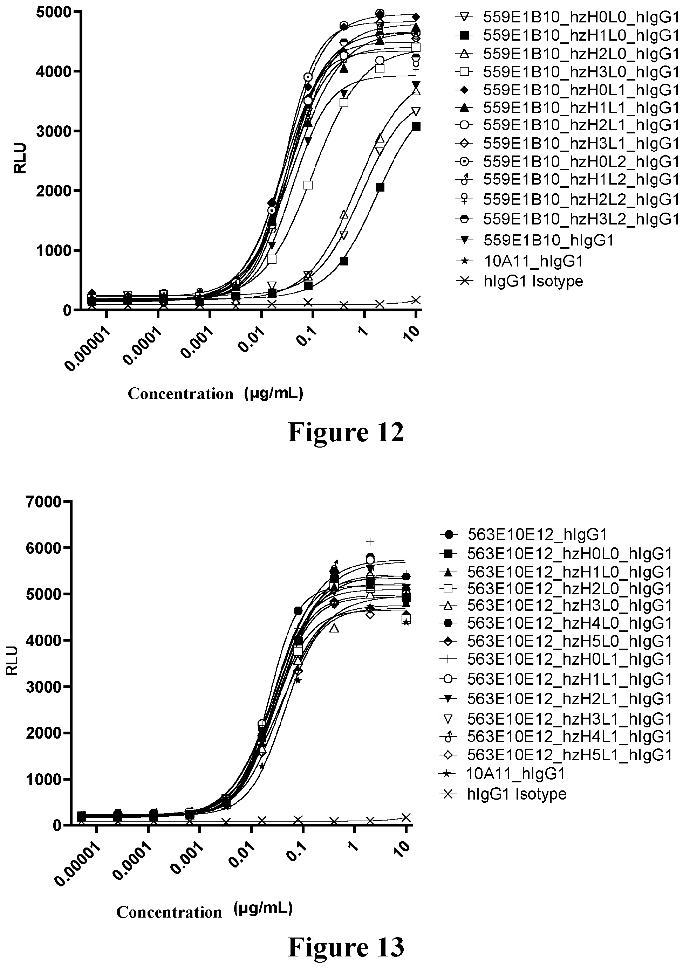

As shown in Figs. 12 and 13, and Tables 18 and 19, chimeric and humanized antibodies of 559E1B10 and 563E10E12 can activate Jurkat-human FcyRIIIa(158V)-NFAT in a dose-dependent manner. The humanized antibodies 559E1B10_hzH0L1, 559E1B10_hzH1L1, 559E1B10_hzH2L1, 559E1B10_hzH3L1, 559E1B10_hzH0L2, 559E1B10_hzH1L2, 559E1B10_hzH2L2 and 559E1B10_hzH3L2 have activities comparable to that of the chimeric parent antibody 559E1B10_hIgG1. All of the humanized antibodies of 563E10E12, such as 563E10E12_hzH0L0, 563E10E12_hzH1L0, 563E10E12_hzH0L1, have activities comparable to that of the chimeric parent antibody thereof.

**Table 18. EC₅₀ Values of Activation of Jurkat-Human FcyRIIIa(158V)-NFAT by the 559E1B10 Humanized Antibodies**

| Antibody name | EC₅₀, ng/mL |
|---|---|
| 10A11_hIgG1 | 38.9 |
| 559E1B10_hIgG1 | 40.1 |
| 559E1B10_hzH0L0_hIgG1 | 854.5 |
| 559E1B10_hzH1L0_hIgG1 | 1691.0 |
| 559E1B10_hzH2L0_hIgG1 | 705.6 |
| 559E1B10_hzH3L0_hIgG1 | 98.5 |
| 559E1B10_hzH0L1_hIgG1 | 30.5 |
| 559E1B10_hzH1L1_hIgG1 | 42.1 |
| 559E1B10_hzH2L1_hIgG1 | 29.2 |
| 559E1B10_hzH3L1_hIgG1 | 28.0 |
| 559E1B10_hzH0L2_hIgG1 | 28.6 |
| 559E1B10_hzH1L2_hIgG1 | 32.5 |
| 559E1B10_hzH2L2_hIgG1 | 33.0 |
| 559E1B10_hzH3L2_hIgG1 | 34.4 |

**Table 19. EC₅₀ Values of Activation of Jurkat-Human FcyRIIIa(158V)-NFAT by the 563E10E12- Humanized Antibodies**

| Antibody name | EC₅₀, ng/mL |
|---|---|
| 10A11_hIgG1 | 43.3 |
| 563E10E12_hIgG1 | 21.6 |
| 563E10E12_hzH0L0_hIgG1 | 28.6 |
| 563E10E12_hzH1L0_hIgG1 | 27.9 |
| 563E10E12_hzH2L0_hIgG1 | 28.1 |
| 563E10E12_hzH3L0_hIgG1 | 35.7 |
| 563E10E12_hzH4L0_hIgG1 | 38.8 |
| 563E10E12_hzH5L0_hIgG1 | 35.5 |
| 563E10E12_hzH0L1_hIgG1 | 29.9 |
| 563E10E12_hzH1L1_hIgG1 | 28.3 |
| 563E10E12_hzH2L1_hIgG1 | 30.5 |
| 563E10E12_hzH3L1_hIgG1 | 26.1 |
| 563E10E12_hzH4L1_hIgG1 | 25.9 |
| 563E10E12_hzH5L1_hIgG1 | 26.4 |

### Example 6. Binding Activity of Anti-CCR8 Antibodies to CCR8 endogenous expressing HuT78 Cell.

Both the human T lymphoma cell line HuT78 and the activated human Treg cells express CCR8 (James M Fox et al. and Yiftah Barsheshet et al., CCR8+ FOXp3+ T reg cells as master drivers of immune regulation. Proc Natl Acad Sci U S A. 2017 Jun 6;114(23):6086-6091. doi: 10.1073/pnas.1621280114). The binding activity of anti-CCR8 antibodies to the endogenously expressed CCR8 was detected by FACS. HuT78 cells (Cobioer, CBP60267) were adjusted to 1×10⁶ cells/mL, placed into 96-well U-bottom plates at 100 µL/well, centrifuged and the supernatant was discarded. The anti-CCR8 antibodies, the control antibodies 433H, 10A11_hIgG1, and B16_hIgG1:7-B16.001 (sequences in Patent US20210277129A1, with the sequence number of the light-chain variable region: 81 and the sequence number of the heavy-chain variable region: 80) and the hIgG1 isotype (Niointron Biotechnology Co., Ltd., B117901) were serially diluted 4-fold with PBS solution containing 2% FBS (FACS buffer), with the initial concentration being 10 µg/mL. Cells were resuspended with the serially diluted antibodies at 100 µL/well, pipetted to mix well, incubated at 4°C for 1 hour, and after then, washed with the FACS buffer three times. Cells were resuspended with the secondary antibody AF647-Goat anti-Human IgG (H+L) (Invitrogen, A21445) or AF647-Donkey anti-Mouse IgG (H+L) (Invitrogen, Cat. No. A31571; detecting the murine-derived antibody 433H) 1:1000 diluted with the FACS buffer at 100 µL/well, pipetted to mix well, and incubated at 4°C for about 45 minutes. After incubation, the cells were centrifuged and washed with FACS buffer three times. Cells were re-suspended with 100 µL/cell FACS buffer. The median fluorescence intensity (MFI) was read with a flow cytometer and the experimental data were analyzed with the software Graphpad Prism 8.0. EC₅₀ was calculated by fitting the dose-effect curve of the anti-CCR8 antibody wtih the chosen four-parameter regression model, with the logarithms of the anti-CCR8 antibody concentrations on the x-axis and the corresponding MFI values on the y-axis.

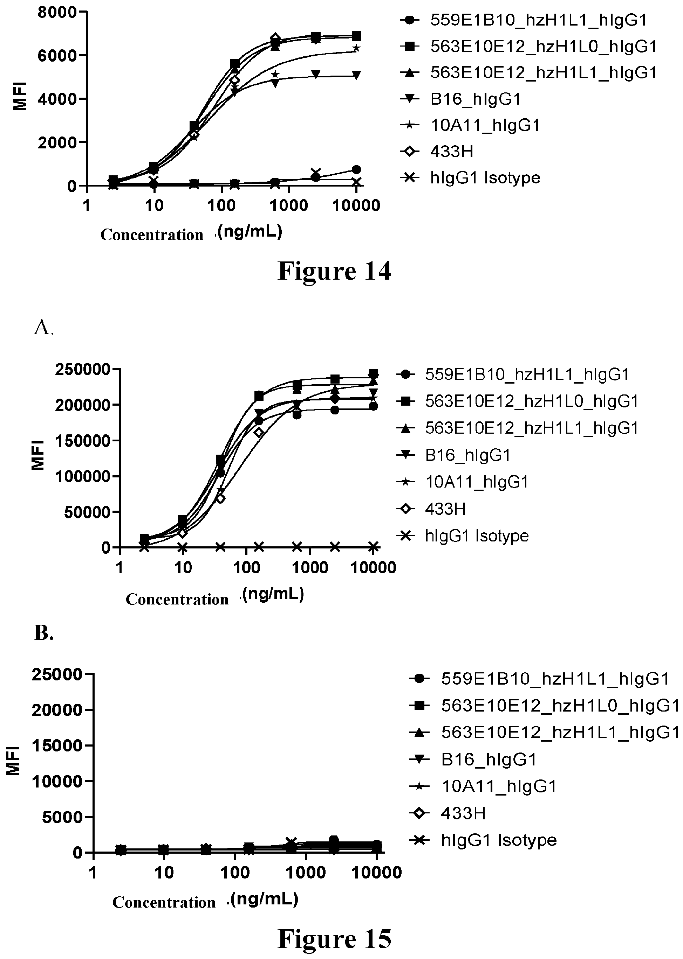

As shown Fig. 14 and Table 20, the humanized antibodies 563E10E12_hzH1L0 and 563E10E12_hzH1L1 bound to HuT78 cells endogenously expressing CCR8 in a dose-dependent manner, with their EC₅₀ of binding activity comparable to those of 433H, B16_hIgG1 and 10A11_hIgG1 and their MFIs of the maximum binding higher than those of B16_hIgG1 and 10A11_hIgG1. However, 559E1B10_hzH1L1 have almost no binding activity to HuT78.

**Table 20. EC₅₀ of the Binding Activity of the Anti-CCR8 Antibodies to the HuT78**

| Antibody | EC₅₀, ng/mL |
|---|---|
| 559E1B10_hzH1L1_hIgG1 | 15235 |
| 563E10E12_hzH1L0_hIgG1 | 54.03 |
| 563E10E12_hzH1L1_hIgG1 | 54.47 |
| B16_hIgG1 | 36.41 |
| 10A11_hIgG1 | 63.65 |
| 433H | 76.32 |
| hIgG1 isotype | N/A |

| | |
|---|---|
| N/A: No activity or no valid EC₅₀ value obtained. | |

### Example 7. Specific Binding of Anti-CCR8 Antibodies to Human CCR8

### 7.1 Specific binding of anti-CCR8 antibodies to CHO-K1 cells expressing human CCR8

CCR8 over-expressing CHO-K1 cells were used during mouse immunization and screening for the CCR8 antibody. Binding of anti-CCR8 antibody to the CCR8 on cell surface was detected using the CHO-K1 cell line overexpressing human CCR8 (established by Shanghai ChemPartner Co., Ltd, CHOK1-hCCR8), the wild-type CHO-K1 cell line (GenomeditechBiotechnology, GM-15570), and the approach from the assay **Example 2-2.1.**

As shown in Fig. 15 and Table 21, 559E1B10_hzH1L1, 563E10E12_hzH1L0 and 563E10E12_hzH1L1 all bound dose-dependently to CHOK1-hCCR8, and not to the CHOK1 blank cells.

**Table 21. EC₅₀ of specific binding of anti-CCR8 antibodies to hCCR8 on cell surfaces**

| | EC₅₀, ng/mL | |
|---|---|---|
| Antibody name | CHO-hCCR8 | CHO-K1 |
| 559E1B10_hzH1L1_hIgG1 | 36.01 | N/A |
| 563E10E12_hzH1L0_hIgG1 | 37.60 | N/A |
| 563E10E12_hzH1L1_hIgG1 | 42.28 | N/A |
| B16_hIgG1 | 35.64 | N/A |
| 10A11_hIgG1 | 52.52 | N/A |
| 433H | 78.75 | N/A |
| hIgG1isotype | N/A | N/A |

| | | |
|---|---|---|
| N/A: No activity or no valid EC₅₀ value obtained. | | |

### 7.2 CCR8-dpendent activation of Jurkat-human FcγRIIIa(158V)-NFAT, mediated by the anti-CCR8 antibody

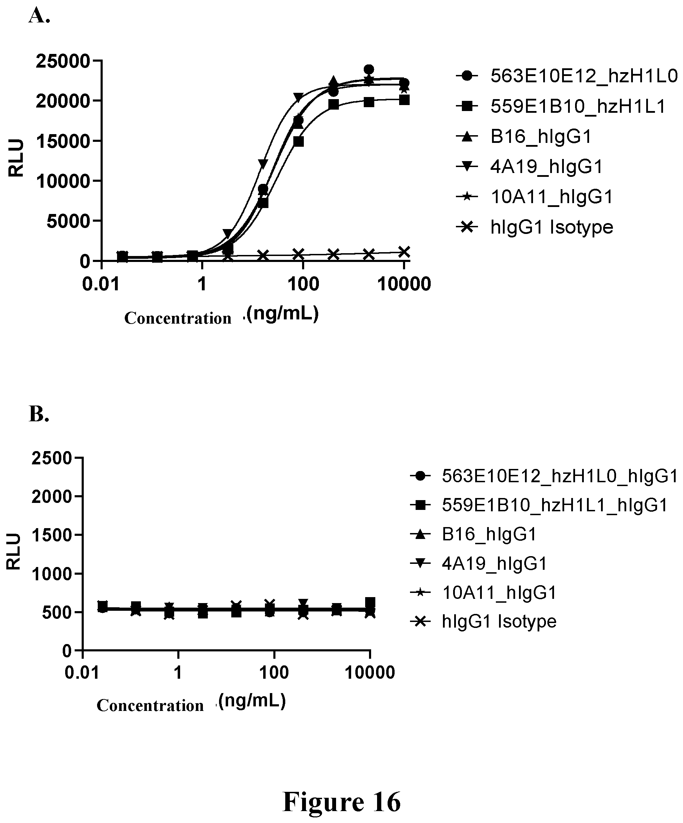

CHO-K1 cell line overexpressing human CCR8 (established by Shanghai Ch emPartner Co., Ltd, CHOK1-hCCR8) and the CHO-K1 blank cell line (Genomed itechBiotechnology, GM-15570) were used to determine that activation of Jurkat-human FcyRIIIa(158V)-NFAT with anti-CCR8 antibody is relies upon CCR8. Th e concentrations of CHOK1-hCCR8 cells and CHOK1 blank cells were adjusted to 3×10⁵ cells/mL with the RPMI-1640 complete culture medium. The procedure in Example 5-5.4 was used to detect activation of Jurkat-human FcyRIIIa(158V)-NFAT with anti-CCR8 antibody. 4A19 is a sequence in Patent WO2021194942A 1, with the numbers of the light- and heavy-chain variable region sequences of 18 and 6, respectively. As shown in Fig. 16 and Table 22, the anti-CCR8 antib ody dose-dependently activated Jurkat-human FcγRIIIa(158V)-NFAT reporter activ ity in presence of CHOK1-hCCR8 cells, and was unable to activate Jurkat-huma n FcyRIIIa(158V)-NFAT in presence of CHO-K1 blank cells, indicating that the anti-CCR8 antibody can specifically recognize CCR8 and mediate the CCR8-dep endent ADCC effect.

**Table 22. EC₅₀ of CCR8-Dependent Activation of Jurkat-human FcγRIIIa(158V)-NFAT, Mediated with the Anti-CCR8 Antibody**

| | EC₅₀, ng/mL | |
|---|---|---|
| Antibody name | CHOK1-hCCR8 | CHOK1-Blank |
| 559E1B10_hzH1L1 | 26.51 | N/A |
| 563E10E12_hzH1L0 | 29.89 | N/A |
| B16_hIgG1 | 26.48 | N/A |
| 4A19_hIgG1 | 14.09 | N/A |
| 10A11_hIgG1 | 24.32 | N/A |
| hIgG1isotype | N/A | N/A |

| | | |
|---|---|---|
| N/A: No activity or no valid EC₅₀ value obtained. | | |

### 7.3 No binding of ati-CCR8 antibodies to human CCR4

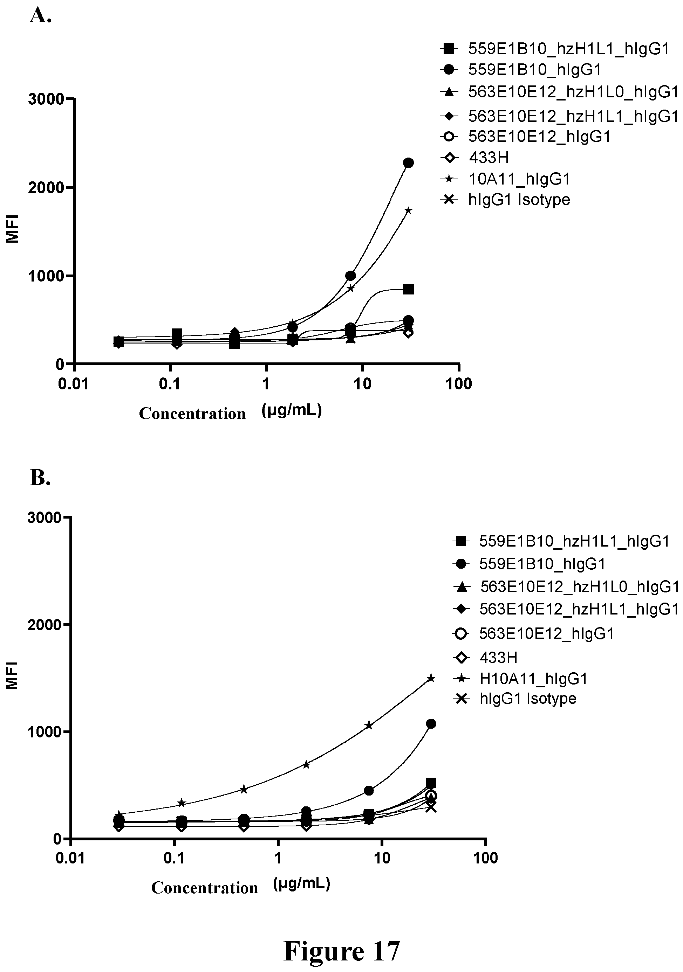

CCR4 is the gene with highest homology to CCR8 in human (Antonio Recchiuti et al., Pro-Resolving Lipid Mediators (SPMs) and Their Actions in Regulating miRNA in Novel Resolution Circuits in Inflammation. Front Immunol. 2012 Oct 22;3:298. doi: 10.3389/fimmu.2012.00298. eCollection 2012.), but it has a homology of 46% to CCR8 only (the result from NCBI blast). The binding activity of the anti-CCR8 antibody to the human CCR4 was detected using the procedure below. 293F cells were transiently transfected with hCCR4-GFP (Sino, HG13064-ACG) according to the steps indicated in ExpiFectamine^{™} 293 Transfection kit (Gibco, A14524). The transfected cells were adjusted to 1×10⁶ cells/mL, placed into 96-well U-bottom plates at 100 µL/well and centrifuged to obtain cells. The anti-CCR8 antibodies were diluted to the initial working concentrations with PBS solution containing 2% FBS (FACS buffer), with the initial concentration of 30 µg/mL, and then serially diluted 4-fold with the FACS buffer. Cells were resuspended at 100 µL/well, pipetted to mix well, incubated at 4°C for 1 hour, and then washed with the FACS buffer three times. Cells were resuspended with the secondary antibody AF647-Goat anti-Human IgG (H+L) (Invitrogen, A21445) 1:1000 diluted with the FACS buffer at 100 µL/well, pipetted to mix well, and incubated at 4°C for about 40 minutes. After incubation, the cells were centrifuged and washed with FACS buffer three times. After dead cells were labeled according to instructions in LIVE/DEAD^{™} Fixable Near IR (780) Viability kit (Invitrogen, L34992), the cells were resuspended with 100 µL/cell of the FACS buffer and the median fluorescence intensities (MFIs) was read with a flow cytometer, respectively. CCR4 expression in cells transfected with hCCR4-GFP was determined with an anti-CCR4 antibody (Biolegend, 359412). The GFP-positive cells transfected with hCCR4-GFP (CCR4-expressing cells) and the live cells transfected with blank plasmids were analyzed for the binding to anti-CCR8 antibodies. As shown in Fig. 17, the 563E10E12- chimeric and humanized antibodies do not bind to cells transfected with hCCR4-GFP (GFP-positive cells) or to 293F cells, while the 559E1B10- chimeric and humanized antibodies showed the weaker binding to hCCR4 at high concentrations.

### 7.4 Binding of anti-CCR8 antibodies to immune cells in PBMCs

CCR8 is low expression on Treg cells and not expressed on other immune cells in the peripheral blood of healthy donors (George Plitas et al., Regulatory T Cells Exhibit Distinct Features in Human Breast Cancer. Immunity. 2016 Nov 15;45(5):1122-1134. doi: 10.1016/j.immuni.2016.10.032). PBMCs of the healthy donors were used to check if there was nonspecific binding of anti-CCR8 antibodies to peripheral immune cells. The cryopreserved PBMCs from healthy donors (Allcells, Cat. No. Z0060 and Y1446) were revived and adjusted to 2×10⁶ cells/mL, placed into 96-well U-bottom plates at 100 µL/well,centrifuged and supernatant was discarded. Dead cells were labeled according to instructions in LIVE/DEAD^{™} Fixable Near IR (780) Viability kit (Invitrogen, L34992). The FcX reagent (Biolegend, 422302) diluted 1:25 with the FACS buffer was added at 50 µL/well to block the staining of Fc receptors by antibodies, and incubated at room temperature for 15 minutes. The mixture of PE anti-human CD3 Antibody (Biolegend-317308, diluted 1:50), FITC-anti-human CD8 (Biolegend-301006, diluted 1:50), Pacific Blue^{™} anti-human CD19 (Biolegend-363036, diluted 1:50) and Brilliant Violet 605^{™} anti-human CD14 (Biolegend-367126, diluted 1:50) prepared with the FACS buffer was added the cells at 40 µL/well and mixed well. The anti-CCR8 antibodies and hIgG1 isotype (Biointron Biotechnology Co., Ltd., B117901) labeled with AF647 labeling kit (Invitrogen, A20186) were diluted with the FACS buffer to the initial concentration of 100 µg/mL, serially diluted 5-fold with the FACS buffer solution, added to the above cells at 10 µL/well, and incubated at 4°C for 1 hour. After the incubation, the cells were washed with the FACS buffer three times and resuspended with the FACS buffer at 100 µL/well. The median fluorescence intensity (MFI) was read with a flow cytometry.

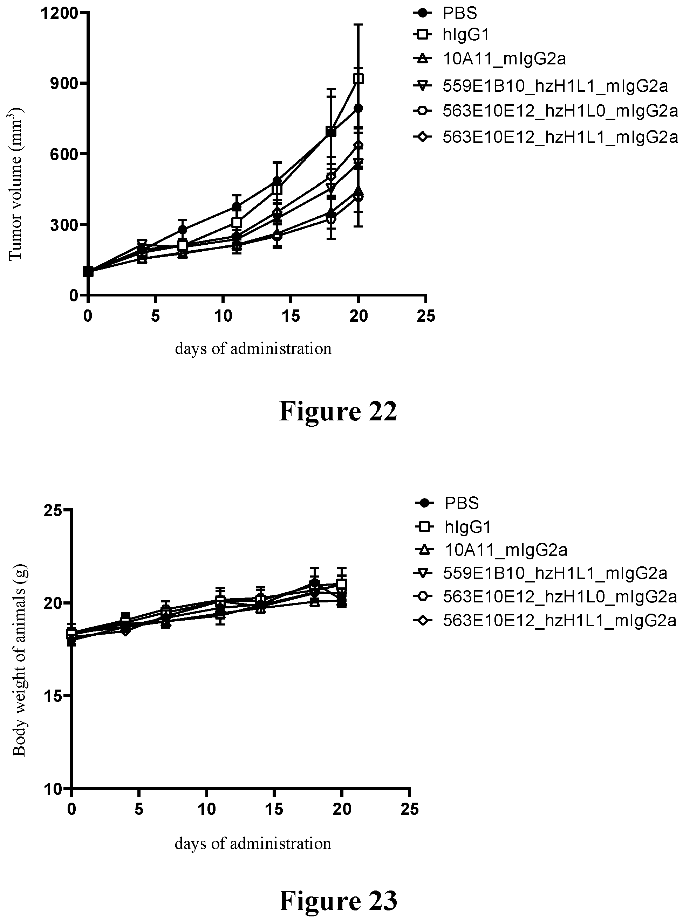

As shown in Fig. 23, the anti-CCR8 antibodies 559E1B10_hzH1L1_hIgG1, 563E10E12_hzH1L0_hIgG1, and 563E10E12_hzH1L1_hIgG1 did not significantly bind to CD8⁺ and CD4⁺ (CD3⁺ CD8⁻) T cells, CD14⁺ monocytes, CD19⁺ B cells and others CD3⁻CD14⁻CD19⁻ immune cells.

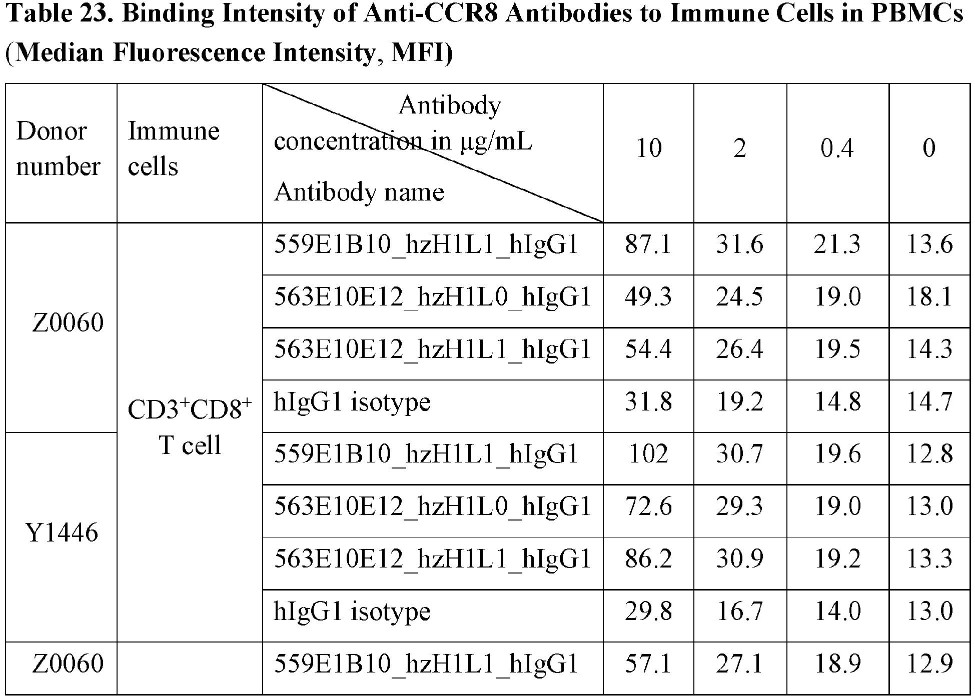

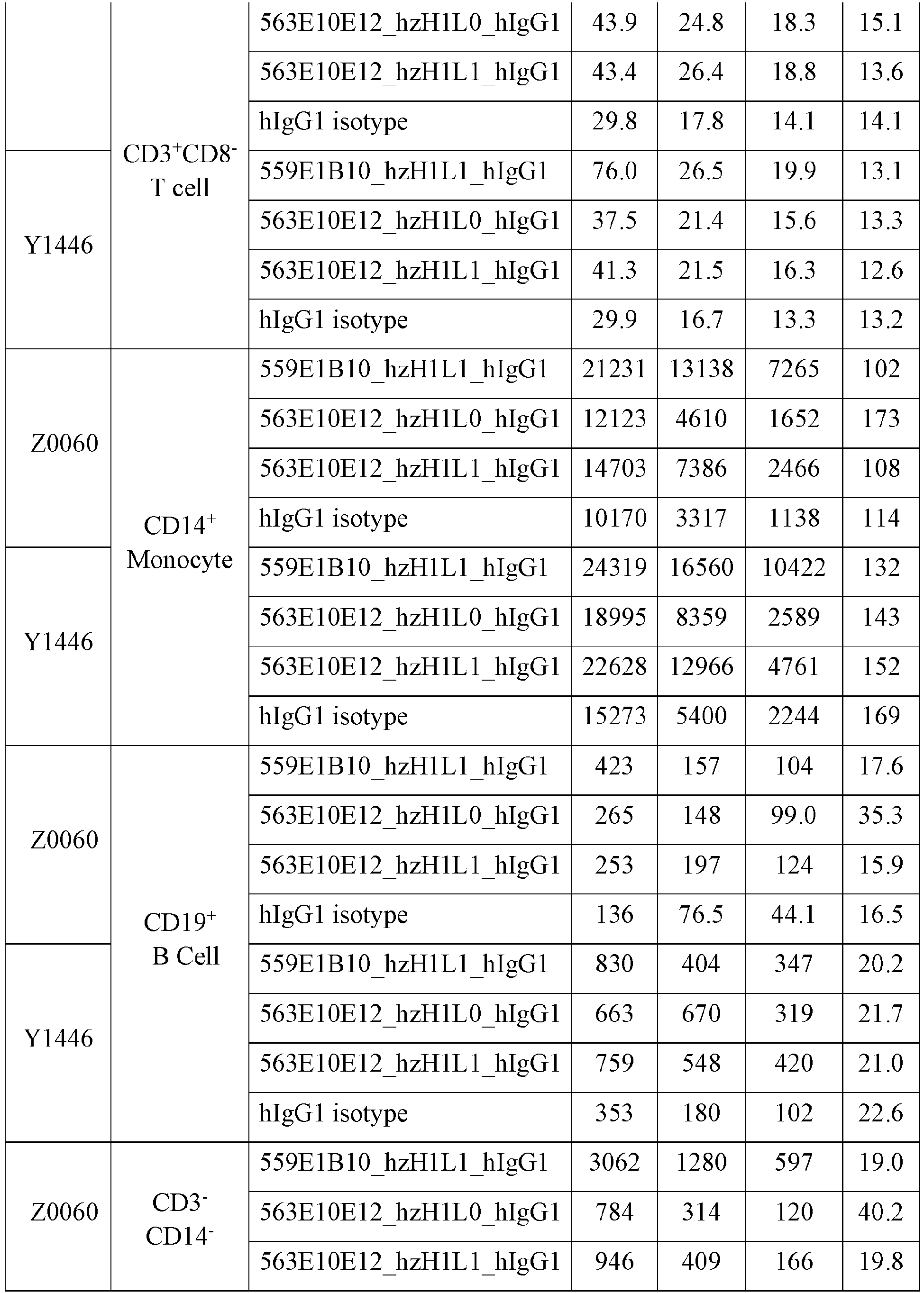

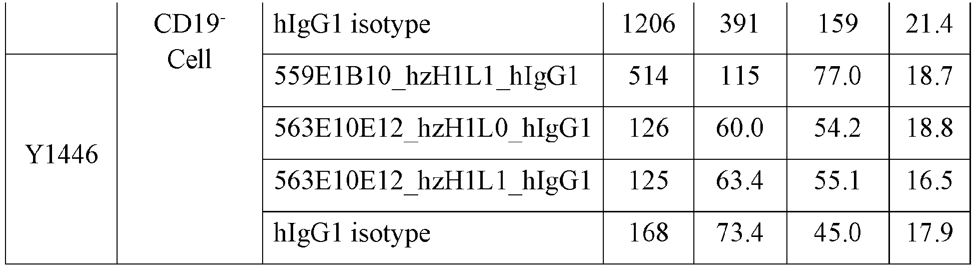

### Example 8. Fc Mutations and afucosylation Enhance Anti-CCR8 Antibody-Mediated ADCC Effect

### 8.1 Mutations S239D/I332E, S239D/A330L/I332E, L235V/F243L/R292P/Y300L/P396L and afucosylation enhance activation of Jurkat-human FcyRIIIa(158V)-NFAT by anti-CCR8 antibody and 293F-humanCCR8

Mutations S239D/I332E, S239D/A330L/I332E or L235V/F243L/R292P/Y300L/P396L in the antibody constant regions (Fc) can enhance affinity for FcyRIIIa, increase the antibody-mediated ADCC effect (Greg A Lazar et al., Engineered antibody Fc variants with enhanced effector function. Proc Natl Acad Sci U S A. 2006 Mar 14;103(11):4005-10. doi: 10.1073/pnas.0508123103. and Jeffrey L Nordstrom et al., Anti-tumor activity and toxicokinetics analysis of MGAH22, an anti-HER2 monoclonal antibody with enhanced Fcy receptor binding properties. Breast Cancer Res. 2011;13(6):R123. doi: 10.1186/bcr3069). These sets of mutations have been used clinically to enhance antibody's ADCC effect (Rena Liu et al., Fc-Engineering for Modulated Effector Functions-Improving Antibodies for Cancer Treatment. Antibodies (Basel). 2020 Nov 17;9(4):64. doi: 10.3390/antib9040064).

The Fc regions of the anti-CCR8 humanized antibodies were subject to the mutations S239D/I332E (DE), S239D/A330L/I332E(DLE) or L235V/F243L/R292P/Y300L/P396L(VLPLL) to enhance the ADCC effect by the antibodies. Afucosylation or reduction in fucosylation can increase the ADCC effect of an antibody. Several afucosylated or less-fucosylated antibodies have been approved for marketing or in the late stage of clinical researches (Natasha A Pereira et al., The "less-is-more" in therapeutic antibodies: Afucosylated antibodies with enhanced antibody-dependent cellular cytotoxicity. MAbs. 2018 Jul;10(5):693-711. doi: 10.1080/19420862.2018.1466767). To inhibit fucosylation, ExpiCHO cells were passaged, expanded and cultured in the growth medium ExpiCHO Expression Medium (Gibco, Cat. No. A2910001) containing 100 µM of 2F-peracetyl-fucose (MILLIPORE, Cat. No. 344827-10MGCN) before transient transfection. On the day of transient transfection, the cells were diluted to 6×10⁶ cells/mL and transient transfection was performed at the concentration of 1 µg plasmid/mL of the cell suspension using Expi CHO Transfection kit (Gibco, Cat. No. A29129). The transiently transfected cells were fed with supplemental nutrients (Gibco, Cat. No. A29129) 18-22 hours after transient transfection, and subsequently cultured in a shaking incubator at 100 rpm at 37°C, 8% CO₂. Cell culture fluid was harvested 7 days after the transient transfection, centrifuged, filtered through 0.45 µm filters to obtain the supernatant. The resultant supernatant was purified with Protein A resin to harvest the antibody.

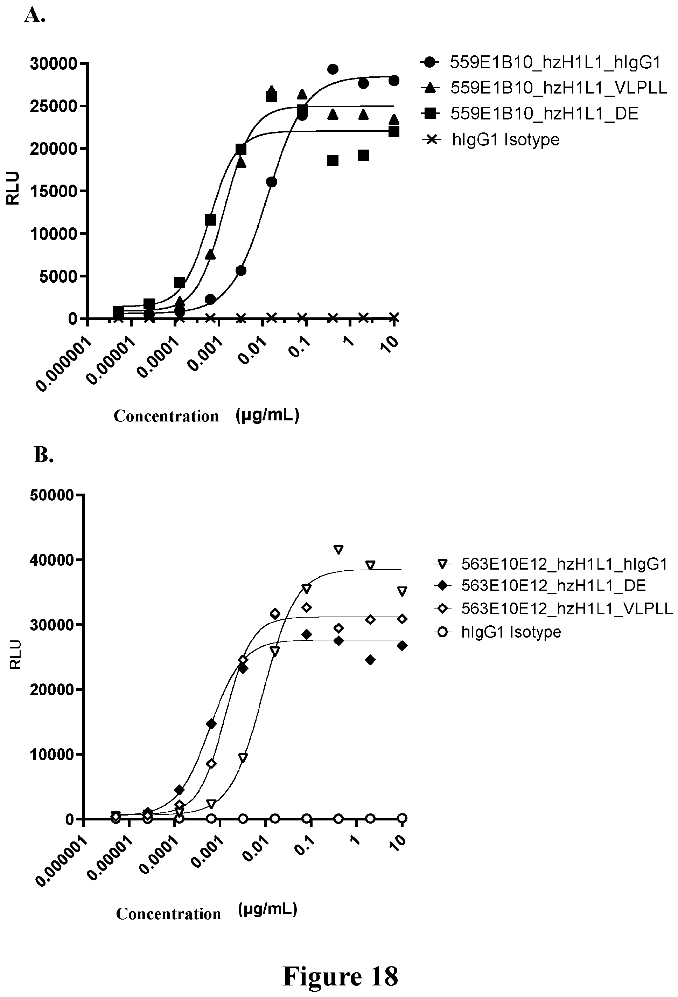

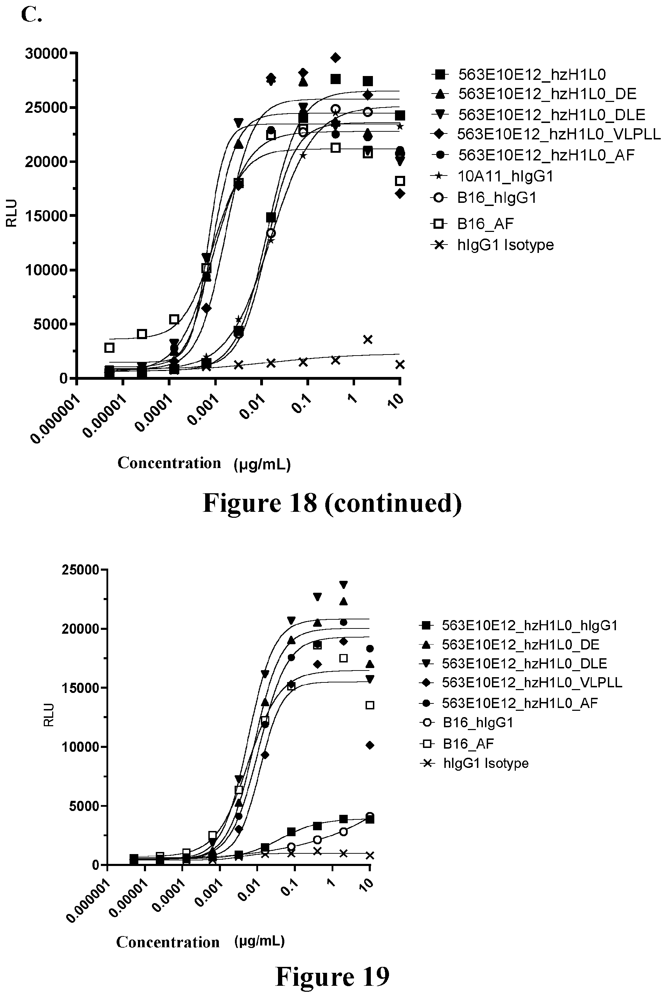

The procedure in **Example 5-5.4** was used to determine the activity of Jurkat-human FcyRIIIa(158V)-NFAT by incubating anti-CCR8 antibodies with 293F-hCCR8 cells. As shown in Fig. 18 and Table 24, compared to 559E1B10_hzH1L1_hIgG1 antibody, the Fc-mutated antibodies 559E1B10_hzH1L1_DE and 559E1B10_hzH1L1_ VLPLL enhanced activation of Jurkat-human FcγRIIIa(158V)-NFAT, with EC₅₀ improved by 9-21 times. Compared to 563E10E12_hzH1L0_hIgG1 antibody, the Fc-mutated antibodies 563E10E12_hzH1L0_DE, 563E10E12_hzH1L0_DLE and 563E10E12_hzH1L0_VLPLL, and the aucosylated antibody 563E10E12_hzH1L0_AF enhance the activity of Jurkat-human FcγRIIIa(158V)-NFAT, with EC₅₀ improved by 7-18 times. Compared to 563E10E12_hzH1L1_hIgG1 antibody, the Fc-mutated antibodies 563E10E12_hzH1L1 _DE and 563E10E12_hzH1L1_VLPLL enhanced activation of Jurkat-human FcγRIIIa(158V)-NFAT, with EC₅₀ improved by 6-14 times. The Fc-mutated humanized antibodies from 559E1B10 and 563E10E12 or the afucosylated antibodies 563E10E12_hzH1L0 show activities comparable to that of the afucosylated B16 (B16_AF).

**Table 24. Activation of Jurkat-Human FcyRIIIa(158V)-NFAT by Incubation of the 293F-HumanCCR8 with the Anti-CCR8 Antibodies With Fc Mutation or afucosylation (EC₅₀, µg/mL)**

| Antibody name | EC₅₀, µg/mL |
|---|---|
| 559E1B10_hzH1L1_hIgG1 | 0.0133 |
| 559E1B10_hzH1L1_DE | 0.0006 |
| 559E1B10_hzH1L1_VLPLL | 0.0014 |
| 563E10E12_hzH1L1_hIgG1 | 0.0089 |
| 563E10E12_hzH1L1_VLPLL | 0.0013 |
| 563E10E12_hzH1L1_DE | 0.0006 |
| 563E10E12_hzH1L0_hIgG1 | 0.0135 |
| 563E10E12_hzH1L0_DE | 0.0009 |
| 563E10E12_hzH1L0_DLE | 0.0007 |
| 563E10E12_hzH1L0_VLPLL | 0.0017 |
| 563E10E12_hzH1L0_AF | 0.0009 |
| 10A11_hIgG1 | 0.0160 |
| B16_hIgG1 | 0.0131 |
| B16_AF | 0.0009 |
| hIgG1 | N/A |

| | |
|---|---|
| N/A: No activity or no valid EC₅₀ value. | |

### 8.2. Fc mutations and afucosylation enhance activity of Jurkat-Human FcyRIIIa(158V)-NFAT by anti-CCR8 antibody and HuT78 cells

The anti-CCR8 antibodies with different Fc mutations or afucosylation were incubated with HuT78 cells and Jurkat-human FcγRIIIa(158V)-NFAT cells, to detect the effect of mutations S239D/I332E, S239D/A330L/I332E, L235V/F243L/R292P/Y300L/P396L or reduction in fucosylation on activity of Jurkat-human FcyRIIIa(158V)-NFAT by anti-CCR8 antibody and HuT78 cells. HuT78 cells as the target cell was adjusted to 3×10⁵ cells/mL with the DMEM culture medium. Activation of Jurkat-human FcγRIIIa(158V)-NFAT by anti-CCR8 antibodies was determined using the procedure in Example 5-5.4.

As shown in Fig. 19 and Table 25, incubation of the Fc-mutated antibodies 563E10E12_hzH1L0_DE, 563E10E12_hzH1L0 DLE, 563E10E12_hzH1L0_VLPLL and the afucoslyated antibody 563E10E12_hzH1L0_AF with HuT78 cells can increase the activity of Jurkat-human FcyRIIIa(158V)-NFAT by 563E10E12_hzH1L0 antibody, with EC₅₀ improved by 2-6 times and the maximum signal increased by 4-5 times. Activation of Jurkat-human FcγRIIIa(158V)-NFAT by the Fc-mutated or afucosylation 563E10E12_hzH1L0 antibody is comparable to that with the afucoslyated B16 antibody.

**Table 25. EC₅₀ of Activation of Jurkat-Human FcγRIIIa(158V)-NFAT by Incubation of the Hut78 Cells with 563E10E12_hzH1L0 Antibody With Fc Mutation or afucosylation**

| **Antibody name** | **EC₅₀, µg/mL** |
|---|---|
| 563E10E12_hzH1L0_hIgG1 | 0.0409 |
| 563E10E12_hzH1L0_DE | 0.0083 |
| 563E10E12_hzH1L0_DLE | 0.0057 |
| 563E10E12_hzH1L0_VLPLL | 0.0117 |
| 563E10E12_hzH1L0_AF | 0.0110 |
| B16_hIgG1 | ~ 2.995e+014 |
| B16_AF | 0.00565 |
| hIgG1 isotype | N/A |

| | |
|---|---|
| N/A: No activity or no valid EC₅₀ value obtained. | |

### Example 9. ADCC Effect Induced by Healthy Human PBMCs and Anti-CCR8 Antibodies

PBMCs as the effector cell and CHOK1-hCCR8 as the target cell were used to determine the ADCC effect by anti-CCR8 antibodies. The cryopreserved PBMCs from healthy donors (Allcells) were revived and adjusted to 2×10⁶ cells/mL with RPMI-1640 complete culture media containing 200 IU/mL IL-2 (Peprotech, Cat. No. 200-02), placed into an incubator at 37 °C, 5% CO₂ and incubated overnight. On the next day, the unattached PBMC were pipetted, centrifuged at 300 g for 5 minutes, followed by discarding the supernatant, and 1640 medium was added to to about 3×10⁶ cells/mL. The anti-CCR8 antibodies and the hIgG1 isotype (Biointron Biotechnology Co., Ltd., Cat. No. B117901) were diluted with RPMI-1640 medium to the 2-fold working concentrations, with the initial concentration being 0.8 µg/mL, and serially diluted 5-fold. The collected CHOK1-hCCR8 cells were centrifuged at 300 g for 5 minutes, supernatant was discarded, adjusted with DPBS to 2×10⁶ cells/mL. CellTrace^{™} Violet (Invitrogen, Cat. No.C34557) was added according to the instructions to label CHOK1-hCCR8 cells and the CHO-hCCR8 cells labeled with CellTrace^{™} Violet were adjusted with RPMI-1640 medium to aboutl.5×10⁵ cells/mL. 50 µL of CHOK1-hCCR8 labeled with CellTrace^{™} Violet, 50 µL of PBMC, and 100 µL of the diluted anti-CCR8 antibodies were added to U-bottom 96-well plates, mixed and then placed into an incubator at 37 °C, 5% CO₂ and incubated for about 5 hours. 2 µL of the propidium iodide (PI) solution (Invitrogen, Cat. No. 006990-50) was added to each well, mixed and then reacted at room temperature in the dark for 5 min, labeling dead cells. The percentage of PI positive cells in the CellTrace^{™} Violet positive cells was read using a flow cytometer. The antibody-induced ADCC effect was calculated on basis of lysis% = the percentage of PI positive cells labeled with CellTrace^{™} Violet. The experimental data were analyzed with the software Graphpad Prism 8.0. The dose-effect curve of the anti-CCR8 antibody is fitted using the chosen four-parameter regression model, with the logarithms of the anti-CCR8 antibody concentrations on the x-axis and the corresponding lysis% values on the y-axis.

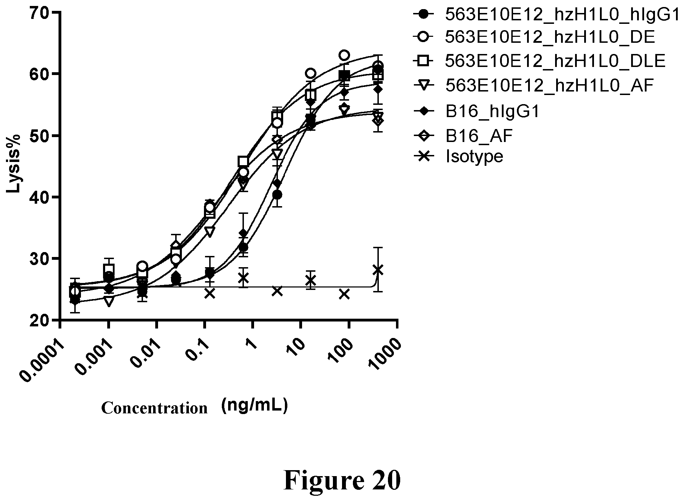

As shown in Fig. 20 and Table 26, compared to 563E10E12_hzH1L0_hIgG1, both the afucosylated humanized antibody 563E10E12_hzH1L0_AF and the Fc-mutated antibodies 563E10E12_hzH1L0_DE and 563E10E12_hzH1L0_DLE increased the ADCC effect induced by the aforementioned antibodies and PBMCs. The humanized antibody 563E10E12_hzH1L0_hIgG has an ADCC activity comparable to that induced by B16_hIgG1. ADCC effect induced by the afucosylated and Fc-mutated humanized antibody 563E10E12_hzH1L0 in combination with PBMCs is similar to the activity induced by the afucosylated B16 antibody.

**Table 26 EC₅₀ of ADCC Effect Induced by Human PBMCs and Anti-CCR8 Antibodies**

| Antibody name | EC₅₀, ng/mL |
|---|---|
| 563E10E12_hzH1L0_hIgG1 | 4.71 |
| 563E10E12_hzH1L0_DE | 0.60 |
| 563E10E12_hzH1L0_DLE | 0.39 |
| 563E10E12_hzH1L0_AF | 0.31 |
| B16_hIgG1 | 2.38 |
| B16_AF | 0.17 |
| hIgG1 isotype | N/A |

| | |
|---|---|
| N/A: Not applicable | |

### Example 10. Specific Elimination of CCR8-Positive Treg Cells in Peripheral PBMCs by Anti-CCR8 Antibodies

The specific depletion of the CCR8-expressing Treg cells in PBMCs by anti-CCR8 antibodies were determined using healthy human PBMCs. As the proportion of the CCR8-expressing Treg cells in healthy human PBMCs is relatively low, IL-2 was used to promote proliferation and activation of Treg cells in this experiment. The cryopreserved PBMCs from healthy donors (Allcells) were revived and adjusted to a cell concentration of 2×10⁶ cells/mL with 1640 complete media containing 200 IU/mL IL-2 (Peprotech, Cat. No. 200-02), added at 100 µL/well to 96-well U-bottom plates, and incubated at 37°C, 5% CO₂ for 48 hours. The anti-CCR8 antibodies and the hIgG1 isotype (Biointron Biotechnology Co., Ltd., Cat. No. B117901) were diluted with RPMI-1640 complete medium, with the initial concentration being 20 µg/mL, and serially diluted 4-fold. The diluted antibody at 100 µL/well was added to the abovementioned 96-well plate, the resultant mixture was pipetted to mix well and incubated at 37°C, 5% CO₂ for 96 hours. Cells were harvested by centrifugation. Dead cells were labeled according to instructions in LIVE/DEAD^{™} Fixable Near IR (780) Viability kit (Invitrogen, L34992). The FcX reagent diluted 1:25 (Biolegend, Cat. No. 422302) was added at 50 µL/well and incubated at room temperature for 15 minutes. The staining mixture of AF700 Anti-Human CD3 (Biolegend, Cat. No. 317340) and Percp cy5.5 Anti-Human CD4 (Biolegend, Cat. No. 300530) diluted 1:50 was added at 50 µL/well and incubated at 4°C for 1 hours, followed by washing with the FACS buffer three times. According to the instructions in Foxp3/Transcription Factor Staining Kit (Invitrogen, Cat. No. 00-5523-00), cells were fixed, labeled with PE anti-human Foxp3 (Biolegend, Cat. No. 320208), and washed with the FACS buffer three times. Cells were re-suspended with 100 µL/cell of the FACS buffer, the relevant information was read with a flow cytometer, and the experimental data were analyzed with the software Graphpad Prism 8.0. At the end of the experiment, the expression of CCR8 on Treg cells (Foxp3⁺CD4⁺) was detected and the proportion of Treg cells in the CCR8 positive cells was 31.5%.

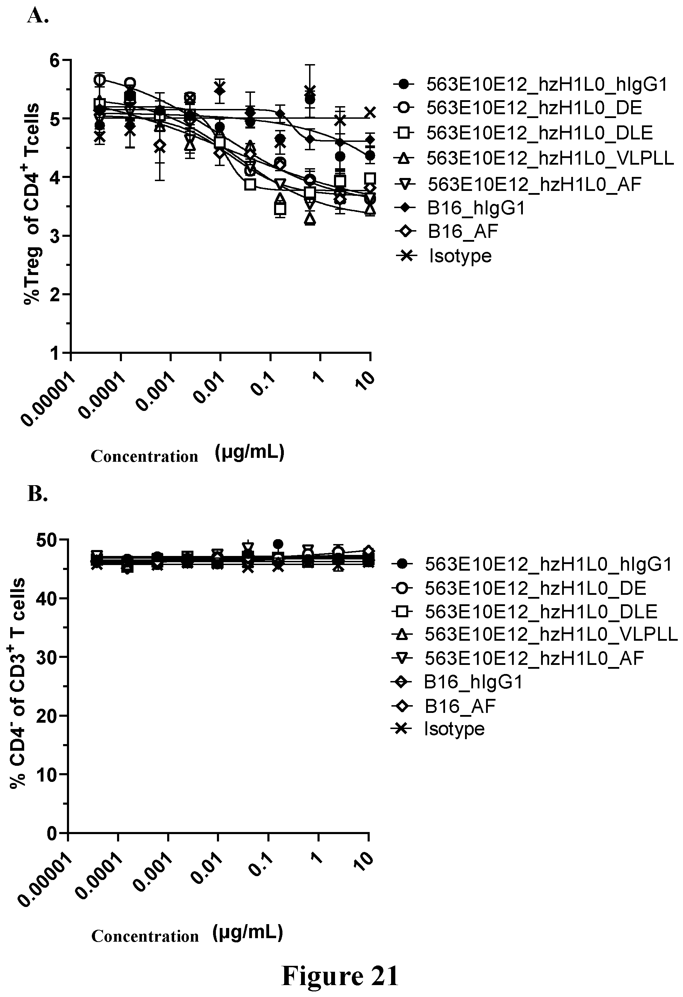

As shown in Fig 21, the afucosylated 563E10E12_hzH1L0_AF antibody and the Fc-mutated 563E10E12_hzH1L0_DE, 563E10E12_hzH1L0_DLE, 563E10E12_hzH1L0_VLPLL antibodies and the afucosylated B16_AF antibody dose-dependently and partially depleted Treg cells in PBMCs, but had no effect on CD8⁺ T (CD3⁺CD4⁻) not expressing of CCR8. The 563E10E12_hzH1L0_hIgG1 and B16_hIgG1 antibodies depleted a small proportion of Treg cells only at higher concentrations. This indicates that after activation of the peripheral PBMC through IL-2, Treg cells exhibit a certain level of CCR8 expression and can be eliminated by anti-CCR8 antibodies while CD8⁺ T cells lacking CCR8 expression are not eliminated.

### Example 11. Anti-CCR8 Antibodies Inhibit Tumor growth in Tumor-Bearing Mouse Models

### 11.1 Anti-CCR8 antibodies suppressing growth of MC38 colon carcinoma in CCR8-humanized mice

An animal model of MC38 colon carcinoma (Biocytogen (Beijing) Pharmaceuticals Co., Ltd.) were established with the human CCR8-humanized mouse (C57BL/6-Ccr8^{tm1(CCR8)}/Bcgen, B-hCCR8, Biocytogen (Beijing) Pharmaceuticals Co., Ltd., Cat. No. 110096) and used for testing the *in vivo* efficacy of anti-CCR8 antibodies. MC38 cells (5×10⁶ cells/mL) resuspended with PBS were inoculated subcutaneously at 100 µL/animal into the right flank of B-hCCR8 mice. When the average tumor volume reached approximately 100 mm³. Mice were divided into groups with 6-8 animals per group and injected subcutaneously with 10 mg/kg of an anti-CCR8 antibody or a negative control (hIgG1) (Biointron Biotechnology Co., Ltd., Cat. No. B117901) or an equal volume of a vehicle control (PBS), administered twice weekly. After administration, the mice were fed normally, their health status were observed, and their body weight and tumor volume were recorded.

Compared to the vehicle control and the negative control (hIgG1), the anti-CCR8 antibodies 559E1B10_hzH1L1_mIgG2a, 563E10E12_hzH1L0_mIgG2a, 563E10E12_hzH1L1_mIgG2a, and 10A11_mIgG2a suppressed growth of MC38 colon carcinoma cells (Fig. 22). On Day 20 post administration, the anti-CCR8 antibodies 559E1B10_hzH1L1_mIgG2a, 563E10E12_hzH1L0_mIgG2a, 563E10E12_hzH1L1_mIgG2a, and 10A11_mIgG2a exhibit the tumor growth inhibition rates (TGIs) (%) of 40.1%, 54.0%, 22.5%, and 53%, respectively. In the experiment, no adverse event of all animals was observed and the animals were in a well-fed state during the administration, and their body weight increased to a certain extent, indicating that the animals well tolerated to anti-CCR8 antibody treatment (Fig. 23).

### 11.2 Fc-mutated and afucosylation anti-CCR8 Antibodies inhibit growth of MC38 colon carcinoma in CCR8-humanized mice

MC38 colon carcinoma models were established in CCR8-humanized B-hCCR8 mice with the method in Example **11-11.1.** When the average of tumor volume reached approximately 100 mm³, mice were divided into groups with 5 animals per group and injected subcutaneously with 10 mg/kg of an anti-CCR8 antibody or the equal volume of a vehicle control (PBS), administered twice weekly. After administration, the mice were fed normally, their survival status were observed, and their body weight and tumor volume were recorded.

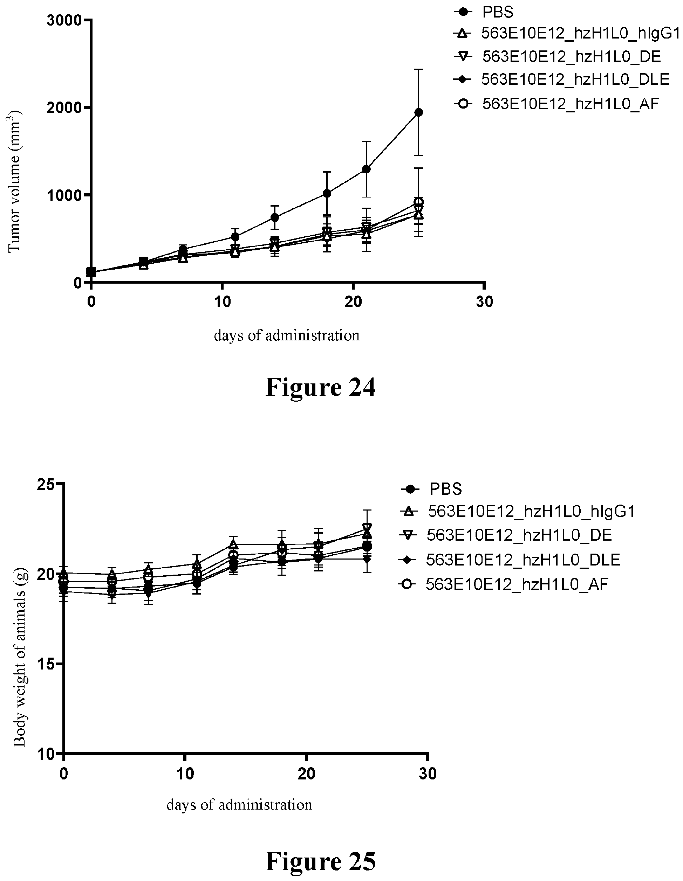

As shown in Fig. 24, compared to the vehicle, the anti-CCR8 antibody 563E10E12_hzHI1L0_hIgG1, the Fc-mutated 563E10E12_hzH1L0_DE and 563E10E12_hzH1L0_DLE antibodies thereof, and the afucosylation 563E10E12_hzH1L0_AF antibody all inhibited growth of MC38 colon carcinoma cells. On Day 25 post administration, the 563E10E12_hzH1L0_hIgG1 antibody, the Fc-mutated 563E10E12_hzH1L0_DE and 563E10E12_hzH1L0_DLE antibodies thereof, and the afucosylation 563E10E12_hzH1L0_AF antibody exhibited the tumor growth inhibition rates (TGIs) (%) of 60.1%, 57.8%, 60.2% and 52.9%, respectively. All mice in the dosing groups showed no behavioral abnormalities or weight loss, indicating that the mice were well tolerated to the anti-CCR8 antibodies at the doses administered (Fig. 25).

### 11.3 Anti-CCR8 antibodies inhibit growth of the human non-small cell lung cancer cells in human CD34⁺ HSC (Hematopoietic Stem Cell)- and PBMC-humanized mice

### 11.3.1 Anti-CCR8 antibodies inhibit growth of the non-small cell lung cancer cell line A549 in human CD34⁺ HSC-humanized mice

Human CD34⁺ HSC-humanized mice were established through immune reconstitution in the irradiated NCG mice engrafted with human hematopoietic cells hCD34 ⁺HSC (GemPharmatech LLC.). Human non-small cell lung cancer A549 cells were inoculated subcutaneously into the right flank the human CD34⁺ HSC-humanized mice at an inoculation volume of 0.2 mL/animal, together with 30% Matrigel. When the average tumor volume was approximately 100 mm³, the mice were randomly divided into groups and administered respectively with an anti-CCR8 antibody (1-20 mg/kg), pembrolizumab (1-20mg/kg), the combination of the anti-CCR8 antibody (1-20 mg/kg) and pembrolizumab (1-20mg/kg), and a negative control (PBS), twice a week for 4 weeks. After administration, the mice were fed normally and the tumor volume was recorded. Tumor samples were harvested at the end of the experiment, minced with an anatomical laboratory scissor. Tumor tissues were then dissociated with a human tumor dissociation kit (Miltenyi Biotech) in combination with the gentleMACS dissociator (Miltenyi Biotec) and the cell suspension obtained was filtered via the 70 µm MACS SmartStrainers to generate a single cell suspension. Immune cell subpopulations (Treg and CD8⁺ T cells) were analyzed with a flow cytometer.

The 563E10E12_hzH1L0_hIgG1 antibody, the Fc-mutated 563E10E12_hzH1L0_VLPLL, 563E10E12_hzH1L0_DE, and 563E10E12_hzH1L0_DLE antibodies, and the afucosylation 563E10E12_hzH1L0_AF antibody were effective in inhibiting tumor growth compared to the negative control group.

### 11.3.2 CCR8 antibodies inhibit growth of the human non-small cell lung cancer cell line NCI-H1299 in human PBMC humanized mouse models

Human non-small cell lung cancer NCI-H1299 cells were inoculated subcutaneously into the right flank the human PBMC humanized mice (GemPharmatech LLC.) at an inoculation volume of 0.2 mL/animal, together with 30% Matrigel. When the average tumor volume was approximately 100 mm³, the mice were randomly divided into groups and administered respectively with an anti-CCR8 antibody (1-20mg/kg), pembrolizumab (1-20mg/kg), the combination of the anti-CCR8 antibody (1-20 mg/kg) and pembrolizumab (1-20mg/kg), and a vehicle control (PBS), twice a week for 4 weeks. After administration, the mice were fed normally and the tumor volume was recorded. Tumor samples were harvested at the end of the experiment, minced with an anatomical laboratory scissor. Tumor tissues were dissociated with a human tumor dissociation kit (Miltenyi Biotech) in combination with the gentleMACS dissociator (Miltenyi Biotec) and the cell suspension obtained was filtered via the 70 µm MACS SmartStrainers to generate a single cell suspension. Immune cell subpopulatios (Treg and CD8⁺ T cells) were analyzed with a flow cytometer.

The 563E10E12_hzH1L0_hIgG1 antibody, the Fc-mutated 563E10E12_hzH1L0_VLPLL, 563E10E12_hzH1L0_DE, and 563E10E12_hzH1L0_DLE antibodies, and the afucosylation 563E10E12_hzH1L0_AF antibody were effective in inhibiting tumor growth compared to the vehicle control group.

### 11.3.3 CCR8 antibodies inhibit growth of the human non-small cell lung cancer NCI-H292 in human PBMC-humanized mouse models

Human non-small cell lung cancer NCI-H292 cells were inoculated subcutaneously into the right flank the human PBMC humanized mice (GemPharmatech LLC.) at an inoculation volume of 0.2 mL/animal, together with 30% Matrigel. When the average tumor volume was approximately 100 mm³, the mice were randomly divided into groups and administered respectively with an anti-CCR8 antibody (1-20 mg/kg), pembrolizumab (1-20mg/kg), the combination of the anti-CCR8 antibody (1-20 mg/kg) and pembrolizumab (1-20mg/kg), and a negative control (PBS), biweekly for 3 weeks. After administration, the mice were fed normally and the tumor volume was recorded. Tumor samples were harvested at the end of the experiment, minced with an anatomical laboratory scissor. Tumor tissues were dissociated with a human tumor dissociation kit (Miltenyi Biotech) in combination with the gentleMACS dissociator (Miltenyi Biotec) and the cell suspension obtained was filtered via the 70 µm MACS SmartStrainers to generate a single cell suspension. Immune cell subpopulations and functional biomarkers were analyzed by flow cytometry. Immune cell subpopulations (Treg and CD8+ T cells) were analyzed by flow cytometry.

The 563E10E12_hzH1L0_hIgG1 antibody, the Fc-mutated 563E10E12_hzH1L0_DE and 563E10E12_hzH1L0_DLE antibodies thereof, and the afucosylation 563E10E12_hzH1L0_AF antibody are effective in inhibiting tumor growth compared to the vehicle control group.

### 11.4 Combination of the CCR8 antibodies and pembrolizumab inhibit growth of human colon carcinoma HT29 in human PBMC-humanized mouse model

Human rectal cancer HT29 cells were inoculated subcutaneously into the right flank the human PBMC humanized mice (Shanghai Model Organisms Center, Inc.,) at an inoculation volume of 0.1 mL/animal, together with 30% Matrigel. When the average tumor volume was approximately 100 mm³, the mice were randomly divided into groups and administered respectively with an anti-CCR8 antibody (10mg/kg), pembrolizumab (3.25mg/kg), the combination of the anti-CCR8 antibody (10mg/kg) and pembrolizumab (3.25mg/kg), and a negative control (PBS), biweekly for 4 weeks. After administration, the mice were fed normally and the tumor volume was recorded. Tumor samples were harvested at the end of the experiment, minced with an anatomical laboratory scissor. Tumor tissues were dissociated with a human tumor dissociation kit (Miltenyi Biotech) in combination with the gentleMACS dissociator (Miltenyi Biotec) and the cell suspension obtained was filtered via the 70 µm MACS SmartStrainers to yield a single cell suspension. Immune cell subpopulations (Treg and CD8⁺ T cells) were analyzed with a flow cytometry.

The 563E10E12_hzH1L0_hIgG1 antibody, the Fc-mutated 563E10E12_hzH1L0_VLPLL, 563E10E12_hzH1L0_DE antibodies thereof and 563E10E12_hzH1L0_DLE, and the fucosylation-reduced antibody 563E10E12_hzH1L0_AF are effective in inhibiting tumor growth compared to the vehicle control group.

### 11.5 Efficacy of anti-CCR8 antibodies in the EMT-6 tumor model of breast cancer in human CCR8-humanized mice

Murine EMT-6 breast cancer cells were inoculated subcutaneously into the right flank the human CCR8 humanized mice (Shanghai Model Organisms Center, Inc.) at an inoculation volume of 0.1 mL/animal (in PBS). When the average tumor volume was approximately 50-100 mm³, the mice were randomly divided into groups and administered respectively with an anti-CCR8 antibody (1-20 mg/kg) and a vehicle control (PBS), twice a week for 3 weeks. After administration, the mice were fed normally and the tumor volume was recorded. Tumor samples were harvested at the end of the experiment, minced with an anatomical laboratory scissor. Tumor tissues were dissociated with a human tumor dissociation kit (Miltenyi Biotech) in combination with the gentleMACS dissociator (Miltenyi Biotec) and the cell suspension obtained was filtered via the 70 µm MACS SmartStrainers to generate a single cell suspension. Immune cell subpopulations (Treg and CD8⁺ T cells) were analyzed with a flow cytometer.

The 563E10E12_hzH1L0_hIgG1 antibody, the Fc-mutated 563E10E12_hzH1L0VLPLL, 563E10E12_hzH1L0_DE, and 563E10E12_hzH1L0_DLE antibodies, and the afucosylation 563E10E12_hzH1L0_AF antibody were effective in inhibiting tumor growth compared to the vehicle control group.

Subsequently, murine EMT-6 breast cancer cells were inoculated subcutaneously into the right flank of human CCR8 humanized mice (Shanghai Model Organisms Center, Inc.) at an inoculation volume of 0.1 mL/animal (in PBS). When the average tumor volume was approximately 50-100 mm³, the mice were randomly divided into groups and administered respectively with an anti-CCR8 antibody (1-20 mg/kg), the anti-mouse PD-1 (mPD-1) (Leinco Technologies, P372), and a vehicle control (PBS), administered twice weekly. After administration, the mice were fed normally and the tumor volume was recorded. Tumor samples were harvested at the end of the experiment, minced with an anatomical laboratory scissor. Tumor tissues were dissociated with a human tumor dissociation kit (Miltenyi Biotech) in combination with the gentleMACS dissociator (Miltenyi Biotec) and the cell suspension obtained was filtered via the 70 µm MACS SmartStrainers to generate a single cell suspension. Immune cell subpopulations (Treg and CD8⁺ T cells) were analyzed with a flow cytometry.

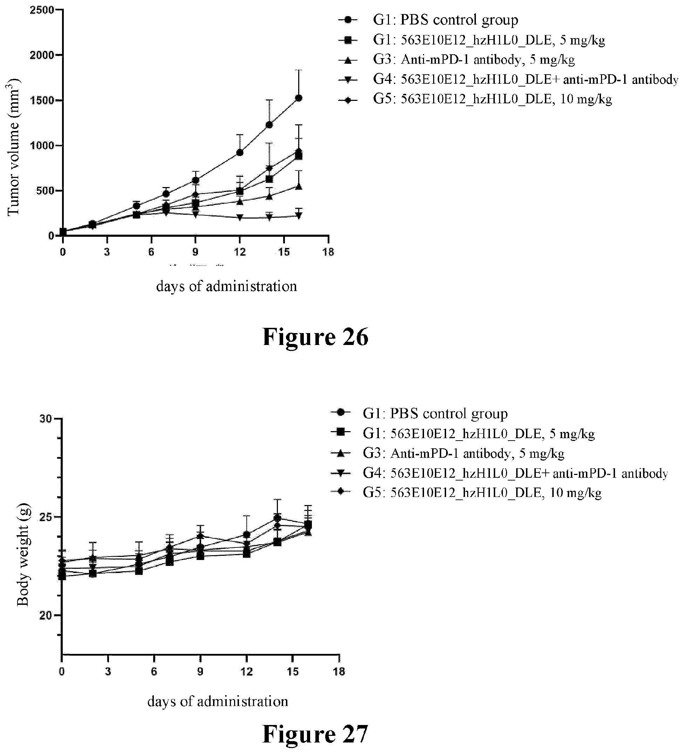

The results are shown in the Fig. 26. 563E10E12_hzH1L0_DLE administered alone at the doses of both 5 mg/kg and 10 mg/kg exerts the significant antitumor activity in the humanized mice B-hCCR8 inoculated with EMT-6 cells, compared to the vehicle control group. The increase in tumor volume was significantly inhibited (p<0.05) (Fig. 26). Moreover, tumor growth inhibition rate for 563E10E12_hzH1L0_DLE at the same dose of 5 mg/kg in combination administration with the anti-mPD-1 antibody (5 mg/kg) (TGI = 94.2%) is better than those for 563E10E12_hzH1L0_DLE alone (TGI = 53.8%) and the anti-mPD-1 antibody alone (TGI = 76.4%).

During the experiment, no obvious adverse event were observed in all treatment groups. Similar to those in the vehicle control group, the body weights of animals in the dosing groups gradually increased, indicating that the animals were well tolerated to the test articles (Fig. 27). At the end of the experiment, the Treg elimination rates in the tumors among the groups of 5 mg/kg 563E10E12_hzH1L0_DLE antibody, anti-mPD-1 antibody, of the combination administration and of 10 mg/kg 563E10E12_hzH1L0 _DLE antibody were 26.3%, 24.9%, 23.4%, and 27.3%, respectively, compared to that in the vehicle control group; with the ratios of CD8⁺ T/Treg cells increased by 32.5%, 22.6%, 46.8%, and 11.5%, respectively.

### 11.6 Efficacy of anti-CCR8 antibodies in the melanoma B16F10 tumor model in human CCR8-humanized mice

Murine B16F10 melanoma cells were inoculated subcutaneously into the right flank the human CCR8 humanized mice (Biocytogen) at an inoculation volume of 0.1 mL/animal (in PBS). When the average tumor volume was approximately 50-100 mm³, the mice were randomly divided into groups and administered respectively with an anti-CCR8 antibody (1-20 mg/kg), the anti-mouse PD1 monoclonal antibody (1-20 mg/kg), the combination of the anti-CCR8 antibody (1-20 mg/kg) and the anti-mouse PD1 monoclonal antibody (1-20 mg/kg), and a vehicle control (PBS), twice a week for 3 weeks. After administration, the mice were fed normally and the tumor volume was recorded. Tumor samples were harvested at the end of the experiment, minced with an anatomical laboratory scissor. Tumor tissues were dissociated with a human tumor dissociation kit (Miltenyi Biotech) in combination with the gentleMACS dissociator (Miltenyi Biotec) and the cell suspension obtained was filtered via the 70 µm MACS SmartStrainers to generate a single cell suspension. Immune cell subpopulations (Treg and CD8⁺ T cells) were analyzed with a flow cytometry.

The 563E10E12_hzH1L0_hIgG1 antibody, the Fc-mutated 563E10E12_hzH1L0VLPLL, 563E10E12_hzH1L0 DE, and 563E10E12_hzH1L0_DLE antibodies, and the afucosylation 563E10E12_hzH1L0_AF antibody were effective in inhibiting tumor growth compared to the vehicle control group.

### 11.7 Efficacy of anti-CCR8 antibodies in the CT26 tumor model of colorectal cancer in human CCR8-humanized mice

Murine CT26 Colorectal carcinoma cells were inoculated subcutaneously into the right flank the human CCR8 humanized mice (Shanghai Model Organisms Center, Inc.) at an inoculation volume of 0.1 mL/animal (in PBS). When the average tumor volume was approximately 50-100 mm³, the mice were randomly divided into groups and administered respectively with an anti-CCR8 antibody (1-20 mg/kg), the anti-mouse PD1 monoclonal antibody (1-20 mg/kg), the combination of the anti-CCR8 antibody (1-20 mg/kg) and the anti-mouse PD1 monoclonal antibody (1-20 mg/kg), and a vehicle control (PBS), twice a week for 3 weeks. After administration, the mice were fed normally and the tumor volume was recorded. Tumor samples were harvested at the endof the experiment, minced with an anatomical laboratory scissor. Tumor tissues were dissociated with a human tumor dissociation kit (Miltenyi Biotech) in combination with the gentleMACS dissociator (Miltenyi Biotec) and the cell suspension obtained was filtered via the 70 µm MACS SmartStrainers to generate a single cell suspension. Immune cell subpopulations (Treg and CD8+ T cells) were analyzed with a flow cytometry.

The 563E10E12_hzH1L0_hIgG1 antibody, the Fc-mutated 563E10E12_hzH1L0_VLPLL, 563E10E12_hzH1L0 DE, and 563E10E12_hzH1L0_DLE antibodies, and the afucosylation 563E10E12_hzH1L0_AF antibody were effective in inhibiting tumor growth compared to the vehicle control group.

Various modifications and variations of the described methods and system of the invention will be apparent to those skilled in the art without departing from the scope and spirit of the invention. Although the present invention has been described in connection with specific preferred embodiments, it should be understood that the invention as claimed should not be unduly limited to such specific embodiments. Indeed, various modifications of the described modes for carrying out the invention which are obvious to those skilled in molecular biology, immunology or related fields are intended to be within the scope of the claims attached.

### Sequence Listing

| Numb er | Sequence | Name |
|---|---|---|
| 1 | AYAMN | 563E10E12 HCDR1 |
| 2 | RIRSKSNNFATYYADSVTD | 563E10E12 HCDR2 |
| 3 | HGRRDLLYSMDF | 563E10E12 HCDR3 |
| 4 | DYYVN | 559E1B10 HCDR1 |
| 5 | VINPHNGDTRYNQKFKG | 559E1B10 HCDR2 |
| 6 | WLRRGYYFDS | 559E1B10 HCDR3 |
| 7 | GYNMN | 27B9-1G3 HCDR1 |
| 8 | NIDPYYGGTNYNQKFKG | 27B9-1G3 HCDR2 |
| 9 | ETWFITTGVIKKGFYFDY | 27B9-1G3 HCDR3 |
| 10 | RIRSKSNDFATYYADSVKG | 569D11B5 HCDR2 |
| 11 | RIRSKSNNFATYYADSVKD | 589D7C7 HCDR2 |
| 12 | RSSKSLLHSNGITYLF | 563E10E12 LCDR1 |
| 13 | QMSNLAS | 563E10E12 LCDR2 |
| 14 | AQSLELPL | 563E10E12 LCDR3 |
| 15 | RASQSISDYLH | 559E1B10 LCDR1 |
| 16 | YVSQSIS | 559E1B10 LCDR2 |
| 17 | QNGHSFPLT | 559E1B10 LCDR3 |
| 18 | SASSIISSNYLH | 27B9-1G3 LCDR1 |
| 19 | RTSSLAS | 27B9-1G3 LCDR2 |
| 20 | QQGSSIPRT | 27B9-1G3 LCDR3 |
| 21 | EVQLVESGGGLVQPGGSLKLSCAASGFNFNAYAMNWVRQAS GKGLEWVGRIRSKSNNFATYYADSVTDRFTISRDDSKNTAYL QMNSLKTEDTAVYYCVRHGRRDLLYSMDFWGQGTTVTVSS | 563E10E12_hzH1 |
| 22 | EVQLVESGGGLVQPGGSLKLSCAASGFTFSAYAMNWVRQAS GKGLEWVGRIRSKSNNFATYYADSVTDRFTISRDDSKNTAYL QMNSLKTEDTAVYYCVRHGRRDLLYSMDFWGQGTTVTVSS | 563E10E12_hzH0 |
| 23 | EVQLVESGGGLVQPGGSLKLSCAASGFTFSAYAMNWVRQAS GKGLEWVARIRSKSNNFATYYADSVTDRFTISRDDSKSTLYL QMNSLKTEDTAVYYCVRHGRRDLLYSMDFWGQGTTVTVSS | 563E10E12_hzH2 |
| 24 | EVQLVESGGGLVQPGGSLKLSCAASGFNFNAYAMNWVRQAS GKGLEWVARIRSKSNNFATYYADSVTDRFTISRDDSKSTLYL QMNSLKTEDTAVYYCVRHGRRDLLYSMDFWGQGTTVTVSS | 563E10E12_hzH3 |
| 25 | EVQLVESGGGLVQPGGSLKLSCAASGFNFNAYAMNWVRQAS GKGLEWVARIRSKSNDFATYYADSVKDRFTISRDDSKSTLYL QMNSLKTEDTAVYYCVRHGRRDLLYSMDFWGQGTTVTVSS | 563E10E12_hzH4 |
| 26 | EVQLVESGGGLVQPGGSLKLSCAASGFNFNAYAMNWVRQAS GKGLEWVARIRSKSNDFATYYADSVKGRFTISRDDSKNTLYL QMNSLKTEDTAVYYCVRHGRRDLLYSMDFWGQGTTVTVSS | 563E10E12_hzH5 |
| 27 | EVQLVESGGGLVQPGGSLKLSCAASGFNFNAYAMNWVRQAS GKGLEWVARIRSKSNNFATYYADSVKDRFTISRDDSKSTVYL QMNSLKTEDTAVYYCVRHGRRDLLYSMDFWGQGTTVTVSS | 563E10E12_hzH6 |
| 28 | QVQLVQSGAEVKKPGASVKVSCKASGYTFTDYYVNWVRQA PGQGLEWMGVINPHNGDTRYNQKFKGRVTMTRDTSTSTVY MELSSLRSEDTAVYYCASWLRRGYYFDSWGQGTTVTVSS | 559E1B10_hzH0 |
| 29 | QVQLVQSGAEVKKPGASVKVSCKASGYTFTDYYVNWVRQA PGQGLEWMGVINPHNGDTRYNQKFKGRVTMTVDKSTSTVY MELSSLRSEDTAVYYCASWLRRGYYFDSWGQGTTVTVSS | 559E1B10_hzH1 |
| 30 | QVQLVQSGAEVKKPGASVKVSCKASGYTFTDYYVNWVRQA PGQGLEWIGVINPHNGDTRYNQKFKGRATLTVDKSTSTVYME LSSLRSEDTAVYYCASWLRRGYYFDSWGQGTTVTVSS | 559E1B10_hzH2: |
| 31 | KVQLVQSGAEVKKPGASVKVSCKASGYTFTDYYVNWVRQA PGQGLEWIGVINPHNGDTRYNQKFKGKATLTVDKSTSTAYM ELSSLRSEDTAVYYCASWLRRGYYFDSWGQGTTVTVSS | 559E1B10_hzH3 |
| 32 | QVQLVQSGAEVKKPGSSVKVSCKASGGTFSGYNMNWVRQA PGQGLEWMGNIDPYYGGTNYNQKFKGRVTITADKSTSTAYM ELSSLRSEDTAVYYCARETWFITTGVIKKGFYFDYWGQGTTV TVSS | 27B9-1G3_hzVH01 |
| 33 | QVQLVQSGAEVKKPGSSVKVSCKASGYSFTGYNMNWVRQA PGQGLEWMGNIDPYYGGTNYNQKFKGRVTITADKSTSTAYM ELSSLRSEDTAVYYCARETWFITTGVIKKGFYFDYWGQGTTV TVSS | 27B9-1G3_hzVH02 |
| 34 | QVQLVQSGAEVKKPGSSVKVSCKASGGTFSGYNMNWVRQA PGQGLEWMGNIDPYYGGTNYNQKFKGKATITADKSTSTAYM ELSSLRSEDTAVYYCARETWFITTGVIKKGFYFDYWGQGTTV TVSS | 27B9-1G3_hzVH03 |
| 35 | QVQLVQSGAEVKKPGSSVKVSCKASGYSFTGYNMNWVRQA PGQGLEWMGNIDPYYGGTNYNQKFKGKATITADKSTSTAYM ELSSLRSEDTAVYYCARETWFITTGVIKKGFYFDYWGQGTTV TVSS | 27B9-1G3_hzVH04 |
| | | |
| 36 | EVQLIESGGGLVQPKGSLKLSCAASGFNFNAYAMNWVRQAP GKGLEWVARIRSKSNNFATYYADSVTDRFTISRDDSESMLYL QMNNLKTEDTAMYYCVRHGRRDLLYSMDFWGQGTSVTVSS | 563E10E12_mVH |
| 37 | EVQLIESGGGLVQPKGSLKLSCAASGFNFNAYAMNWVRQAP GKGLEWVARIRSKSNDFATYYADSVKGRFTISRDDSENMLFL QMNNLKTEDTAMYYCVRHGRRDLLYSMDFWGQGTSVTVSS | 569D11B5 mVH |
| 38 | EVQLVESGGGLVQPKGSLKLSCAASGFNFNAYAMNWVRQAP GKGLEWVARIRSKSNNFATYYADSVKDRFTISRDDSESMVYL EMNNLKTEDTAMYYCVRHGRRDLLYSMDFWGQGTSVTVSS | 589D7C7_mVH |
| 39 | KVQLQQSGPVLVKPGASVKMSCKASGYTFTDYYVNWMKQN YGKSPEWIGVINPHNGDTRYNQKFKGKATLTVDKSSSTANME LTSLTSGDSAVYYCASWLRRGYYFDSWGQGTTLTVSS | 559E1B10 mVH |
| 40 | EIQLQQSGPELEKPGASVKISCKASGYSFTGYNMNWVKKSNG KSLEWIGNIDPYYGGTNYNQKFKGKATLTVDKSSSTAYMQL KSLTSEDSAVYYCARETWFITTGVIKKGFYFDYWGQGTTLTV SS | 27B9-1G3_mVH |
| 41 | IRSKSNDFATYYADSVKD | 563E10E12_hzH4 HCDR2 |
| 42 | DIVMTQSPLSLPVTPGEPASISCRSSKSLLHSNGITYLFWYLQK PGQSPQLLIYQMSNLASGVPDRFSGSGSGTDFTLKISRVEAED VGVYYCAQSLELPLTFGGGTKVEIK | 563E10E12_hzL0 |
| 43 | DVVMTQSPLSLPVTPGEPASISCRSSKSLLHSNGITYLFWYLQK PGQSPQLLIYQMSNLASGVPDRFSGSGSGTDFTLKISRVEAED VGVYYCAQSLELPLTFGGGTKVEIK | 563E10E12_hzL1 |
| 44 | EIVLTQSPATLSLSPGERATLSCRASQSISDYLHWYQQKPGQA PRLLIYYVSQSISGIPARFSGSGSGTDFTLTISSLEPEDFAVYYC QNGHSFPLTFGGGTKVEIK | 559E1B10_hzL0 |
| 45 | EIVLTQSPATLSLSPGERATLSCRASQSISDYLHWYQQKPGQA PRLLLKYVSQSISGIPARFSGSGSGTDFTLTISSLEPEDFAVYYC QNGHSFPLTFGGGTKVEIK | 559E1B10_hzL1 |
| 46 | DIVMTQSPATLSLSPGERATLSCRASQSISDYLHWYQQKPGQA PRLLLKYVSQSISGIPARFSGSGSGTDFTLTISSLEPEDFAVYYC QNGHSFPLTFGGGTKVEIK | 559E1B10_hzL2 |
| 47 | EIVLTQSPATLSLSPGERATLSCSASSIISSNYLHWYQQKPGQA PRLLIYRTSSLASGIPARFSGSGSGTDFTLTISSLEPEDFAVYYC QQGSSIPRTFGGGTKVEIK | 27B9-1G3_hzVL01 |
| 48 | EIVLTQSPATLSLSPGERATLACSASSIISSNYLHWYQQKPGQA PRLLIYRTSSLASGIPARFSGSGSGTDYTLTISSLEPEDFAVYYC QQGSSIPRTFGGGTKVEIK | 27B9-1G3_hzVL02 |
| | | |
| 49 | EIVLTQSPATLSLSPGERATLACSASSIISSNYLHWYQQKPGQS PRLLIYRTSSLASGVPARFSGSGSGTSYTLTISSLEPEDFAVYYC QQGSSIPRTFGGGTKVEIK | 27B9-1G3_hzVL03 |
| 50 | DVVMTQAAFSNPVTLGTSTSISCRSSKSLLHSNGITYLFWYLQ KPGQSPQLLIYQMSNLASGVPDRFSGSGSGTDFTLRISRVEAE DVGVYYCAQSLELPLTFGGGTKLEIK | 563E10E12 mVL |
| 51 | DIVMTQAAFSNPVTLGTSASISCRSSKSLLHSNGITYLFWYLQ KPGQSPQLLIYQMSNLASGVPDRFSGSGSGTDFTLRISRVEAE DVGVYYCAQSLELPLTFGGGTKLEIK | 569D11B5 mVL |
| 52 | DIVMTQAAFSNPVTLGTSASISCRSSKSLLHSNGITYLFWYLQ KPGQSPQLLIYQMSNLASGVPDRFSGSGSGTDFTLRISRVEAE DVAVYYCAQSLELPLTFGGGTKLEIK | 589D7C7 mVL |
| 53 | DIVMTQSPATLSVTPGDRVSLSCRASQSISDYLHWYQQKSHES PRLLLKYVSQSISGIPSRFSGSGSGSDFTLTINSVEPEDVGVYYC QNGHSFPLTFGAGTKLELK | 559E1B10 mVL |
| 54 | EIVLTQSPTTMAASPGEKITLACSASSIISSNYLHWYQQKPGFS PKLLIYRTSSLASGVPARFSGSGSGTSYSLTIGTMEAEDVATYY CQQGSSIPRTFGGGTKLEIK | 27B9-1G3 mVL |
| 55 | EVQLVESGGGLVQPGGSLKLSCAASGFNFNAYAMNWVRQAS GKGLEWVGRIRSKSNNFATYYADSVTDRFTISRDDSKNTAYL QMNSLKTEDTAVYYCVRHGRRDLLYSMDFWGQGTTVTVSS ASTKGPSVFPLAPSSKSTSGGTAALGCLVKDYFPEPVTVSWNS GALTSGVHTFPAVLQSSGLYSLSSVVTVPSSSLGTQTYICNVN HKPSNTKVDKKVEPKSCDKTHTCPPCPAPELLGGPSVFLFPPK PKDTLMISRTPEVTCVVVDVSHEDPEVKFNWYVDGVEVHNA KTKPREEQYNSTYRVVSVLTVLHQDWLNGKEYKCKVSNKAL PAPIEKTISKAKGQPREPQVYTLPPSRDELTKNQVSLTCLVKGF YPSDIAVEWESNGQPENNYKTTPPVLDSDGSFFLYSKLTVDKS RWQQGNVFSCSVMHEALHNHYTQKSLSLSPGK | 563E10E12_hzH1 Heavy chain |
| 56 | EVQLVESGGGLVQPGGSLKLSCAASGFTFSAYAMNWVRQAS GKGLEWVGRIRSKSNNFATYYADSVTDRFTISRDDSKNTAYL QMNSLKTEDTAVYYCVRHGRRDLLYSMDFWGQGTTVTVSS ASTKGPSVFPLAPSSKSTSGGTAALGCLVKDYFPEPVTVSWNS GALTSGVHTFPAVLQSSGLYSLSSVVTVPSSSLGTQTYICNVN HKPSNTKVDKKVEPKSCDKTHTCPPCPAPELLGGPSVFLFPPK PKDTLMISRTPEVTCVVVDVSHEDPEVKFNWYVDGVEVHNA KTKPREEQYNSTYRVVSVLTVLHQDWLNGKEYKCKVSNKAL PAPIEKTISKAKGQPREPQVYTLPPSRDELTKNQVSLTCLVKGF YPSDIAVEWESNGQPENNYKTTPPVLDSDGSFFLYSKLTVDKS RWQQGNVFSCSVMHEALHNHYTQKSLSLSPGK | 563E10E12_hzH0 Heavy chain |
| 57 | EVQLVESGGGLVQPGGSLKLSCAASGFTFSAYAMNWVRQAS GKGLEWVARIRSKSNNFATYYADSVTDRFTISRDDSKSTLYL QMNSLKTEDTAVYYCVRHGRRDLLYSMDFWGQGTTVTVSS ASTKGPSVFPLAPSSKSTSGGTAALGCLVKDYFPEPVTVSWNS GALTSGVHTFPAVLQSSGLYSLSSVVTVPSSSLGTQTYICNVN HKPSNTKVDKKVEPKSCDKTHTCPPCPAPELLGGPSVFLFPPK PKDTLMISRTPEVTCVVVDVSHEDPEVKFNWYVDGVEVHNA KTKPREEQYNSTYRVVSVLTVLHQDWLNGKEYKCKVSNKAL PAPIEKTISKAKGQPREPQVYTLPPSRDELTKNQVSLTCLVKGF YPSDIAVEWESNGQPENNYKTTPPVLDSDGSFFLYSKLTVDKS RWQQGNVFSCSVMHEALHNHYTQKSLSLSPGK | 563E10E12_hzH2 Heavy chain |
| 58 | EVQLVESGGGLVQPGGSLKLSCAASGFNFNAYAMNWVRQAS GKGLEWVARIRSKSNNFATYYADSVTDRFTISRDDSKSTLYL QMNSLKTEDTAVYYCVRHGRRDLLYSMDFWGQGTTVTVSS ASTKGPSVFPLAPSSKSTSGGTAALGCLVKDYFPEPVTVSWNS GALTSGVHTFPAVLQSSGLYSLSSVVTVPSSSLGTQTYICNVN HKPSNTKVDKKVEPKSCDKTHTCPPCPAPELLGGPSVFLFPPK PKDTLMISRTPEVTCVVVDVSHEDPEVKFNWYVDGVEVHNA KTKPREEQYNSTYRVVSVLTVLHQDWLNGKEYKCKVSNKAL PAPIEKTISKAKGQPREPQVYTLPPSRDELTKNQVSLTCLVKGF YPSDIAVEWESNGQPENNYKTTPPVLDSDGSFFLYSKLTVDKS RWQQGNVFSCSVMHEALHNHYTQKSLSLSPGK | 563E10E12_hzH3 Heavy chain |
| 59 | EVQLVESGGGLVQPGGSLKLSCAASGFNFNAYAMNWVRQAS GKGLEWVARIRSKSNDFATYYADSVKDRFTISRDDSKSTLYL QMNSLKTEDTAVYYCVRHGRRDLLYSMDFWGQGTTVTVSS ASTKGPSVFPLAPSSKSTSGGTAALGCLVKDYFPEPVTVSWNS GALTSGVHTFPAVLQSSGLYSLSSVVTVPSSSLGTQTYICNVN HKPSNTKVDKKVEPKSCDKTHTCPPCPAPELLGGPSVFLFPPK PKDTLMISRTPEVTCVVVDVSHEDPEVKFNWYVDGVEVHNA KTKPREEQYNSTYRVVSVLTVLHQDWLNGKEYKCKVSNKAL PAPIEKTISKAKGQPREPQVYTLPPSRDELTKNQVSLTCLVKGF YPSDIAVEWESNGQPENNYKTTPPVLDSDGSFFLYSKLTVDKS RWQQGNVFSCSVMHEALHNHYTQKSLSLSPGK | 563E10E12_hzH4 Heavy chain |
| 60 | EVQLVESGGGLVQPGGSLKLSCAASGFNFNAYAMNWVRQAS GKGLEWVARIRSKSNDFATYYADSVKGRFTISRDDSKNTLYL QMNSLKTEDTAVYYCVRHGRRDLLYSMDFWGQGTTVTVSS ASTKGPSVFPLAPSSKSTSGGTAALGCLVKDYFPEPVTVSWNS GALTSGVHTFPAVLQSSGLYSLSSVVTVPSSSLGTQTYICNVN HKPSNTKVDKKVEPKSCDKTHTCPPCPAPELLGGPSVFLFPPK PKDTLMISRTPEVTCVVVDVSHEDPEVKFNWYVDGVEVHNA KTKPREEQYNSTYRVVSVLTVLHQDWLNGKEYKCKVSNKAL PAPIEKTISKAKGQPREPQVYTLPPSRDELTKNQVSLTCLVKGF YPSDIAVEWESNGQPENNYKTTPPVLDSDGSFFLYSKLTVDKS RWQQGNVFSCSVMHEALHNHYTQKSLSLSPGK | 563E10E12_hzH5 Heavy chain |
| 61 | EVQLVESGGGLVQPGGSLKLSCAASGFNFNAYAMNWVRQAS GKGLEWVARIRSKSNNFATYYADSVKDRFTISRDDSKSTVYL QMNSLKTEDTAVYYCVRHGRRDLLYSMDFWGQGTTVTVSS ASTKGPSVFPLAPSSKSTSGGTAALGCLVKDYFPEPVTVSWNS GALTSGVHTFPAVLQSSGLYSLSSVVTVPSSSLGTQTYICNVN HKPSNTKVDKKVEPKSCDKTHTCPPCPAPELLGGPSVFLFPPK PKDTLMISRTPEVTCVVVDVSHEDPEVKFNWYVDGVEVHNA | 563E10E12_hzH6 Heavy chain |
| | KTKPREEQYNSTYRVVSVLTVLHQDWLNGKEYKCKVSNKAL PAPIEKTISKAKGQPREPQVYTLPPSRDELTKNQVSLTCLVKGF YPSDIAVEWESNGQPENNYKTTPPVLDSDGSFFLYSKLTVDKS RWQQGNVFSCSVMHEALHNHYTQKSLSLSPGK | |
| 62 | EVQLVESGGGLVQPGGSLKLSCAASGFNFNAYAMNWVRQAS GKGLEWVGRIRSKSNNFATYYADSVTDRFTISRDDSKNTAYL QMNSLKTEDTAVYYCVRHGRRDLLYSMDFWGQGTTVTVSS ASTKGPSVFPLAPSSKSTSGGTAALGCLVKDYFPEPVTVSWNS GALTSGVHTFPAVLQSSGLYSLSSVVTVPSSSLGTQTYICNVN HKPSNTKVDKKVEPKSCDKTHTCPPCPAPELVGGPSVFLLPPK PKDTLMISRTPEVTCVVVDVSHEDPEVKFNWYVDGVEVHNA KTKPPEEQYNSTLRVVSVLTVLHQDWLNGKEYKCKVSNKAL PAPIEKTISKAKGQPREPQVYTLPPSRDELTKNQVSLTCLVKGF YPSDIAVEWESNGQPENNYKTTPLVLDSDGSFFLYSKLTVDKS RWQQGNVFSCSVMHEALHNHYTQKSLSLSPGK | 563E10E12_hzH1 _VLPLL Heavy Chain |
| 63 | EVQLVESGGGLVQPGGSLKLSCAASGFNFNAYAMNWVRQAS GKGLEWVGRIRSKSNNFATYYADSVTDRFTISRDDSKNTAYL QMNSLKTEDTAVYYCVRHGRRDLLYSMDFWGQGTTVTVSS ASTKGPSVFPLAPSSKSTSGGTAALGCLVKDYFPEPVTVSWNS GALTSGVHTFPAVLQSSGLYSLSSVVTVPSSSLGTQTYICNVN HKPSNTKVDKKVEPKSCDKTHTCPPCPAPELLGGPDVFLFPPK PKDTLMISRTPEVTCVVVDVSHEDPEVKFNWYVDGVEVHNA KTKPREEQYNSTYRVVSVLTVLHQDWLNGKEYKCKVSNKAL PAPEEKTISKAKGQPREPQVYTLPPSRDELTKNQVSLTCLVKG FYPSDIAVEWESNGQPENNYKTTPPVLDSDGSFFLYSKLTVDK SRWQQGNVFSCSVMHEALHNHYTQKSLSLSPGK | 563E10E12_hzH1 _DE Heavy Chain |
| 64 | EVQLVESGGGLVQPGGSLKLSCAASGFNFNAYAMNWVRQAS GKGLEWVGRIRSKSNNFATYYADSVTDRFTISRDDSKNTAYL QMNSLKTEDTAVYYCVRHGRRDLLYSMDFWGQGTTVTVSS ASTKGPSVFPLAPSSKSTSGGTAALGCLVKDYFPEPVTVSWNS GALTSGVHTFPAVLQSSGLYSLSSVVTVPSSSLGTQTYICNVN HKPSNTKVDKKVEPKSCDKTHTCPPCPAPELLGGPDVFLFPPK PKDTLMISRTPEVTCVVVDVSHEDPEVKFNWYVDGVEVHNA KTKPREEQYNSTYRVVSVLTVLHQDWLNGKEYKCKVSNKAL PLPEEKTISKAKGQPREPQVYTLPPSRDELTKNQVSLTCLVKG FYPSDIAVEWESNGQPENNYKTTPPVLDSDGSFFLYSKLTVDK SRWQQGNVFSCSVMHEALHNHYTQKSLSLSPGK | 563E10E12_hzH1 _DLE Heavy Chain |
| 65 | QVQLVQSGAEVKKPGASVKVSCKASGYTFTDYYVNWVRQA PGQGLEWMGVINPHNGDTRYNQKFKGRVTMTRDTSTSTVY MELSSLRSEDTAVYYCASWLRRGYYFDSWGQGTTVTVSSAS TKGPSVFPLAPSSKSTSGGTAALGCLVKDYFPEPVTVSWNSGA LTSGVHTFPAVLQSSGLYSLSSVVTVPSSSLGTQTYICNVNHK PSNTKVDKKVEPKSCDKTHTCPPCPAPELLGGPSVFLFPPKPK DTLMISRTPEVTCVVVDVSHEDPEVKFNWYVDGVEVHNAKT KPREEQYNSTYRVVSVLTVLHQDWLNGKEYKCKVSNKALPA PIEKTISKAKGQPREPQVYTLPPSRDELTKNQVSLTCLVKGFYP SDIAVEWESNGQPENNYKTTPPVLDSDGSFFLYSKLTVDKSR WQQGNVFSCSVMHEALHNHYTQKSLSLSPGK | 559E1B10_hzH0 Heavy chain |
| 66 | QVQLVQSGAEVKKPGASVKVSCKASGYTFTDYYVNWVRQA PGQGLEWMGVINPHNGDTRYNQKFKGRVTMTVDKSTSTVY MELSSLRSEDTAVYYCASWLRRGYYFDSWGQGTTVTVSSAS TKGPSVFPLAPSSKSTSGGTAALGCLVKDYFPEPVTVSWNSGA LTSGVHTFPAVLQSSGLYSLSSVVTVPSSSLGTQTYICNVNHK PSNTKVDKKVEPKSCDKTHTCPPCPAPELLGGPSVFLFPPKPK DTLMISRTPEVTCVVVDVSHEDPEVKFNWYVDGVEVHNAKT KPREEQYNSTYRVVSVLTVLHQDWLNGKEYKCKVSNKALPA PIEKTISKAKGQPREPQVYTLPPSRDELTKNQVSLTCLVKGFYP SDIAVEWESNGQPENNYKTTPPVLDSDGSFFLYSKLTVDKSR WQQGNVFSCSVMHEALHNHYTQKSLSLSPGK | 559E1B10_hzH1 Heavy chain |
| 67 | QVQLVQSGAEVKKPGASVKVSCKASGYTFTDYYVNWVRQA PGQGLEWIGVINPHNGDTRYNQKFKGRATLTVDKSTSTVYME LSSLRSEDTAVYYCASWLRRGYYFDSWGQGTTVTVSSASTK GPSVFPLAPSSKSTSGGTAALGCLVKDYFPEPVTVSWNSGALT SGVHTFPAVLQSSGLYSLSSVVTVPSSSLGTQTYICNVNHKPS NTKVDKKVEPKSCDKTHTCPPCPAPELLGGPSVFLFPPKPKDT LMISRTPEVTCVVVDVSHEDPEVKFNWYVDGVEVHNAKTKP REEQYNSTYRVVSVLTVLHQDWLNGKEYKCKVSNKALPAPI EKTISKAKGQPREPQVYTLPPSRDELTKNQVSLTCLVKGFYPS DIAVEWESNGQPENNYKTTPPVLDSDGSFFLYSKLTVDKSRW QQGNVFSCSVMHEALHNHYTQKSLSLSPGK | 559E1B10_hzH2 Heavy chain |
| 68 | KVQLVQSGAEVKKPGASVKVSCKASGYTFTDYYVNWVRQA PGQGLEWIGVINPHNGDTRYNQKFKGKATLTVDKSTSTAYM ELSSLRSEDTAVYYCASWLRRGYYFDSWGQGTTVTVSSASTK GPSVFPLAPSSKSTSGGTAALGCLVKDYFPEPVTVSWNSGALT SGVHTFPAVLQSSGLYSLSSVVTVPSSSLGTQTYICNVNHKPS NTKVDKKVEPKSCDKTHTCPPCPAPELLGGPSVFLFPPKPKDT LMISRTPEVTCVVVDVSHEDPEVKFNWYVDGVEVHNAKTKP REEQYNSTYRVVSVLTVLHQDWLNGKEYKCKVSNKALPAPI EKTISKAKGQPREPQVYTLPPSRDELTKNQVSLTCLVKGFYPS DIAVEWESNGQPENNYKTTPPVLDSDGSFFLYSKLTVDKSRW QQGNVFSCSVMHEALHNHYTQKSLSLSPGK | 559E1B10_hzH3 Heavy chain |
| 69 | QVQLVQSGAEVKKPGASVKVSCKASGYTFTDYYVNWVRQA PGQGLEWMGVINPHNGDTRYNQKFKGRVTMTVDKSTSTVY MELSSLRSEDTAVYYCASWLRRGYYFDSWGQGTTVTVSSAS TKGPSVFPLAPSSKSTSGGTAALGCLVKDYFPEPVTVSWNSGA LTSGVHTFPAVLQSSGLYSLSSVVTVPSSSLGTQTYICNVNHK PSNTKVDKKVEPKSCDKTHTCPPCPAPELLGGPDVFLFPPKPK DTLMISRTPEVTCVVVDVSHEDPEVKFNWYVDGVEVHNAKT KPREEQYNSTYRVVSVLTVLHQDWLNGKEYKCKVSNKALPA PEEKTISKAKGQPREPQVYTLPPSRDELTKNQVSLTCLVKGFY PSDIAVEWESNGQPENNYKTTPPVLDSDGSFFLYSKLTVDKSR WQQGNVFSCSVMHEALHNHYTQKSLSLSPGK | 559E1B10_hzH1L 1_DE Heavy Chain |
| 70 | QVQLVQSGAEVKKPGASVKVSCKASGYTFTDYYVNWVRQA PGQGLEWMGVINPHNGDTRYNQKFKGRVTMTVDKSTSTVY MELSSLRSEDTAVYYCASWLRRGYYFDSWGQGTTVTVSSAS TKGPSVFPLAPSSKSTSGGTAALGCLVKDYFPEPVTVSWNSGA LTSGVHTFPAVLQSSGLYSLSSVVTVPSSSLGTQTYICNVNHK PSNTKVDKKVEPKSCDKTHTCPPCPAPELVGGPSVFLLPPKPK DTLMISRTPEVTCVVVDVSHEDPEVKFNWYVDGVEVHNAKT KPPEEQYNSTLRVVSVLTVLHQDWLNGKEYKCKVSNKALPA PIEKTISKAKGQPREPQVYTLPPSRDELTKNQVSLTCLVKGFYP SDIAVEWESNGQPENNYKTTPLVLDSDGSFFLYSKLTVDKSR WQQGNVFSCSVMHEALHNHYTQKSLSLSPGK | 559E1B10_hzH1L 1_VLPLL Heavy Chain |
| 71 | QVQLVQSGAEVKKPGSSVKVSCKASGGTFSGYNMNWVRQA PGQGLEWMGNIDPYYGGTNYNQKFKGRVTITADKSTSTAYM ELSSLRSEDTAVYYCARETWFITTGVIKKGFYFDYWGQGTTV TVSSASTKGPSVFPLAPSSKSTSGGTAALGCLVKDYFPEPVTV SWNSGALTSGVHTFPAVLQSSGLYSLSSVVTVPSSSLGTQTYI CNVNHKPSNTKVDKKVEPKSCDKTHTCPPCPAPELLGGPSVF LFPPKPKDTLMISRTPEVTCVVVDVSHEDPEVKFNWYVDGVE VHNAKTKPREEQYNSTYRVVSVLTVLHQDWLNGKEYKCKVS NKALPAPIEKTISKAKGQPREPQVYTLPPSRDELTKNQVSLTCL VKGFYPSDIAVEWESNGQPENNYKTTPPVLDSDGSFFLYSKLT VDKSRWQQGNVFSCSVMHEALHNHYTQKSLSLSPGK | 27B9-1G3_hzVH01 Heavy chain |
| 72 | QVQLVQSGAEVKKPGSSVKVSCKASGYSFTGYNMNWVRQA PGQGLEWMGNIDPYYGGTNYNQKFKGRVTITADKSTSTAYM ELSSLRSEDTAVYYCARETWFITTGVIKKGFYFDYWGQGTTV TVSSASTKGPSVFPLAPSSKSTSGGTAALGCLVKDYFPEPVTV SWNSGALTSGVHTFPAVLQSSGLYSLSSVVTVPSSSLGTQTYI CNVNHKPSNTKVDKKVEPKSCDKTHTCPPCPAPELLGGPSVF LFPPKPKDTLMISRTPEVTCVVVDVSHEDPEVKFNWYVDGVE VHNAKTKPREEQYNSTYRVVSVLTVLHQDWLNGKEYKCKVS NKALPAPIEKTISKAKGQPREPQVYTLPPSRDELTKNQVSLTCL VKGFYPSDIAVEWESNGQPENNYKTTPPVLDSDGSFFLYSKLT VDKSRWQQGNVFSCSVMHEALHNHYTQKSLSLSPGK | 27B9-1G3_hzVH02 Heavy chain |
| 73 | QVQLVQSGAEVKKPGSSVKVSCKASGGTFSGYNMNWVRQA PGQGLEWMGNIDPYYGGTNYNQKFKGKATITADKSTSTAYM ELSSLRSEDTAVYYCARETWFITTGVIKKGFYFDYWGQGTTV TVSSASTKGPSVFPLAPSSKSTSGGTAALGCLVKDYFPEPVTV SWNSGALTSGVHTFPAVLQSSGLYSLSSVVTVPSSSLGTQTYI CNVNHKPSNTKVDKKVEPKSCDKTHTCPPCPAPELLGGPSVF LFPPKPKDTLMISRTPEVTCVVVDVSHEDPEVKFNWYVDGVE VHNAKTKPREEQYNSTYRVVSVLTVLHQDWLNGKEYKCKVS NKALPAPIEKTISKAKGQPREPQVYTLPPSRDELTKNQVSLTCL VKGFYPSDIAVEWESNGQPENNYKTTPPVLDSDGSFFLYSKLT VDKSRWQQGNVFSCSVMHEALHNHYTQKSLSLSPGK | 27B9-1G3_hzVH03 Heavy chain |
| 74 | QVQLVQSGAEVKKPGSSVKVSCKASGYSFTGYNMNWVRQA PGQGLEWMGNIDPYYGGTNYNQKFKGKATITADKSTSTAYM ELSSLRSEDTAVYYCARETWFITTGVIKKGFYFDYWGQGTTV TVSSASTKGPSVFPLAPSSKSTSGGTAALGCLVKDYFPEPVTV SWNSGALTSGVHTFPAVLQSSGLYSLSSVVTVPSSSLGTQTYI CNVNHKPSNTKVDKKVEPKSCDKTHTCPPCPAPELLGGPSVF LFPPKPKDTLMISRTPEVTCVVVDVSHEDPEVKFNWYVDGVE VHNAKTKPREEQYNSTYRVVSVLTVLHQDWLNGKEYKCKVS NKALPAPIEKTISKAKGQPREPQVYTLPPSRDELTKNQVSLTCL VKGFYPSDIAVEWESNGQPENNYKTTPPVLDSDGSFFLYSKLT VDKSRWQQGNVFSCSVMHEALHNHYTQKSLSLSPGK | 27B9-1G3_hzVH04 Heavy chain |
| 75 | DIVMTQSPLSLPVTPGEPASISCRSSKSLLHSNGITYLFWYLQK PGQSPQLLIYQMSNLASGVPDRFSGSGSGTDFTLKISRVEAED VGVYYCAQSLELPLTFGGGTKVEIKRTVAAPSVFIFPPSDEQL KSGTASVVCLLNNFYPREAKVQWKVDNALQSGNSQESVTEQ DSKDSTYSLSSTLTLSKADYEKHKVYACEVTHQGLSSPVTKSF NRGEC | 563E10E12_hzL0 Light chain |
| 76 | DVVMTQSPLSLPVTPGEPASISCRSSKSLLHSNGITYLFWYLQK PGQSPQLLIYQMSNLASGVPDRFSGSGSGTDFTLKISRVEAED VGVYYCAQSLELPLTFGGGTKVEIKRTVAAPSVFIFPPSDEQL KSGTASVVCLLNNFYPREAKVQWKVDNALQSGNSQESVTEQ DSKDSTYSLSSTLTLSKADYEKHKVYACEVTHQGLSSPVTKSF NRGEC | 563E10E12_hzL1 Light chain |
| 77 | EIVLTQSPATLSLSPGERATLSCRASQSISDYLHWYQQKPGQA PRLLIYYVSQSISGIPARFSGSGSGTDFTLTISSLEPEDFAVYYC QNGHSFPLTFGGGTKVEIKRTVAAPSVFIFPPSDEQLKSGTASV VCLLNNFYPREAKVQWKVDNALQSGNSQESVTEQDSKDSTY SLSSTLTLSKADYEKHKVYACEVTHQGLSSPVTKSFNRGEC | 559E1B10_hzL0 Light chain |
| 78 | EIVLTQSPATLSLSPGERATLSCRASQSISDYLHWYQQKPGQA PRLLLKYVSQSISGIPARFSGSGSGTDFTLTISSLEPEDFAVYYC QNGHSFPLTFGGGTKVEIKRTVAAPSVFIFPPSDEQLKSGTASV VCLLNNFYPREAKVQWKVDNALQSGNSQESVTEQDSKDSTY SLSSTLTLSKADYEKHKVYACEVTHQGLSSPVTKSFNRGEC | 559E1B10_hzL1 Light chain |
| 79 | DIVMTQSPATLSLSPGERATLSCRASQSISDYLHWYQQKPGQA PRLLLKYVSQSISGIPARFSGSGSGTDFTLTISSLEPEDFAVYYC QNGHSFPLTFGGGTKVEIKRTVAAPSVFIFPPSDEQLKSGTASV VCLLNNFYPREAKVQWKVDNALQSGNSQESVTEQDSKDSTY SLSSTLTLSKADYEKHKVYACEVTHQGLSSPVTKSFNRGEC | 559E1B10_hzL2 Light chain |
| 80 | EIVLTQSPATLSLSPGERATLSCSASSIISSNYLHWYQQKPGQA PRLLIYRTSSLASGIPARFSGSGSGTDFTLTISSLEPEDFAVYYC QQGSSIPRTFGGGTKVEIKRTVAAPSVFIFPPSDEQLKSGTASV VCLLNNFYPREAKVQWKVDNALQSGNSQESVTEQDSKDSTY SLSSTLTLSKADYEKHKVYACEVTHQGLSSPVTKSFNRGEC | 27B9-1G3_hzVL01 Light chain |
| 81 | EIVLTQSPATLSLSPGERATLACSASSIISSNYLHWYQQKPGQA PRLLIYRTSSLASGIPARFSGSGSGTDYTLTISSLEPEDFAVYYC QQGSSIPRTFGGGTKVEIKRTVAAPSVFIFPPSDEQLKSGTASV VCLLNNFYPREAKVQWKVDNALQSGNSQESVTEQDSKDSTY SLSSTLTLSKADYEKHKVYACEVTHQGLSSPVTKSFNRGEC | 27B9-1G3_hzVL02 Light chain |
| 82 | EIVLTQSPATLSLSPGERATLACSASSIISSNYLHWYQQKPGQS PRLLIYRTSSLASGVPARFSGSGSGTSYTLTISSLEPEDFAVYYC QQGSSIPRTFGGGTKVEIKRTVAAPSVFIFPPSDEQLKSGTASV VCLLNNFYPREAKVQWKVDNALQSGNSQESVTEQDSKDSTY SLSSTLTLSKADYEKHKVYACEVTHQGLSSPVTKSFNRGEC | 27B9-1G3_hzVL03 Light chain |
| 83 | AKTTAPSVYPLAPVCGDTTGSSVTLGCLVKGYFPEPVTLTWNS GSLSSGVHTFPAVLQSDLYTLSSSVTVTSSTWPSQSITCNVAHP ASSTKVDKKIEPRGPTIKPCPPCKCPAPNLLGGPSVFIFPPKIKD VLMISLSPIVTCVVVDVSEDDPDVQISWFVNNVEVHTAQTQT HREDYNSTLRVVSALPIQHQDWMSGKEFKCKVNNKDLPAPIE RTISKPKGSVRAPQVYVLPPPEEEMTKKQVTLTCMVTDFMPE DIYVEWTNNGKTELNYKNTEPVLDSDGSYFMYSKLRVEKKN WVERNSYSCSVVHEGLHNHHTTKSFSRTPGK | mIgG2a Heavy Chain Constant Region |
| 84 | RADAAPTVSIFPPSSEQLTSGGASVVCFLNNFYPKDINVKWKI DGSERQNGVLNSWTDQDSKDSTYSMSSTLTLTKDEYERHNSY TCEATHKTSTSPIVKSFNRNEC | mIgG2a Light Chain Constant Region |
| 85 | | IgG1Heavy Chain Constant Region |
| 86 | | IgGK Light Chain Constant Region |
| 87 | ASTKGPSVFPLAPSSKSTSGGTAALGCLVKDYFPEPVTVSWNS GALTSGVHTFPAVLQSSGLYSLSSVVTVPSSSLGTQTYICNVN HKPSNTKVDKKVEPKSCDKTHTCPPCPAPELVGGPSVFLLPPK PKDTLMISRTPEVTCVVVDVSHEDPEVKFNWYVDGVEVHNA KTKPPEEQYNSTLRVVSVLTVLHQDWLNGKEYKCKVSNKAL PAPIEKTISKAKGQPREPQVYTLPPSRDELTKNQVSLTCLVKGF YPSDIAVEWESNGQPENNYKTTPLVLDSDGSFFLYSKLTVDKS RWQQGNVFSCSVMHEALHNHYTQKSLSLSPGK | IgG1Heavy Chain Constant Region with the VLPLL mutation |
| 88 | ASTKGPSVFPLAPSSKSTSGGTAALGCLVKDYFPEPVTVSWNS GALTSGVHTFPAVLQSSGLYSLSSVVTVPSSSLGTQTYICNVN HKPSNTKVDKKVEPKSCDKTHTCPPCPAPELLGGPDVFLFPPK PKDTLMISRTPEVTCVVVDVSHEDPEVKFNWYVDGVEVHNA KTKPREEQYNSTYRVVSVLTVLHQDWLNGKEYKCKVSNKAL PAPEEKTISKAKGQPREPQVYTLPPSRDELTKNQVSLTCLVKG FYPSDIAVEWESNGQPENNYKTTPPVLDSDGSFFLYSKLTVDK SRWQQGNVFSCSVMHEALHNHYTQKSLSLSPGK | IgG1Heavy Chain Constant Region with the DE mutation |
| 89 | ASTKGPSVFPLAPSSKSTSGGTAALGCLVKDYFPEPVTVSWNS GALTSGVHTFPAVLQSSGLYSLSSVVTVPSSSLGTQTYICNVN HKPSNTKVDKKVEPKSCDKTHTCPPCPAPELLGGPDVFLFPPK PKDTLMISRTPEVTCVVVDVSHEDPEVKFNWYVDGVEVHNA KTKPREEQYNSTYRVVSVLTVLHQDWLNGKEYKCKVSNKAL PLPEEKTISKAKGQPREPQVYTLPPSRDELTKNQVSLTCLVKG FYPSDIAVEWESNGQPENNYKTTPPVLDSDGSFFLYSKLTVDK SRWQQGNVFSCSVMHEALHNHYTQKSLSLSPGK | IgG1Heavy Chain Constant Region with the DLE mutation |
| 90 | EIVLTQSPATLSLSPGERATLSCRASQSVSSYLAWYQQKPGQA PRLLIYDASNRATGIPARFSGSGSGTDFTLTISSLEPEDFAVYYC | IGKV3-11*01 |
| 91 | QVQLVQSGAEVKKPGSSVKVSCKASGGTFSSYAISWVRQAPG QGLEWMGGIIPIFGTANYAQKFQGRVTITADKSTSTAYMELSS LRSEDTAVYYCAR | IGHV169*06 |
| 92 | QVQLVQSGAEVKKPGASVKVSCKASGYTFTSYYMHWVRQA PGQGLEWMGIINPSGGSTSYAQKFQGRVTMTRDTSTSTVYME LSSLRSEDTAVYYC | hVH 1-46 |
| 93 | EIVLTQSPATLSLSPGERATLSCRASQSVSSYLAWYQQKPGQA PRLLIYDASNRATGIPARFSGSGSGTDFTLTISSLEPEDFAVYYC | hVK 3-11 |
| 94 | EVQLVESGGGLVQPGGSLKLSCAASGFTFSGSAMHWVRQAS GKGLEWVGRIRSKANSYATAYAASVKGRFTISRDDSKNTAYL QMNSLKTEDTAVYYC | hVH 3-73 |
| 95 | DIVMTQSPLSLPVTPGEPASISCRSSQSLLHSNGYNYLDWYLQ KPGQSPQLLIYLGSNRASGVPDRFSGSGSGTDFTLKISRVEAED VGVYYC | hVK 2-28 |

## Claims

1. An antibody that binds to CCR8 or an antigen-binding fragment thereof, having one or more of the following characteristics:
(1) binding specifically to the human CCR8 and/or the cynomolgus CCR8 with a EC₅₀ not exceeding 500 ng/mL;
(2) blocking the binding of the human- and/or cynomolgus-CCR8 to the ligand CCL1;
(3) blocking CCL1-induced β-arrestin recruitment;
(4) eliminating CCR8⁺ Treg cells;
(5) suppressing tumor growth, preferably with no effect on the body weight.

2. An antibody that binds to CCR8 or an antigen-binding fragment thereof, comprising
(i) three complementarity-determining regions HCDR1, HCDR2, and HCDR3 of a heavy-chain variable region as set forth in any of SEQ ID NOs: 21-40, and/or three complementarity-determining regions LCDR1, LCDR2, and LCDR3 of a light-chain variable region as set forth in any of SEQ ID NOs: 42-54;
(ii) a combination of the CDRs as set forth in (i), and wherein, as compared with the HCDR1, the HCDR2, the HCDR3, the LCDR1, the LCDR2, and/or the LCDR3, at least one amino acid addition, substitution or deletion or any combination thereof (e.g. 1, 2, or 3 amino acid additions, substitutions or deletions or any combinations thereof), preferably amino acid substitution(s), and preferably conservative amino acid substitution(s), is further comprised in the sequence(s) thereof, with the affinity for the CCR8 being maintained.

3. An antibody that binds to CCR8 or an antigen-binding fragment thereof comprising a heavy-chain variable region and/or a light-chain variable region, wherein
the heavy-chain variable region comprises:
(1) HCDR1, HCDR2, and HCDR3 having an amino acid sequence as set forth in SEQ ID NO: 1, SEQ ID NO: 2, 10, 11, or 41, and SEQ ID NO: 3, respectively; or HCDR1, HCDR2, and HCDR3 being the same with, or having at least one amino acid addition, substitution or deletion or any combination thereof (e.g. 1, 2, or 3 amino acid additions, substitutions or deletions or any combinations thereof) when compared with the amino acid sequence as set forth in SEQ ID NO: 1, SEQ ID NO: 2, 10, 11, or 41, and SEQ ID NO: 3, respectively; or
(2) HCDR1, HCDR2, and HCDR3 having an amino acid sequence as set forth in SEQ ID NO: 4, SEQ ID NO: 5, and SEQ ID NO: 6, respectively; or HCDR1, HCDR2, and HCDR3 being the same with, or having at least one amino acid addition, substitution or deletion or any combination thereof (e.g. 1, 2, or 3 amino acid additions, substitutions or deletions or any combinations thereof) when compared with the amino acid sequence as set forth in SEQ ID NO: 4, SEQ ID NO: 5, and SEQ ID NO: 6, respectively; or
(3) HCDR1, HCDR2, and HCDR3 having an amino acid sequence as set forth in SEQ ID NO: 7, SEQ ID NO: 8, and SEQ ID NO: 9, respectively; or HCDR1, HCDR2, and HCDR3 being the same with, or having at least one amino acid addition, substitution or deletion or any combination thereof (e.g. 1, 2, or 3 amino acid additions, substitutions or deletions or any combinations thereof) when compared with the amino acid sequence as set forth in SEQ ID NO: 7, SEQ ID NO: 8, and SEQ ID NO: 9, respectively;
and/or
the light-chain variable region comprises:
(1) LCDR1, LCDR2, and LCDR3 having an amino acid sequence as set forth in SEQ ID NO: 12, SEQ ID NO: 13, and SEQ ID NO: 14, respectively; or LCDR1, LCDR2, and LCDR3 being the same with, or having at least one amino acid addition, substitution or deletion or any combination thereof (e.g. 1, 2, or 3 amino acid additions, substitutions or deletions or any combinations thereof) when compared with the amino acid sequence as set forth in SEQ ID NO: 12, SEQ ID NO: 13, and SEQ ID NO: 14, respectively; or
(2) LCDR1, LCDR2, and LCDR3 having an amino acid sequence as set forth in SEQ ID NO: 15, SEQ ID NO: 16, and SEQ ID NO: 17, respectively; or LCDR1, LCDR2, and LCDR3 being the same with, or having at least one amino acid addition, substitution or deletion or any combination thereof (e.g. 1, 2, or 3 amino acid additions, substitutions or deletions or any combinations thereof) when compared with the amino acid sequence as set forth in SEQ ID NO: 15, SEQ ID NO: 16, and SEQ ID NO: 17, respectively; or
(3) LCDR1, LCDR2, and LCDR3 having an amino acid sequence as set forth in SEQ ID NO: 18, SEQ ID NO: 19, and SEQ ID NO: 20, respectively; or LCDR1, LCDR2, and LCDR3 being the same with, or having at least one amino acid addition, substitution or deletion or any combination thereof (e.g. 1, 2, or 3 amino acid additions, substitutions or deletions or any combinations thereof) when compared with the amino acid sequence as set forth in SEQ ID NO: 18, SEQ ID NO: 19, and SEQ ID NO: 20, respectively.

4. An antibody that binds to CCR8 or an antigen-binding fragment thereof, comprising HCDR1, HCDR2, HCDR3, LCDR1, LCDR2, and LCDR3 as shown in any one of the combinations in the table below:
| Combination # | HCDR1 comprising or consisting of the following sequence (or a sequence having at least one amino acid addition, substitution or deletion or any combinatio n thereof when compared to the following sequence) (SEQ ID NO: ) | HCDR2 comprising or consisting of the following sequence (or a sequence having at least one amino acid addition, substitutio n or deletion or any combinatio n thereof when compared to the following sequence) (SEQ ID NO: ) | HCDR3 comprising or consisting of the following sequence (or a sequence having at least one amino acid addition, substitutio n or deletion or any combinatio n thereof when compared to the following sequence) (SEQ ID NO: ) | LCDR1 comprising or consisting of the following sequence (or a sequence having at least one amino acid addition, substitutio n or deletion or any combinatio n thereof when compared to the following sequence) (SEQ ID NO: ) | LCDR2 comprising or consisting of the following sequence (or a sequence having at least one amino acid addition, substitutio n or deletion or any combinatio n thereof when compared to the following sequence) (SEQ ID NO: ) | LCDR3 comprising or consisting of the following sequence (or a sequence having at least one amino acid addition, substitutio n or deletion or any combinatio n thereof when compared to the following sequence) (SEQ ID NO: ) |
|---|---|---|---|---|---|---|
| **1** | 1 | 2 | 3 | 12 | 13 | 14 |
| **2** | 1 | 10 | 3 | 12 | 13 | 14 |
| **3** | 1 | 11 | 3 | 12 | 13 | 14 |
| **4** | 1 | 41 | 3 | 12 | 13 | 14 |
| **5** | 4 | 5 | 6 | 15 | 16 | 17 |
| **6** | 7 | 8 | 9 | 18 | 19 | 20 |

5. An antibody that binds to CCR8 or an antigen-binding fragment thereof, comprising a heavy-chain variable region VH and/or a light-chain variable region VL, wherein,
(a) the heavy-chain variable region VH
(i) comprises or consists of an amino acid sequence having at least 90%, 91%, 92%, 93%, 94%, 95%, 96%, 97%, 98%, or 99% identity to the amino acid sequence set forth in any one selected from SEQ ID NOs: 21-40; or
(ii) comprises or consists of the amino acid sequence as set forth in any one selected from SEQ ID NOs: 21-40; or
(iii) comprises an amino acid sequence having at least one amino acid addition, substitution or deletion or any combination thereof (e.g. 1, 2, or 3 amino acid additions, substitutions or deletions or any combinations thereof) in comparison to the amino acid sequence as set forth in any one selected from SEQ ID NOs: 21-40, where preferably, said amino acid change does not occur in the CDRs, and preferably, said amino acid change occurs in the framework regions (FRs).
and/or
(b) light-chain variable region VL
(i) comprises or consists of an amino acid sequence having at least 90%, 91%, 92%, 93%, 94%, 95%, 96%, 97%, 98%, or 99% identity to the amino acid sequence set forth in any one selected from SEQ ID NOs: 42-54;
(ii) comprises or consists of the amino acid sequence as set forth in any one selected from SEQ ID NOs: 42-54; or
(iii) comprises an amino acid sequence having at least one amino acid addition, substitution or deletion or any combination thereof (e.g. 1, 2, or 3 amino acid additions, substitutions or deletions or any combinations thereof) in comparison to the amino acid sequence as set forth in any one selected from SEQ ID NOs: 42-54, where preferably, said amino acid change does not occur in the CDRs, and preferably, said amino acid change occurs in the FRs.

6. An antibody that binds to CCR8 or an antigen-binding fragment thereof, comprising a heavy-chain variable region VH and a light-chain variable region VL as set forth in any one of the combinations in the table below:
| Combinat ion# | a VH compri sing or consist ing of the followi ng sequen ce (or a sequen ce having at least one amino acid differe nce therefr om) (SEQ ID NO:) | a VL compri sing or consisti ng of the followi ng sequen ce (or a sequen ce having at least one amino acid differe nce therefr om) (SEQ ID NO:) | Combinat ion# | a VH compri sing or consisti ng of the followi ng sequen ce (or a sequen ce having at least one amino acid differe nce therefr om) (SEQ ID NO:) | a VL compri sing or consisti ng of the followi ng sequen ce (or a sequen ce having at least one amino acid differe nce therefr om) (SEQ ID NO:) | Combinat ion# | a VH compri sing or consisti ng of the followi ng sequen ce (or a sequen ce having at least one amino acid differe nce therefr om) (SEQ ID NO:) | a VL compr ising or consisti ng of the followi ng sequen ce (or a sequen ce having at least one amino acid differe nce therefr om) (SEQ ID NO:) |
|---|---|---|---|---|---|---|---|---|
| **1** | 21 | 42 | **16** | 28 | 45 | **31** | 33 | 48 |
| **2** | 21 | 43 | **17** | 28 | 46 | **32** | 33 | 49 |
| **3** | 22 | 42 | **18** | 29 | 44 | **33** | 34 | 47 |
| **4** | 22 | 43 | **19** | 29 | 45 | **34** | 34 | 48 |
| **5** | 23 | 42 | **20** | 29 | 46 | **35** | 34 | 49 |
| **6** | 23 | 43 | **21** | 30 | 44 | **36** | 35 | 47 |
| **7** | 24 | 42 | **22** | 30 | 45 | **37** | 35 | 48 |
| **8** | 24 | 43 | **23** | 30 | 46 | **38** | 35 | 49 |
| **9** | 25 | 42 | **24** | 31 | 44 | **39** | 36 | 50 |
| **10** | 25 | 43 | **25** | 31 | 45 | **40** | 37 | 51 |
| **11** | 26 | 42 | **26** | 31 | 46 | **41** | 38 | 52 |
| **12** | 26 | 43 | **27** | 32 | 47 | **42** | 39 | 53 |
| **13** | 27 | 42 | **28** | 32 | 48 | **43** | 40 | 54 |
| **14** | 27 | 43 | **29** | 32 | 49 | | | |
| **15** | 28 | 44 | **30** | 33 | 47 | | | |

7. The antibody or an antigen-binding fragment thereof of any one of claims 1 to 6, which is a mouse-derived antibody, a chimeric antibody, a humanized antibody or a fully human antibody.

8. The antibody or an antigen-binding fragment thereof of any one of claims 1 to 7, comprising a heavy-chain constant region and/or a light-chain constant region, where preferably, the light-chain constant region is the λ- or κ-chain constant region; and the heavy-chain constant region is selected from a murine mIgG2a, a human IgG1, a human IgG2, a human IgG3, and an IgG4 subclass, more preferably, the heavy-chain constant region is of the human IgG1 subclass, or of the human IgG4 subclass with a S228P mutation.

9. The antibody or an antigen-binding fragment thereof of any one of claims 1 to 7, comprising a heavy-chain and/or light-chain, wherein
(a) the heavy chain
(i) comprises or consists of an amino acid sequence having at least 85%, 90%, 91%, 92%, 93%, 94%, 95%, 96%, 97%, 98%, or 99% identity to the amino acid sequence selected from any one of SEQ ID NOs: 55-74;
(ii) comprises or consists of the amino acid sequence selected from any one of SEQ ID NOs: 55-74; or
(iii) comprises an amino acid sequence having at least one amino acid addition, substitution or deletion or any combination thereof (e.g. 1, 2, or 3 amino acid additions, substitutions or deletions or any combinations thereof) in comparison to the amino acid sequence as set forth in any one selected from SEQ ID NOs: 55-74, where preferably, said amino acid change does not occur in the CDRs of the heavy-chain variable region, more preferably, said amino acid change does not occur in the heavy-chain variable region, and most preferably, the amino acid change occurs in the heavy-chain constant region;
and/or
(b) the light chain
(i) comprises or consists of an amino acid sequence having at least 85%, 90%, 91%, 92%, 93%, 94%, 95%, 96%, 97%, 98%, or 99% identity to the amino acid sequence selected from any one of SEQ ID NOs: 75-82;
(ii) comprises or consists of the amino acid sequence selected from any one of SEQ ID NOs: 75-82; or
(iii) comprises an amino acid sequence having at least one amino acid addition, substitution or deletion or any combination thereof (e.g. 1, 2, or 3 amino acid additions, substitutions or deletions or any combinations thereof) in comparison to the amino acid sequence as set forth in any one selected from SEQ ID NOs: 75-82, where preferably, said amino acid change does not occur in the CDRs of the light-chain variable region, more preferably, said amino acid change does not occur in the light-chain variable region, and most preferably, the amino acid change occurs in the light-chain constant region.

10. The antibody or the antigen-binding fragment thereof of claim 9, wherein the antibody or the antigen-binding fragment thereof comprises a heavy-chain constant region, whose sequence has one or more amino acid substitutions as compared to that of a native human heavy-chain constant region, and preferably, the one or more amino acid substitutions enhance the ADCC effect of the antibody; more preferably, the one or more amino acid substitutions occur at one or more of the following sites in the sequence of the heavy-chain constant region: 238, 239, 248, 249, 252, 254, 255, 256, 258, 265, 267, 268, 269, 270, 272, 276, 278, 280, 283, 285, 286, 289, 290, 292, 293, 294, 295, 296, 298, 301, 303, 305, 307, 309, 312, 315, 320, 322, 324, 326, 327, 328, 329, 330, 331, 333, 334, 335, 337, 338, 340, 360, 373, 376, 378, 382, 388, 389, 398, 414, 416, 419, 430, 434, 435, 437, 438, and 439, numbered according to EU numbering system.

11. The antibody or an antigen-binding fragment thereof of claim 10, wherein said one or more amino acid substitutions occur at one or more of the following sites in the sequence of the heavy-chain constant region: L234, L235, G236, S239, F243, T256, D265, H268, D270, K290, R292, S298, Y300, V305, K326, A330, 1332, E333, K334, A339, and P396, numbered according to EU numbering system.

12. The antibody or an antigen-binding fragment thereof of claim 11, wherein said one or more amino acid substitutions are selected from one or more of the substitutions G236A, S239D, F243L, T256A, K290A, R292P, S298A, Y300L, V305I, A330L, 1332E, E333A, K334A, A339T and P396L, numbered according to the EU numbering system.

13. The antibody or an antigen-binding fragment thereof of claim 10, wherein said one or more amino acid substitutions are selected from one or more of N297A substitution, N297Q substitution, L235A together with L237A substitutions, L234A together with L235A substitutions, E233P substitution, L234V substitution, L235A substitution, C236 deletion, P238A substitution, D265A substitution, A327Q substitution, and P329A substitution, numbered according to the EU numbering system.

14. The antibody or an antigen-binding fragment thereof of claim 10, wherein said one or more amino acid substitutions occur at one or more of the following sites in the sequence of the heavy-chain constant region: 235, 239, 243, 292, 300, 330, 332, and 396, numbered according to the EU numbering system.

15. The antibody or an antigen-binding fragment thereof of claim 14, wherein one or more amino acid substitutions are selected from at least one of the substitutions S239D, L235V, F243L, R292P, Y300L, A330L, I332E, and P396L, numbered according to the EU numbering system.

16. The antibody or an antigen-binding fragment thereof of claim 10, wherein said heavy-chain constant region has one or more sets of simultaneous mutations at the following combinations of sites selected from (1) L235/F243/R292/Y300/P396, (2) F243/R292/Y300/V305/P396, (3) D270/K326/A330/K334, (4) S239/A330/I332, (5) S298/E333/K334, (6) L234/L235/G236/S239/H268/D270/S298, (7) M252/S254/T256, (8) L234/L235/D265, (9) G236/S239/1332, and (10) S239/1332, numbered according to the EU numbering system.

17. The antibody or an antigen-binding fragment thereof of claim 10, wherein said heavy-chain constant region has one or more sets of the mutations selected from the following mutation combinations: (1) L235V/F243LIR292P/Y300L/P396L, (2) F243L/R292P/Y300L/V305I/P396L, (3) D270E/K326D/A330M/K334E, (4) S239D/A330L/1332E, (5) S298A/E333A/K334A, (6) L234Y/L235Q/G236W/S239M/H268D/D270E/S298A, (7) M252Y/S254T/T256E, (8) L234A/L235A/D265A, (9) L234F/L235E/D265A, (10) G236A/S239D/1332E, and (11) S239D/1332E, numbered according to the EU numbering system.

18. An antibody that binds to CCR8 or an antigen-binding fragment thereof, comprising a heavy chain HC and a light chain LC as shown in any one of the combinations in the table below:
| Combinat ion# | a heavy chain compri sing or consisti ng of the followi ng sequen ce (or a sequen ce having at least one amino acid differe nce therefr om) (SEQ ID NO: ) | a light chain compri sing or consisti ng of the followi ng sequen ce (or a sequen ce having at least one amino acid differen ce therefr om) (SEQ ID NO: ) | Combination # | a heavy chain compri sing or consisti ng of the followi ng sequen ce (or a sequen ce having at least one amino acid differen ce therefr om) (SEQ ID NO: ) | a light chain compri sing or consisti ng of the followi ng sequen ce (or a sequen ce having at least one amino acid differen ce therefr om) (SEQ ID NO: ) | Combinat ion# | a heavy chain compri sing or consisti ng of the followi ng sequen ce (or a sequen ce having at least one amino acid differen ce therefr om) (SEQ ID NO: ) | a light chain compri sing or consisti ng of the followi ng sequen ce (or a seque nce having at least one amino acid differen ce therefr om) (SEQ ID NO: ) |
|---|---|---|---|---|---|---|---|---|
| **1** | 55 | 75 | **16** | 60 | 75 | **31** | 68 | 77 |
| **2** | 62 | 75 | **17** | 60 | 76 | **32** | 68 | 78 |
| **3** | 63 | 75 | **18** | 61 | 75 | **33** | 68 | 79 |
| **4** | 64 | 75 | **19** | 61 | 76 | **34** | 71 | 80 |
| **5** | 55 | 76 | **20** | 65 | 77 | **35** | 71 | 81 |
| **6** | 62 | 76 | **21** | 65 | 78 | **36** | 71 | 82 |
| **7** | 63 | 76 | **22** | 65 | 79 | **37** | 72 | 80 |
| **8** | 56 | 75 | **23** | 66 | 77 | **38** | 72 | 81 |
| **9** | 56 | 76 | **24** | 66 | 78 | **39** | 72 | 82 |
| **10** | 57 | 75 | **25** | 69 | 78 | **40** | 73 | 80 |
| **11** | 57 | 76 | **26** | 70 | 78 | **41** | 73 | 81 |
| **12** | 58 | 75 | **27** | 66 | 79 | **42** | 73 | 82 |
| **13** | 58 | 76 | **28** | 67 | 77 | **43** | 74 | 80 |
| **14** | 59 | 75 | **29** | 67 | 78 | **44** | 74 | 81 |
| **15** | 59 | 76 | **30** | 67 | 79 | **45** | 74 | 82 |

19. The antibody or an antigen-binding fragment thereof of any one of claims 1 to 18, wherein the constant region of the antibody or the antigen-binding fragment thereof is afucosylated or less-fucosylated.

20. The antibody that binds to CCR8 or an antigen-binding fragment thereof according to any one of claims 1 to 19, wherein said antibody is a monoclonal antibody.

21. The antibody or an antigen-binding fragment thereof of any one of claims 1 to 19, wherein said antigen-binding fragment is an antibody fragment selected from a Fab, a Fab', a Fd, a Fab'-SH, a Fv, a single chain antibody (for instance, a scFv) or a (Fab')₂, a single-domain antibody, a diabody (dAb) or a linear antibody.

22. An isolated polynucleotide encoding one or more chains of the antibody that binds to CCR8 or the antigen-binding fragment thereof of any one of claims 1 to 21.

23. A vector comprising the polynuceotide of claim 22, wherein preferably, the vector is an expression vector.

24. A host cell comprising the polynucleotide of claim 22 or the vector of claim 23, wherein preferably, the host cell is prokaryotic or eukaryotic cells, more preferably selected from yeast cells, mammalian cells (e.g., said host cell is a CHO cell, e.g. a CHO-K1 cell or an expiCHO cell, or said host cell is a 293 cell, e.g. an HEK293 cell) or other cells suitable for preparing antibodies or antigen-binding fragments thereof.

25. A method for preparing the antibody that binds to CCR8 or antigen-binding fragment thereof, wherein the method comprises culturing a host cell comprising the nucleic acid encoding the antibody or antigen-binding fragments thereof of any one of claims 1 to 21 under the condition suitable for expressing antibodies, and optionally, said method further comprises recovering the antibody or the antigen-binding fragment thereof from the host cell.

26. An immunoconjugate comprising the antibody that binds to CCR8 or the antigen-binding fragment thereof of any one of claims 1 to 21 and other substances, such as therapeutic agents or labels.

27. A pharmaceutical composition comprising the antibody that binds to CCR8 or the antigen-binding fragment thereof of any one of claims 1 to 21 or the immunoconjugate of claim 26, and optionally one or more of other therapeutic agents, and optionally pharmaceutically acceptable excipient.

28. A pharmaceutical combination comprising the antibody that binds to CCR8 or the antigen-binding fragment thereof of any one of claims 1 to 21 or the immunoconjugate of claim 26, and optionally one or more of other therapeutic agents, wherein preferably the other therapeutic agent is a monoclonal antibody, more preferably a monoclonal antibody targeting an immune checkpoint, and most preferably a monoclonal antibody targeting PD-1.

29. A method for enhancing the immune response (e.g., the anti-tumor immune response) in a subject, comprising administering to the subject an effective amount of the antibody or the antigen-binding fragment thereof of any one of claims 1 to 21, or the polynucleotide of claim 22, or the vector of claim 23, or the host cell of claim 24, or the immunoconjugate of claim 26, or the pharmaceutical composition of claim 27, or the combination product of claim 28.

30. A method for preventing and/or treating a tumor, an autoimmune or an infectious disease in a subject, the method comprising administering to the subject an effective amount of the antibody or the antigen-binding fragment thereof of any one of claims 1 to 21, or the polynucleotide of claim 22, or the vector of claim 23, or the host cell of claim 24, or the immunoconjugate of claim 26, or the pharmaceutical composition of claim 27, or the combination product of claim 28, wherein preferably, the disease is a tumor, e.g., melanoma, breast cancer, colon carcinoma, rectal cancer, colorectal cancer, gastric cancer, esophageal cancer, head and neck cancer, lung cancer, ovarian cancer, kidney cancer, bladder cancer, liver cancer or thymus cancer and the metastatic cancers thereof.

31. The method of claim 30, further comprising the co-administration of one or more therapies to the subject, such as treatment modalities and/or other therapeutic agents, wherein preferably, the treatment includes surgical treatments and/or radiation therapies.

32. Use of an effective amount of the anti-CCR8 antibody or the antigen-binding fragment thereof of any one of claims 1 to 21, or the polynucleotide of claim 22, or the vector of claim 23, or the host cell of claim 24, or the immunoconjugate of claim 26, or the pharmaceutical composition of claim 27, or the pharmaceutical combination of claim 28 in the manufacture of a medicament for preventing and/or treating tumors, autoimmune or infectious diseases.

33. Use of an effective amount of the anti-CCR8 antibody or the antigen-binding fragment thereof of any one of claims 1 to 21, or the polynucleotide of claim 22, or the vector of claim 23, or the host cell of claim 24, or the immunoconjugate of claim 26, or the pharmaceutical composition of claim 27 in combination with other therapeutic agents in the manufacture of a medicament for preventing and/or treating tumors, autoimmune or infectious diseases, wherein preferably, the other therapeutic agent is a monoclonal antibody, more preferably a monoclonal antibody targeting an immune checkpoint, and most preferably a monoclonal antibody targeting PD-1.

34. The use according to claim 32 or 33, wherein said tumor is a solid tumor.

35. The use according to claim 34, wherein said tumor is melanoma, breast cancer, colon carcinoma, rectal cancer, colorectal cancer, gastric cancer, esophageal cancer, head and neck cancer, lung cancer, ovarian cancer, kidney cancer, bladder cancer, hepatocellular carcinoma or thymic carcinoma and the metastatic cancers thereof.

36. Use of any one of claims 32 to 35, further comprising administered to the subject one or more other therapies in combination.

37. The use of the claim 36, wherein said therapy comprises treatment modalities and/or other therapeutic agents.

38. A method of detecting CCR8 in a sample, comprising the step of using the antibody or the antigen-binding fragment thereof of any one of claims 1 to 21 or the immunuoconjugate of claim 26.

39. A detection kit comprising the antibody or the antigen-binding fragment thereof of any one of claims 1 to 21 or the immunuoconjugate of claim 26.
